# EUROPEAN PATENT APPLICATION

(11) **EP 4 818 188 A1**
(43) Date of publication of application: **07.10.2026**
(21) Application number: 25771719.9
(22) Date of filing: 12.03.2025
(51) Int. Cl.: C07K 5/062, C07K 1/02, C07K 4/00, C07K 5/083, C07K 7/54

(54) **METHOD FOR PRODUCING PEPTIDE COMPOUND HAVING RING STRUCTURE**

(30) Priority: 13.03.2024 JP 2024038864
(71) Applicant: CHUGAI SEIYAKU KABUSHIKI KAISHA, Tokyo 115-8543 (JP)
(72) Inventor: NOGI, Keisuke, Tokyo 115-8543 (JP); SATO, Kazuto, Tokyo 115-8543 (JP)
(74) Representative: Vossius & Partner Patentanwälte Rechtsanwälte mbB
(86) International application number: PCT/JP2025/009231
(87) International publication number: WO 2025/192626

(57) **Abstract**

The present invention provides a novel ring-closing metathesis reaction for constructing a peptide structure with a medium ring. The present invention provides a method for carrying out the ring-closing metathesis reaction under a low dilution condition (e.g., at a concentration of 0.1 mol/L). The present invention is to provide a method for carrying out a ring-closing metathesis reaction suitable for mass production.

## Description

### [Technical Field]

The present invention relates to a method for producing a peptide compound having a ring structure using a ring-closing metathesis reaction.

### [Background Art]

Recently, it has been found that the metabolic stability and membrane permeability of the peptides are improved among certain cyclic peptide compounds containing non-natural amino acids (Non Patent Literatures 1 and 2). Some of the cyclic peptides have a dipeptide structure with a medium ring (Patent Literature 1). Among the structures with a medium ring, those with 5- to 7-membered rings are specifically referred to as Friedinger Lactam. Friedinger Lactam is a structure characterized by its ability to change the pharmacological activity and metabolic stability by fixing the conformation, and is sometimes observed in drug design (Non Patent Literature 3).

The medium ring is typically formed through a ring-closing metathesis reaction using a ruthenium-alkylidene complex typified by Hoveyda-Grubbs catalyst. A substrate in which an N-terminal amino group is protected with Boc, Fmoc or the like is typically used when the ring-closing metathesis reaction is carried out (Non Patent Literatures 4 and 5). However, in these methods, unless high dilution conditions (e.g., 0.01 mol/L) are employed, intermolecular reactions compete with the desired ring-closing metathesis reaction (i.e., intramolecular reaction), and the desired product cannot be obtained with a high yield. On the other hand, when the ring-closing metathesis reaction is carried out under a high dilution condition, there is a possibility that the rate of the ring-closing metathesis reaction decreases because of a low concentration of a substrate, and the catalyst deactivates, which results in an increase in the catalyst amount, and associated addition of a catalyst removal process. Furthermore, since high dilution conditions require the use of a large amount of solvent, they are unsuitable for large scale synthesis, such as in active pharmaceutical ingredient (API) production, from the perspective of productivity.

### [Citation List]

### [Patent Literature]

[Patent Literature 1] International Publication No. WO 2022/234853

### [Non Patent Literature]

[Non Patent Literature 1] Acc. Chem. Res. 2008, 41, 1331-1342.
[Non Patent Literature 2] Angew. Chem. Int. Ed., 2013, 52, 254-269.
[Non Patent Literature 3] Science, 1980, 210, 676-658.
[Non Patent Literature 4] J. Org. Chem., 2003,68,62-69.
[Non Patent Literature 5] J. Org. Chem., 2015,80,4904-4918.

### [Summary of Invention]

### [Technical Problem]

The present invention has been made in view of such circumstances. In one aspect, a problem to be solved is to establish a novel ring-closing metathesis reaction for constructing a peptide structure with a medium ring. In one aspect, a problem to be solved is to establish a method for carrying out a ring-closing metathesis reaction under a low dilution condition (e.g., 0.1 mol/L). In one aspect, a problem to be solved is to establish a method for carrying out a ring-closing metathesis reaction with a high yield. In one aspect, a problem to be solved is to establish a method for carrying out a ring-closing metathesis reaction with reduced by-product formation. In one aspect, a problem to be solved is to establish a method for carrying out a ring-closing metathesis reaction suitable for mass production.

### [Solution to Problem]

The present inventors have conducted intensive studies to solve the above problems, and found that a ring-closing metathesis reaction can proceed while maintaining high intramolecular reaction selectivity even at relatively high concentrations, in the presence of a catalyst such as a metal-alkylidene complex, by using a substrate in which an N-terminal amino group is an unprotected amino acid.

In one non-limiting specific aspect, the present invention encompasses the following.
[1] A method for producing a compound represented by formula (1) or a salt thereof, comprising the step (metathesis step) of bringing a compound represented by formula (2) or a salt thereof into contact with a catalyst: 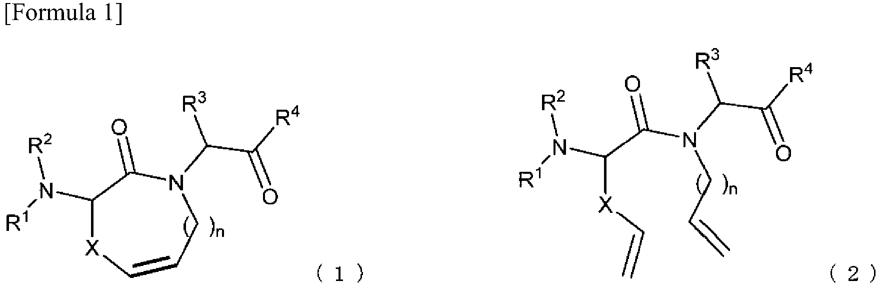 wherein
   R¹ is hydrogen,
   R² is hydrogen or C₁-C₆ alkyl;
   R³ is hydrogen, optionally substituted C₁-C₆ alkyl, optionally substituted halo-C₁-C₆ alkyl, optionally substituted C₂-C₆ alkenyl, optionally substituted C₂-C₆ alkynyl, optionally substituted C₃-C₈ cycloalkyl, optionally substituted C₆-C₁₄ aryl, optionally substituted 5- to 14-membered heteroaryl, optionally substituted C₇-C₁₄ aralkyl, optionally substituted 3- to 14-membered heterocyclyl, optionally substituted 5- to 10-membered heteroaryl-C₁-C₆ alkyl, optionally substituted C₁-C₆ alkoxy-C₁-C₆ alkyl, optionally substituted C₁-C₆ alkylsulfanyl-C₁-C₆ alkyl, optionally substituted C₁-C₆ alkylsulfinyl-C₁-C₆ alkyl, optionally substituted C₁-C₆ alkylsulfonyl-C₁-C₆ alkyl, optionally substituted carboxy-C₁-C₆ alkyl, optionally substituted C₇-C₁₄ aralkoxy-C₁-C₆ alkyl, optionally substituted C₃-C₈ cycloalkyl-C₁-C₆ alkyl, optionally substituted C₃-C₈ cycloalkoxy-C₁-C₆ alkyl, optionally substituted 4- to 7-membered heterocyclyl-C₁-C₃ alkyl, optionally substituted 5- to 10-membered heteroaryl-C₁-C₆ alkoxy-C₁-C₆ alkyl, or optionally substituted aminocarbonyl (the relevant amino is selected from the group consisting of -NH₂, mono-C₁-C₆ alkylamino, di-C₁-C₆ alkylamino, N-C₁-C₆ alkyl-N-C₂-C₆ alkenylamino, N-C₁-C₆ alkyl-N-C₁-C₆ alkoxy-C₁-C₆ alkylamino, and 4- to 9-membered cyclic amino),
   R⁴ is OR⁵, NHR⁵', an amino acid residue, or a peptide chain containing 1 to 20 amino acid residues, where the amino acid residue and the peptide chain may have a protective group,
   R⁵ is a protective group for a carboxy group,
   R⁵' is a protective group for an amide group,
   n is an integer of 1 to 4, and
   X is optionally substituted C₁-C₃ alkylene, -CH₂OCH₂-, or -CH₂SCH₂-.
[2] A method for producing a compound represented by formula (3) or a salt thereof, comprising the step of hydrogenating a compound represented by formula (1) or a salt thereof, which is obtained by the method of [1]: 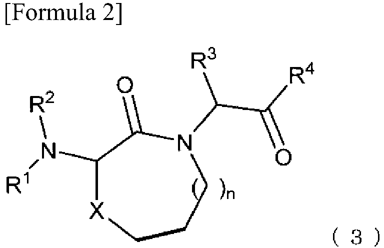 wherein R¹, R², R³, R⁴, n and X have the same meaning as R¹, R², R³, R⁴, n and X in [1], respectively.
[3] A method for producing a compound represented by formula (4) or a salt thereof, comprising the step of protecting an amino group of a compound represented by formula (1) or a salt thereof, which is obtained by the method of [1], with a carbamate protective group: 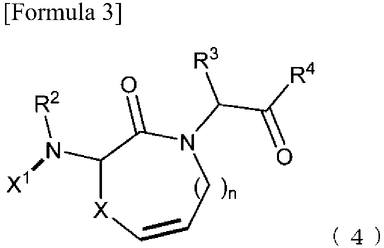 wherein R², R³, R⁴, n and X have the same meaning as R², R³, R⁴, n and X in [1], respectively, and X¹ is a carbamate protective group.
[4] A method for producing a compound represented by formula (5) or a salt thereof, comprising the step of protecting an amino group of a compound represented by formula (3) or a salt thereof, which is obtained by the method of [2], with a carbamate protective group: 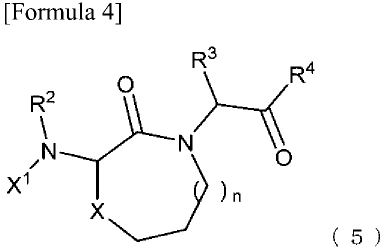 wherein R², R³, R⁴, n and X have the same meaning as R², R³, R⁴, n and X in [1], respectively, and X¹ is a carbamate protective group.
[5] The method according to any of [1] to [4], wherein the catalyst is a metal-alkylidene complex.
[6] The method according to any of [1] to [5], wherein the catalyst is a ruthenium-alkylidene complex, a molybdenum-alkylidene complex, or a tungsten-alkylidene complex.
[7] The method according to any of [1] to [6], wherein the catalyst is a ruthenium-alkylidene complex.
[8] The method according to any of [1] to [7], wherein the catalyst is a ruthenium-alkylidene complex containing phosphine and/or N-heterocyclic carbene as a ligand.
[9] The method according to any of [1] to [8], wherein the catalyst is first-generation Grubbs catalyst (dichloro(benzylidene)bis(tricyclohexylphosphine)ruthenium (II)), second-generation Grubbs catalyst(dichloro[1,3-bis(2,4,6-trimethylphenyl)-2-imidazolidinylidene](benzylidene)(tricyclohexylphosphine)ruthenium (II)), third-generation Grubbs catalyst(dichloro[1,3-bis(2,4,6-trimethylphenyl)-2-imidazolidinylidene](benzylidene)bis(3-bromopyridine)ruthenium (II)), Stewart-Grubbs catalyst(dichloro[1,3-bis(2-methylphenyl)-2-imidazolidinylidene](2-isopropoxyphenylmethylene)ruthenium (II)), nitro-Grela catalyst(dichloro[1,3-bis(2,4,6-trimethylphenyl)-2-imidazolidinylidene][(2-isopropoxy-5-nitrobenzylidene)]ruthenium (II)), Zhan catalyst-1B(dichloro[1,3-bis(2,4,6-trimethylphenyl)-2-imidazolidinylidene][[5-[(dimethylamino)sulfonyl]-2-(1-methylethoxy-O)phenyl]methylene-C]ruthenium(II)), Zhan catalyst-1C({[2-(isopropoxy)-5-(N,N-dimethylaminosulfonyl)phenyl]methylene}(tricyclohexylphosphine)dichlororuthenium(II)), first-generation Hoveyda-Grubbs catalyst(dichloro(2-isopropoxyphenylmethylene)(tricyclohexylphosphine)ruthenium), second-generation Hoveyda-Grubbs catalyst((1,3-bis-(2,4,6-trimethylphenyl)-2-imidazolidinylidene)dichloro(o-isopropoxyphenylmethylene)ruthenium), [1,3-bis(2,6-isopropylphenyl)-2-imidazolidinylidene]dichloro(2-isopropoxybenzylidene)ruthenium (II), bis[1-(2,6-diethylphenyl)-3,5,5-trimethyl-3-phenylpyrrolidine-2-ylidene](3-phenyl-1H-inden-1-ylidene)ruthenium chloride, [1-(2,6-diethylphenyl)-3,5,5-trimethyl-3-phenylpyrrolidin-2-yl](2-isopropoxy-5-nitrobenzylidene)ruthenium (II) chloride, or [1-[2,4,6-trimethylphenyl]-3,5,5-trimethyl-3-phenyl-2-pyrrolinylidene]dichloro(2-isopropoxybenzylidene)ruthenium (II).
[10] The method according to any of [1] to [9], wherein the catalyst is first-generation Hoveyda-Grubbs catalyst(dichloro(2-isopropoxyphenylmethylene)(tricyclohexylphosphine)ruthenium), second-generation Hoveyda-Grubbs catalyst((1,3-bis-(2,4,6-trimethylphenyl)-2-imidazolidinylidene)dichloro(o-isopropoxyphenylmethylene)ruthenium), or [1-[2,4,6-trimethylphenyl]-3,5,5-trimethyl-3-phenyl-2-pyrrolinylidene]dichloro(2-isopropoxybenzylidene)ruthenium (II).
[11] The method according to any of [1] to [10], wherein the compound represented by formula (2) or a salt thereof is a salt of a compound represented by formula (2).
[12] The method according to [11], wherein the salt of a compound represented by formula (2) is a salt with a Bronsted acid having a pKa of 3 or less.
[13] The method according to [12], wherein the salt of a compound represented by formula (2) is a hydrochloric acid salt, a hydrobromic acid salt, a sulfuric acid salt, a phosphoric acid salt, a methanesulfonic acid salt, a para-toluenesulfonic acid salt, a trifluoromethanesulfonic acid salt, or a trifluoroacetic acid salt.
[14] The method according to [12] or [13], wherein the salt of the compound represented by formula (2) is a hydrochloric acid salt.
[15] The method according to any of [1] to [10], wherein the compound represented by formula (2) or a salt thereof is a compound represented by formula (2), and an acid is used in the metathesis step.
[16] The method according to [15], wherein the acid is a Bronsted acid having a pKa of 3 or less.
   [16-1] The method according to [16], wherein the Bronsted acid is at least one Bronsted acid selected from the group consisting of hydrogen chloride, hydrobromic acid, sulfuric acid, phosphoric acid, methanesulfonic acid, para-toluenesulfonic acid, trifluoromethanesulfonic acid, and trifluoroacetic acid.
[17] The method according to [16], wherein the Bronsted acid is hydrogen chloride.
[18] The method according to [15], wherein the acid is a Lewis acid.
   [18-1] The method according to [18], wherein the Lewis acid is at least one Lewis acid selected from the group consisting of boron trihalide, a solvation complex thereof, titanium tetrahalide, and tetraalkoxy titanium.
[19] The method according to [18], wherein the acid is a boron trifluoride-tetrahydrofuran complex or a boron trifluoride-diethyl ether complex.
[20] The method according to any of [1] to [10], wherein alcohols and/or Bronsted acids are further used as an additive in the metathesis step, and pKa of the additive is 4 to 17.
   [20-1] The method according to [12], [16] or [20], wherein the pKa is a measured value of pKa when water at 25°C is used as a solvent.
[21] The method according to any of [20], wherein the additive is at least one selected from the group consisting of 1,1,1,3,3,3-hexafluoro-2-propanol (HFIP), 2-propanol, methanol, 2,2,2-trifluoroethanol (TFE), and acetic acid.
[22] The method according to [20] or [21], wherein the additive is 1,1,1,3,3,3-hexafluoro-2-propanol (HFIP), or acetic acid.
[23] The method according to any of [20] to [22], wherein the additive is used in an amount of 0.1 mL to 100 mL per g of the compound represented by formula (2).
   [23-1] The method according to any of [20] to [22], wherein the additive is used in an amount of 1 mL to 10 mL per g of the compound represented by formula (2).
[24] The method according to any of [1] to [23], wherein the metathesis step is performed by a liquid-phase synthesis method.
[25] The method according to [24], wherein the solvent used in the liquid-phase synthesis method comprises at least one selected from the group consisting of a ketone-based solvent, a nitrile-based solvent, a halogen-based solvent, an ether-based solvent, an amide-based solvent, an ester-based solvent, an aromatic hydrocarbon-based solvent, an aliphatic hydrocarbon-based solvent, and a carbonate-ester-based solvent.
[26] The method according to [25], wherein the ketone-based solvent is one or more selected from the group consisting of acetone, methyl ethyl ketone, methyl isobutyl ketone, cyclohexanone, cyclopentanone, and diethyl ketone,
   the nitrile-based solvent is one or more selected from the group consisting of acetonitrile and propionitrile,
   the halogen-based solvent is one or more selected from the group consisting of dichloromethane, chloroform and 1,2-dichloroethane,
   the ether-based solvent is one or more selected from the group consisting of diethyl ether, tetrahydrofuran, 2-methyltetrahydrofuran, cyclopentyl methyl ether, 4-methyltetrahydropyran, 1,3-dioxolane, 1,4-dioxane, 1,2-dimethoxyethane, diisopropyl ether, methyl t-butyl ether, diglyme, triglyme, anisole, and tetraglyme,
   the amide-based solvent is one or more selected from the group consisting of N,N-dimethylformamide (DMF), N-methyl-2-pyrrolidone (NMP), N,N-dimethylacetamide (DMA), N-ethyl-2-pyrrolidone (NEP), N-butyl-2-pyrrolidone (NBP), 1,3-dimethyl-2-imidazolidinone (DMI), and formamide.
   the ester-based solvent is one or more selected from the group consisting of methyl acetate, ethyl acetate, methyl propionate, butyl acetate, propyl acetate, isopropyl acetate, isobutyl acetate, pentyl acetate, and y-valerolactone,
   the aromatic hydrocarbon-based solvent is one or more selected from the group consisting of toluene, o-xylene, m-xylene, p-xylene, mesitylene, chlorobenzene, fluorobenzene, and anisole,
   the aliphatic hydrocarbon-based solvent is one or more selected from the group consisting of pentane, hexane, heptane, octane, and cyclohexane, and
   the carbonate-ester-based solvent is one or more selected from the group consisting of dimethyl carbonate, diethyl carbonate and dibutyl carbonate.
[27] The method according to any of [24] to [26], wherein the solvent used in the liquid-phase synthesis method is one or more selected from the group consisting of acetone, methyl t-butyl ether, dimethyl carbonate, 2-methyltetrahydropyran, 4-methyltetrahydropyran, tetrahydrofuran, ethyl acetate, isopropyl acetate, dichloromethane, 1,2-dichloroethane, toluene, chlorobenzene, heptane, and cyclohexane.
[28] The method according to any of [24] to [27], wherein the solvent used in the liquid-phase synthesis method is one or more selected from the group consisting of acetone, methyl t-butyl ether, dimethyl carbonate, ethyl acetate, toluene, and dichloromethane.
[29] The method according to any of [24] to [28], wherein the solvent used in the liquid-phase synthesis method is acetone or toluene.
[30] The method according to any of [24] to [29], wherein the solvent used in the liquid-phase synthesis method is acetone.
[31] The method according to any of [25] to [30], wherein a concentration of the compound represented by formula (2) or a salt thereof, with respect to the solvent used in the liquid-phase synthesis method is 0.01 to 0.3 mol/L.
   [31-1] The method according to any of [25] to [31], wherein a concentration of the compound represented by formula (2) or a salt thereof, with respect to the solvent used in the liquid-phase synthesis method is 0.02 to 0.3 mol/L.
   [31-2] The method according to any of [25] to [31-1], wherein a concentration of the compound represented by formula (2) or a salt thereof, with respect to the solvent used in the liquid-phase synthesis method is 0.033 to 0.3 mol/L.
   [31-3] The method according to any of [25] to [31-2], wherein preparation of a reaction mixture in the liquid phase-synthesis method is one of the following (a) to (d):
      (a) preparing a mixture of the compound represented by formula (2) or a salt thereof, a catalyst, an acid, an additive and a solvent by adding a solvent mixture containing the compound represented by formula (2) or a salt thereof to a solvent mixture containing a catalyst;
      (b) preparing a mixture of the compound represented by formula (2) or a salt thereof, a catalyst, an acid, an additive and a solvent by adding a solvent mixture containing the compound represented by formula (2) or a salt thereof to a solvent mixture containing a heated catalyst;
      (c) preparing a mixture of the compound represented by formula (2) or a salt thereof, a catalyst, an acid, an additive and a solvent by adding a solvent mixture containing a catalyst to a solvent mixture containing the compound represented by formula (2) or a salt thereof; and
      (d) preparing a mixture by adding a solvent to the compound represented by formula (2) or a salt thereof, and then a catalyst, an acid and an additive.
   [31-4] The method according to [31-3] (b), wherein the heating is performed near a boiling point of the solvent.
[32] The method according to any of [1] to [31-4], wherein the compound represented by formula (2) or a salt thereof is obtained by condensing a compound represented by formula (6) and a compound represented by formula (7), and then deprotecting the carbamate protective group. 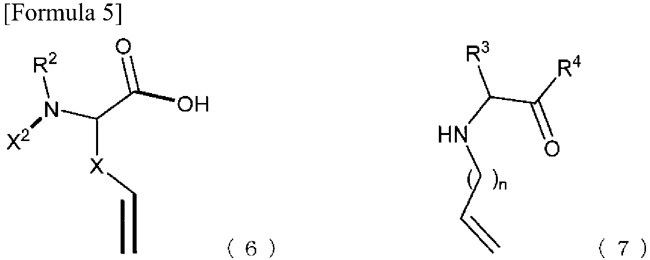 wherein R², R³, R⁴, n and X have the same meaning as R², R³, R⁴, n and X in [1], respectively, and X² is a carbamate protective group.
[33] The method according to any of [1] to [32], wherein R² is hydrogen or linear C₁-C₃ alkyl.
[34] The method according to any of [1] to [33], wherein R² is hydrogen or methyl.
[35] The method according to any of [1] to [34], wherein R³ is hydrogen, optionally substituted C₁-C₆ alkyl, optionally substituted C₂-C₆ alkenyl, optionally substituted C₂-C₆ alkynyl, optionally substituted C₃-C₈ cycloalkyl, optionally substituted C₇-C₁₄ aralkyl, optionally substituted 5- to 10-membered heteroaryl-C₁-C₆ alkyl, or optionally substituted 3- to 14-membered heterocyclyl.
[36] The method according to any of [1] to [35], wherein R³ is hydrogen, C₁-C₆ alkyl, or C₇-C₁₄ aralkyl optionally substituted with halo-C₁-C₃ alkyl or C₁-C₆ alkyl.
[37] The method according to any of [1] to [36], wherein R³ is hydrogen, 2-methylpropyl, p-(trifluoromethyl)benzyl, or p-methylbenzyl.
[38] The method according to any of [1] to [37], wherein R⁴ is OR⁵, NHR^{5'}, an amino acid residue, or a peptide chain containing 2 to 13 amino acid residues,
   R⁵ is an alkyl ester-type protective group, a benzyl ester-type protective group, a substituted alkyl ester-type protective group, or an alkenyl ester-type protective group, preferably a methyl group, an ethyl group, a t-Bu group, a benzyl group, a trityl group, a cumyl group, a methoxytrityl group, a 2-(trimethylsilyl)ethyl group, a 2,2,2-trichloroethyl group, or an allyl group, and more preferably tert-butyl, or benzyl,
   R⁵' is a phenylamide-type protective group, an alkylamide-type protective group, an alkenylamide-type protective group, a benzylamide-type protective group, or an alkoxyalkylamide-type protective group, preferably a phenyl group, a t-Bu group, an allyl group, a benzyl group, a 4-methoxybenzyl group, a trityl group, a cumyl group, a methoxymethyl group, or a benzyloxy-methyl group, and more preferably phenyl, and the 2 to 13 amino acid residues are one or more selected from glycine, alanine, isoleucine, leucine, valine, methionine, phenylalanine, tyrosine, proline, N-methylglycine, N-methylalanine, N-methylisoleucine, N-methylleucine, N-methylvaline, N-methylmethionine, N-methylphenylalanine, N-methyltyrosine, and N-methylproline.
[39] The method according to any of [1] to [38], wherein n is an integer of 1 or 2.
[40] The method according to any of [1] to [39], wherein n is 2.
[41] The method according to any of [1] to [40], wherein X is optionally substituted C₁-C₃ alkylene.
[42] The method according to any of [1] to [41], wherein X is C₁-C₃ alkylene.
[43] The method according to any of [1] to [42], wherein X is methylene.
   [43-1] The method according to any of [1] to [42], wherein X is methylene, and n is an integer of 1 or 2.
   [43-2] The method according to any of [1] to [42], wherein X is methylene, and n is 2.
[44] The method according to any of [1] to [43], wherein X¹ is one selected from the group consisting of an Fmoc group, a Cbz group, a Troc group, an Alloc group, a Teoc group, a TSoc group, a BIBSoc group, an IPCSoc group, a BBSoc group, a CHBSoc group, a CDBSoc group, and a Boc group.
[45] The method according to any of [1] to [44], wherein X¹ is one selected from the group consisting of an Fmoc group, a Cbz group, and a Boc group.
[46] The method according to any of [1] to [45], wherein X¹ is a Fmoc group.
[47] The method according to any of [32] to [46], wherein X² is one selected from the group consisting of an Fmoc group, a Cbz group, a Troc group, an Alloc group, a Teoc group, a TSoc group, a BIBSoc group, an IPCSoc group, a BBSoc group, a CHBSoc group, a CDBSoc group, and a Boc group.
[48] The method according to any of [32] to [47], wherein X² is one selected from the group consisting of an Fmoc group, a Cbz group, and a Boc group.
[49] The method according to any of [32] to [48], wherein X² is an Fmoc group.
[50] A method for producing a cyclic peptide compound or a salt thereof, comprising the step set forth in any of [1] to [49], wherein the cyclic peptide compound or a salt thereof contains, as a partial structure, residues of the peptide produced in the step set forth in any of [1] to [49].
[51] The method according to [50], wherein the cyclic peptide compound or a salt thereof contains 8 to 20 amino acid residues.
[52] The method according to [50] or [51], wherein the cyclic peptide compound or a salt thereof contains 11 to 14 amino acid residues.
[53] The method according to any of [50] to [52], wherein the cyclic peptide compound contains at least one non-natural amino acid residue.
[54] The method according to any of [50] to [53], wherein the cyclic peptide compound contains at least four non-natural amino acid residues.
[55] The method according to any of [50] to [54], wherein the cyclic peptide compound contains at least five non-natural amino acid residues.
[56] The method according to any of [50] to [55], wherein the cyclic peptide compound is produced by amide cyclization of an N-terminal amino group and a C-terminal carboxy group of a peptide compound.
[57] The method according to any of [50] to [55], wherein the cyclic peptide compound is produced by thioether cyclization of N-terminal chloroacetyl and a C-terminal cysteine side chain of a peptide compound.
[58] The method according to any of [53] to [57], wherein the non-natural amino acid is an N-methylamino acid residue.
[59] The method according to any of [50] to [58], wherein the cyclic peptide compound produced is (1S,4S,10S,13S,17S,20S,26S,28R,32S,38S,42Z)-20-cyclopentyl-28-ethoxy-32-[2-[3-methoxy-4-(trifluoromethyl)phenyl]ethyl]-N,N,2,14,18,21,24,36-octamethyl-10-[(1S)-1-methylpropyl]-3,9,12,15,19,22,25,31,34,37,45-undecaoxo-13-propyl-38-[[4-(trifluoromethyl)phenyl]methyl]spiro[2,8,11,14,18,21,24,30,33,36,39-undecazatetracyclo[37.5.1.0^{4,8}.0^{26,30}]pentatetracont-42-ene-23,1'-cyclobutane]-17-carboxamide.
[60] (1S,4S,10S,13S,17S,20S,26S,28R,32S,38S,42Z)-20-cyclopentyl-28-ethoxy-32-[2-[3-methoxy-4-(trifluoromethyl)phenyl]ethyl]-N,N,2,14,18,21,24,36-octamethyl-10-[(1S)-1-methylpropyl]-3,9,12,15,19,22,25,31,34,37,45-undecaoxo-13-propyl-38-[[4-(trifluoromethyl)phenyl]methyl]spiro[2,8,11,14,18,21,24,30,33,36,39-undecazatetracyclo[37.5.1.0^{4,8}.0^{26,30}]pentatetracont-42-ene-23,1'-cyclobutane]-17-carboxamide produced by the method according to any of [50] to [58].
[61] A compound represented by formula (1a) or a salt thereof: 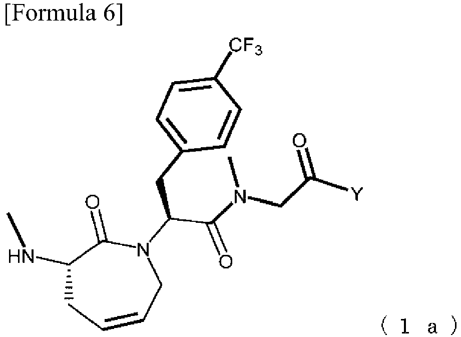 wherein Y is a hydroxyl group, optionally substituted C₁-C₁₀ alkoxy, optionally substituted C₆-C₁₆ aryloxy, optionally substituted C₇-C₁₄ alkoxy, or optionally substituted 3- to 12-membered cyclic aminooxy.
[62] The compound or a salt thereof according to [61], wherein Y is tert-butoxy.
[63] tert-Butyl N-methyl-N-((S)-2-((S)-3-(methylamino)-2-oxo-2,3,4,7-tetrahydro-1H-azepin-1-yl)-3-(4-(trifluoromethyl)phenyl)propanoyl)glycinate or a hydrochloride thereof.
[64] A compound represented by formula (1b) or a salt thereof: 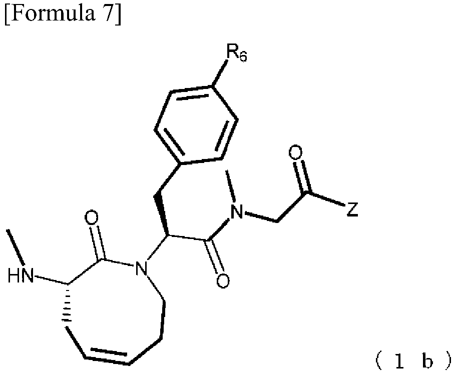
   wherein R⁶ is methyl or trifluoromethyl, and
   Z is a hydroxyl group, optionally substituted C₁-C₁₀ alkoxy, optionally substituted C₆-C₁₆ aryloxy, optionally substituted C₇-C₁₄ alkoxy, or optionally substituted 3- to 12-membered cyclic aminooxy.
[65] The compound or a salt thereof according to [64], wherein Z is tert-butoxy.
[66] The compound or a salt thereof according to [64] or [65], wherein R⁶ is methyl.
[67] The compound or a salt thereof according to [64] or [65], wherein R⁶ is trifluoromethyl.
[68] tert-Butyl N-methyl-N-((S)-2-((S,Z)-3-(methylamino)-2-oxo-3,4,7,8-tetrahydroazocin-1(2H)-yl)-3-(p-tolyl)propanoyl)glycinate or a hydrochloride thereof.
[69] tert-Butyl N-methyl-N-((S)-2-((S,Z)-3-(methylamino)-2-oxo-3,4,7,8-tetrahydroazocin-1(2H)-yl)-3-(4-(trifluoromethyl)phenyl)propanoyl)glycinate or a hydrochloride thereof.
[70] tert-Butyl N-methyl-N-((S)-2-((S,Z)-3-(methylamino)-2-oxo-2,3,4,5,8,9-hexahydro-1H-azonin-1-yl)-3-(p-tolyl)propanoyl)glycinate or a hydrochloride thereof.
[71] (S)-N,N-dimethyl-2-((S,Z)-3-(methylamino)-2-oxo-3,4,7,8-tetrahydroazocin-1(2H)-yl)-3-(4-(trifluoromethyl)phenyl)propaneamide or a hydrochloride thereof.
[72] The method for producing a compound represented by formula (1) or a salt thereof according to [1], the method comprising the step (metathesis step) of bringing a compound represented by formula (2) or a salt thereof into contact with a catalyst, wherein formation of an impurity is suppressed as compared to a case where R¹ in formulae (1) and (2) is a carbamate protective group.
[73] The method for producing a compound represented by formula (1) or a salt thereof according to [1], the method comprising the step (metathesis step) of bringing a compound represented by formula (2) or a salt thereof into contact with a catalyst, wherein formation of a dimer is suppressed as compared to a case where R¹ in formulae (1) and (2) is a carbamate protective group.
[74] A compound represented by formula (1) or a salt thereof, wherein a purity of the compound represented by formula (1) or a salt thereof is 90% or more, preferably 95% or more, more preferably 98% or more, and most preferably 99% or more as determined by a UVArea value at 210 nm from HPLC analysis.
[75] A compound represented by formula (1) or a salt thereof, wherein a content ratio of an impurity contained in the compound represented by formula (1) or a salt thereof is less than 10%, preferably less than 5%, more preferably less than 1%, still more preferably less than 0.5%, and most preferably an undetectable level as determined by a UVArea value at 210 nm from HPLC analysis.

In the above numberings, the number cited in the dependent item includes the branch number of the number, unless otherwise mentioned. For example, the [31] cited in the dependent item shows that not only [31] but also its branch numbers [31-1], [31-2] and [31-3] are included. The same applies to other numberings.

### [Advantageous Effects of Invention]

According to the present invention, a peptide compound having a peptide structure with a medium ring can be efficiently produced. The production method of the present invention enables reducing costs in production of peptide compounds and also reducing the environmental loads, thereby the production method of the present invention is particularly useful for the synthesis of peptides on a large scale.

### [Description of Embodiments]

### Abbreviations

The abbreviations used herein are listed below.
MTBE: methyl tert-butyl ether
HFIP: 1,1,1,3,3,3-hexafluoro-2-propanol
MeTHF: 2-methyltetrahydrofuran
DBU: 1,8-diazabicyclo[5.4.0]-7-undecene
TFE: 2,2,2-trifluoroethanol
TCFH: chloro-N,N,N',N'-tetramethylformamidinium hexafluorophosphate
DIPEA: diisopropylethylamine
Fmoc: 9-fluorenylmethyloxycarbonyl
Cbz: benzyloxycarbonyl
Troc: 2,2,2-trichloroethoxycarbonyl
Alloc: allyloxycarbonyl
Teoc: 2-(trimethylsilyl)ethoxycarbonyl
TSoc: triisopropylsilyloxycarbonyl
BIBSoc: di-t-butylisobutylsilyloxycarbonyl
IPCSoc: di-i-propyl-t-butylsilyloxycarbonyl
BBSoc: benzyl-di-t-butylsilyloxycarbonyl
CHBSoc: di-t-butylcyclohexylsilyloxycarbonyl
CDBSoc: t-butyloctadecylsilyloxycarbonyl
Boc: t-butoxycarbonyl

### Definitions of functional groups and the like (The terms explained below are illustrative and not intended to be particularly limiting, and are those that can be commonly understood by those skilled in the art.)

The "halogen" as used herein include fluorine, chlorine, bromine, and iodine. As used herein, F refers to fluorine, Cl refers to chlorine, Br refers to bromine, and I refers to iodine. Examples of the halogen include fluorine, chlorine and bromine, and preferably fluorine and chlorine.

The "alkyl" as used herein is a linear or branched monovalent saturated hydrocarbon group derived from an aliphatic saturated hydrocarbon by removing any one hydrogen atom, the group having a subset of a hydrocarbyl or hydrocarbon group structure containing hydrogen and carbon atoms without containing a heteroatom (which refers to an atom other than carbon and hydrogen atoms) or an unsaturated carbon-carbon bond in the skeleton. The alkyl includes not only a linear alkyl but also a branched alkyl. Specifically, the alkyl is an alkyl having 1 to 20 carbon atoms (C₁-C₂₀, wherein "Cₚ-C_{q}" means that the number of carbon atoms is from p to q), preferably C₁-C₁₀ alkyl, more preferably C₁-C₆ alkyl. Specific examples of the alkyl include methyl, ethyl, n-propyl, i-propyl, n-butyl, s-butyl, t-butyl, isobutyl (2-methylpropyl), n-pentyl, s-pentyl (1-methylbutyl), t-pentyl (1,1-dimethylpropyl), neopentyl (2,2-dimethylpropyl), isopentyl (3-methylbutyl), 3-pentyl (1-ethylpropyl), 1,2-dimethylpropyl, 2-methylbutyl, n-hexyl, 1,1,2-trimethylpropyl, 1,2,2-trimethylpropyl, 1,1,2,2-tetramethylpropyl, 1,1-dimethylbutyl, 1,2-dimethylbutyl, 1,3-dimethylbutyl, 2,2-dimethylbutyl, 2,3-dimethylbutyl, 3,3-dimethylbutyl, 1-ethylbutyl, and 2-ethylbutyl. Specific examples of the linear C₁-C₆ alkyl include methyl, ethyl, n-propyl, n-butyl, n-pentyl, and n-hexyl. Specific examples of the linear C₁-C₃ alkyl include methyl, ethyl, and n-propyl. Specific examples of the branched C₃-C₆ alkyl include i-propyl, s-butyl, t-butyl, isobutyl (2-methylpropyl), s-pentyl (1-methylbutyl), t-pentyl (1,1-dimethylpropyl), neopentyl (2,2-dimethylpropyl), isopentyl (3-methylbutyl), 3-pentyl (1-ethylpropyl), 1,2-dimethylpropyl, 2-methylbutyl, 1,1,2-trimethylpropyl, 1,2,2-trimethylpropyl, 1,1,2,2-tetramethylpropyl, 1,1-dimethylbutyl, 1,2-dimethylbutyl, 1,3-dimethylbutyl, 2,2-dimethylbutyl, 2,3-dimethylbutyl, 3,3-dimethylbutyl, 1-ethylbutyl, and 2-ethylbutyl.

The "haloalkyl" as used herein is a group in which one or more hydrogen atoms of the "alkyl" as defined herein are substituted with halogen. The haloalkyl is preferably a group in which one or more and six or less hydrogen atoms that are allowed to be substituted of the hydrogen atoms of the alkyl are substituted with halogen. The haloalkyl is, for example, halo-C₁-C₂₀ alkyl, preferably halo-C₁-C₁₀ alkyl, more preferably halo-C₁-C₈ alkyl, still more preferably halo-C₁-C₆ alkyl, most preferably halo-C₁-C₃ alkyl. The halo-C₁-C₆ alkyl is, for example, a group in which one or more and six or less hydrogen atoms allowed to be substituted of the hydrogen atoms of the alkyl are substituted with fluorine, preferably a group in which one or more and five or less hydrogen atoms are substituted with fluorine, more preferably a group in which one or more and four or less hydrogen atoms are substituted with fluorine, most preferably a group in which one or more and three or less hydrogen atoms are substituted with fluorine. Specific examples of the haloalkyl include difluoromethyl, trifluoromethyl, 2,2-difluoroethyl, 2,2,2-trifluoroethyl, 3,3-difluoropropyl, 4,4-difluorobutyl, and 5,5-difluoropentyl.

The "alkenyl" as used herein is a linear or branched monovalent unsaturated hydrocarbon group having one or more carbon-carbon double bonds (bonds by two adjacent sp2 carbon atoms). Depending on the conformation of the atoms or groups of atoms attached to the sp2 carbon atoms, the geometric morphology of the double bond can assume entgegen (E) or zusammen (Z) and cis or trans conformations. The alkenyl is, for example, C₂-C₁₀ alkenyl, preferably C₂-C₈ alkenyl, more preferably C₂-C₇ alkenyl, most preferably C₂-C₆ alkenyl. Specific examples of the alkenyl include ethenyl (vinyl), 1-propenyl, 2-propenyl (allyl), isopropenyl, 1-butenyl, 2-butenyl (including cis, trans), 3-butenyl, pentenyl, and hexenyl.

The "alkynyl" as used herein is a linear or branched monovalent unsaturated hydrocarbon group having one or more carbon-carbon triple bonds (bonds by two adjacent sp carbon atoms). The alkynyl is, for example, C₂-C₁₀ alkynyl, preferably C₂-C₈ alkynyl, more preferably C₂-C₇ alkynyl, most preferably C₂-C₆ alkynyl. Specific examples of the alkynyl include ethynyl, 1-propynyl, propargyl (2-propynyl), 1-butynyl, 2-butynyl, 3-butynyl, pentynyl, and hexynyl.

The "cycloalkyl" as used herein is a saturated or partially saturated cyclic monovalent non-aromatic hydrocarbon ring group (alicyclic ring group). A carbon atom constituting the ring may be oxidated to form carbonyl. The cycloalkyl may be selected from the group consisting of a monocyclic ring, a condensed ring, and a spiro ring. As used herein, the cycloalkyl containing a monocyclic ring is referred to as monocyclic cycloalkyl or a monocyclic alicyclic ring group; the cycloalkyl containing a condensed ring is referred to as condensed cyclic cycloalkyl or a condensed cyclic alicyclic ring group; and the cycloalkyl containing a spiro ring is referred to as spirocyclic cycloalkyl or a spirocyclic alicyclic ring group. The cycloalkyl may form a condensed ring with a saturated alicyclic ring such as a cyclopentane ring or a cyclohexane ring, an unsaturated alicyclic ring such as a cyclopentene ring or a cyclohexene ring, or an aromatic hydrocarbon ring such as a benzene ring or a naphthalene ring. The cycloalkyl may form a spiro ring with a saturated alicyclic ring such as a cyclopropane ring, a cyclobutane ring, a cyclopentane ring, or a cyclohexane ring. The cycloalkyl is, for example, C₃-C₁₀ cycloalkyl, preferably C₃-C₈ cycloalkyl, more preferably C₃-C₇ cycloalkyl, most preferably C₃-C₆ cycloalkyl. Specific examples of the cycloalkyl include cyclopropyl, cyclobutyl, cyclopentyl, cyclohexyl, cycloheptyl, cyclooctyl, bicyclo[2.2.1]heptyl, spiro[3.3]heptyl, and cyclohexenyl.

The "aryl" as used herein is a monovalent aromatic hydrocarbon ring group composed of a monovalent, monocyclic or condensed ring that exhibits aromaticity. As used herein, the aryl composed of a monocyclic ring is referred to as a monocyclic aryl, and an aryl composed of a condensed ring is referred to as a condensed cyclic aryl. The aryl is, for example, C₆-C₁₄ aryl, preferably C₆ aryl, C₁₀ aryl and C₁₄ aryl, more preferably C₆ aryl and C₁₀ aryl, most preferably C₆ aryl. Specific examples of the aryl include phenyl, 1-naphthyl, 2-naphthyl, tolyl, and xylyl.

The "aralkyl (arylalkyl)" as used herein is a group in which one or more hydrogen atoms of the "alkyl" as defined herein are substituted with the "aryl" as defined herein. The aralkyl is, for example, C₇-C₂₀ aralkyl, preferably C₇-C₁₈ aralkyl, more preferably C₇-C₁₆ aralkyl, most preferably C₇-C₁₄ aralkyl. The C₇-C₂₀ aralkyl is, for example, C₆-C₁₀ aryl-C₁-C₁₀ alkyl, preferably C₆-C₁₀ aryl-C₁-C₈ alkyl, more preferably C₆ aryl-C₁-C₈ alkyl or C₁₀ aryl-C₁-C₈ alkyl, most preferably C₆ aryl-C₁-C₈ alkyl. The C₇-C₁₈ aralkyl is, for example, C₆-C₁₀ aryl-C₁-C₈ alkyl, preferably C₆-C₁₀ aryl-C₁-C₆ alkyl, more preferably C₆ aryl-C₁-C₆ alkyl or C₁₀ aryl-C₁-C₆ alkyl, most preferably C₆ aryl-C₁-C₆ alkyl. The C₇-C₁₆ aralkyl is, for example, C₆-C₁₀ aryl-C₁-C₆ alkyl, preferably C₆-C₁₀ aryl-C₁-C₄ alkyl, more preferably C₆ aryl-C₁-C₄ alkyl or C₁₀ aryl-C₁-C₄ alkyl, most preferably C₆ aryl-C₁-C₄ alkyl. The C₇-C₁₄ aralkyl is, for example, C₆-C₁₀ aryl-C₁-C₄ alkyl, preferably C₆-C₁₀ aryl-C₁-C₃ alkyl, more preferably C₆ aryl-C₁-C₃ alkyl or C₁₀ aryl-C₁-C₃ alkyl, most preferably C₆ aryl-C₁-C₃ alkyl. Specific examples of the aralkyl include benzyl, phenethyl, and 3-phenylpropyl.

The "heterocyclyl" as used herein is a heterocyclic group that contains, as atoms constituting the ring other than carbon atoms, preferably 1 to 5 hetero atoms, more preferably 1 to 3 hetero atoms selected from the group consisting of nitrogen atoms, oxygen atoms and sulfur atoms, and that may have a double and/or triple bond in the ring. A carbon atom in the ring of the heterocyclyl may be oxidated to form carbonyl. As used herein, the heterocyclyl containing a monocyclic ring is referred to as a monocyclic heterocyclyl; the heterocyclyl containing a condensed ring is referred to as a condensed cyclic heterocyclyl; and the heterocyclyl containing a spiro ring is referred to as a spirocyclic heterocyclyl. The heterocyclyl may form a condensed ring or a spiro ring with a saturated alicyclic ring such as a cyclopentane ring or a cyclohexane ring, or a saturated heterocycle such as a tetrahydropyran ring, a dioxane ring, or a pyrrolidine ring. The number of atoms constituting the ring of heterocyclyl is, for example, 3 to 14 (3- to 14-membered heterocyclyl), preferably 3 to 12 (3- to 12-membered heterocyclyl), more preferably 3 to 10 (3- to 10-membered heterocyclyl), most preferably 4 to 7 (4- to 7-membered heterocyclyl). Specific examples of the heterocyclyl include azetidinyl, oxiranyl, oxetanyl, thietanyl, tetrahydrofuranyl, pyrrolidinyl, pyrazolidinyl, imidazolidinyl, oxazolidinyl, isoxazolidinyl, thiazolidinyl, isothiazolidinyl, thiadiazolidinyl, oxazolidinyl, dioxolanyl, tetrahydropyranyl, morpholinyl, thiomorpholinyl, 4-oxopyrrolidinyl, piperidinyl, 4-oxopiperidinyl, piperazinyl, and dioxanyl, and rings in which one or more single bonds in these saturated heterocycles are replaced with a double bond or a triple bond.

The "heteroaryl" as used herein is a monovalent aromatic heterocyclic group that contains at least one heteroatom in addition to the carbon atom and is composed of a monocyclic or condensed ring that exhibits aromaticity. As used herein, a heteroaryl composed of a monocyclic ring is referred to as a monocyclic heteroaryl, and a heteroaryl composed of a condensed ring is referred to as a condensed cyclic heteroaryl. The number of atoms constituting the ring of heteroaryl is, for example, 5 to 14 (5- to 14-membered heteroaryl), preferably 5 to 13 (5- to 13-membered heteroaryl), more preferably 5 to 10 (5- to 10-membered heteroaryl), most preferably 5 to 7 (5- to 7-membered heteroaryl). Specific examples of the heteroaryl include 5-membered heteroaryl such as furyl, thienyl, pyrrolyl, imidazolyl, pyrazolyl, thiazolyl, oxazolyl, triazolyl, or tetrazolyl; 6-membered heteroaryl such as pyridyl, pyrimidyl, pyridazinyl, pyrazinyl, or triazinyl; 9-membered heteroaryl such as benzofuranyl, benzothienyl, benzothiazolyl, benzimidazolyl, benzotriazolyl, indolyl, indazolyl, or pyrazolopyridyl; and 10-membered heteroaryl such as quinolyl, isoquinolyl, cinolinyl, quinazolinyl, or quinoxalinyl.

The "alkoxy" as used herein is a group in which "alkyl" as defined herein is attached to an oxygen atom (-OR, wherein R is alkyl). The alkoxy is, for example, C₁-C₂₀ alkoxy, preferably C₁-C₁₀ alkoxy, more preferably C₁-C₈ alkoxy, most preferably C₁-C₆ alkoxy. Specific examples of the alkoxy include methoxy, ethoxy, 1-propoxy, 2-propoxy, n-butoxy, i-butoxy, sec-butoxy, tert-butoxy, pentyloxy, and 3-methylbutoxy.

The "alkylsulfanyl" as used herein is a group in which "alkyl" as defined herein is attached to a sulfur atom (-SR, wherein R is alkyl). This is also called alkylthio. The alkylsulfanyl is, for example, C₁-C₂₀ alkylsulfanyl, preferably C₁-C₁₀ alkylsulfanyl, more preferably C₁-C₈ alkylsulfanyl, and most preferably C₁-C₆ alkylsulfanyl. Specific examples of the alkylsulfanyl include methylsulfanyl, ethylsulfanyl, 1-propylsulfanyl, 2-propylsulfanyl, n-butylsulfanyl, i-butylsulfanyl, s-butylsulfanyl, t-butylsulfanyl, pentylsulfanyl, and 3-methylbutylsulfanyl.

The "alkylsulfinyl" as used herein is a sulfinyl group to which "alkyl" as defined herein is attached (-S(=O)-R, wherein R is alkyl). The alkylsulfinyl is, for example, C₁-C₂₀ alkylsulfinyl, preferably C₁-C₁₀ alkylsulfinyl, more preferably C₁-C₈ alkylsulfinyl, and most preferably C₁-C₆ alkylsulfinyl. Specific examples of the alkylsulfinyl include methylsulfinyl, ethylsulfinyl, 1-propylsulfinyl, 2-propylsulfinyl, n-butylsulfinyl, i-butylsulfinyl, s-butylsulfinyl, t-butylsulfinyl, pentylsulfinyl, and 3-methylbutylsulfinyl.

The "alkylsulfonyl" as used herein is a sulfonyl group to which "alkyl" as defined herein is attached (-S(=O)₂-R, wherein R is alkyl). The alkylsulfonyl is, for example, C₁-C₂₀ alkylsulfonyl, preferably C₁-C₁₀ alkylsulfonyl, more preferably C₁-C₈ alkylsulfonyl, most preferably C₁-C₆ alkylsulfonyl. Specific examples of the alkylsulfinyl include methylsulfonyl, ethylsulfonyl, 1-propylsulfonyl, 2-propylsulfonyl, n-butylsulfonyl, i-butylsulfonyl, s-butylsulfonyl, t-butylsulfonyl, pentylsulfonyl, and 3-methylbutylsulfonyl.

The "carboxyalkyl" as used herein is a group in which one or more hydrogen atoms of the "alkyl" as defined herein are substituted with "carboxy". The carboxyalkyl is preferably a group in which one hydrogen atom of the alkyl is substituted with carboxy. The carboxyalkyl is, for example, carboxy C₁-C₂₀ alkyl, preferably carboxy C₁-C₁₅ alkyl, more preferably carboxy C₁-C₁₀ alkyl, most preferably carboxy C₁-C₆ alkyl. Specific examples of the carboxyalkyl include carboxymethyl and carboxyethyl.

The "aralkoxy" as used herein is a group (-O-R-Ar, wherein R is alkylene) in which an alkyl moiety in "aralkyl" as defined herein is attached to an oxygen atom. The aralkoxy is, for example, C₇-C₂₀ aralkoxy, preferably C₇-C₁₈ aralkoxy, more preferably C₇-C₁₆ aralkoxy, most preferably C₇-C₁₄ aralkoxy. The C₇-C₂₀ aralkoxy is, for example, C₆-C₁₀ aryl-C₁-C₁₀ alkoxy, preferably C₆-C₁₀ aryl-C₁-C₈ alkoxy, more preferably C₆ aryl-C₁-C₈ alkoxy or C₁₀ aryl-C₁-C₈ alkoxy, most preferably C₆ aryl-C₁-C₈ alkoxy. The C₇-C₁₈ aralkoxy is, for example, C₆-C₁₀ aryl-C₁-C₈ alkoxy, preferably C₆-C₁₀ aryl-C₁-C₆ alkoxy, more preferably C₆ aryl-C₁-C₆ alkoxy or C₁₀ aryl-C₁-C₆ alkoxy, most preferably C₆ aryl-C₁-C₆ alkoxy. The C₇-C₁₆ aralkoxy is, for example, C₆-C₁₀ aryl-C₁-C₆ alkoxy, preferably C₆-C₁₀ aryl-C₁-C₄ alkoxy, more preferably C₆ aryl-C₁-C₄ alkoxy or C₁₀ aryl-C₁-C₄ alkoxy, most preferably C₆ aryl-C₁-C₄ alkoxy. The C₇-C₁₄ aralkoxy is, for example, C₆-C₁₀ aryl-C₁-C₄ alkoxy, preferably C₆-C₁₀ aryl-C₁-C₃ alkoxy, more preferably C₆ aryl-C₁-C₃ alkoxy or C₁₀ aryl-C₁-C₃ alkoxy, most preferably C₆ aryl-C₁-C₃ alkoxy. Specific examples of the aralkoxy include benzyloxy, phenethyloxy, and 3-phenylpropoxy.

The "aralkoxyalkyl" as used herein means a group in which one or more hydrogen atoms of the "alkyl" as defined herein are substituted with the "aralkoxy" as defined herein. The aralkoxyalkyl is preferably C₇-C₁₄ aralkoxy-C₁-C₆ alkyl, more preferably C₇-C₁₄ aralkoxy-C₁-C₂ alkyl. Specific examples of the aralkoxyalkyl include benzyloxymethyl and 1-(benzyloxy)ethyl.

The "cycloalkoxy" as used herein is a group (-OR, wherein R is cycloalkyl) in which "cycloalkyl" as defined herein is attached to an oxygen atom. The cycloalkoxy is, for example, C₃-C₁₀ cycloalkoxy, preferably C₃-C₈ cycloalkoxy, more preferably C₃-C₇ cycloalkoxy, most preferably C₃-C₆ cycloalkoxy. Specific examples of the cycloalkoxy include cyclopropoxy, cyclobutoxy, and cyclopentyloxy.

The "aryloxy" as used herein is a group (-OAr, wherein Ar is aryl) in which "aryl" as defined herein is attached to an oxygen atom. The aryloxy is, for example, C₆-C₁₄ aryloxy, preferably C₆ aryloxy, C₁₀ aryloxy and C₁₄ aryloxy, more preferably C₆ aryloxy and C₁₀ aryloxy, most preferably C₆ aryloxy. Specific examples of the aryloxy include phenoxy, 1-naphthyloxy, 2-naphthyloxy, tolyloxy, and xylyloxy.

The "amino" as used herein means -NRR' wherein N represents a nitrogen atom and R and R' are each independently selected from the group consisting of a hydrogen atom, alkyl, alkenyl, alkynyl, cycloalkyl, heterocyclyl, aryl, and heteroaryl, or R and R' form a ring together with the nitrogen atom to which they are attached. Examples of the amino include -NH₂, mono-C₁-C₆ alkylamino, di-C₁-C₆ alkylamino, N-C₁-C₆ alkyl-N-C₂-C₆ alkenylamino, N-C₁-C₆ alkyl-N-C₁-C₆ alkoxy-C₁-C₆ alkylamino, and 4- to 9-membered cyclic amino.

The "monoalkylamino" as used herein means a group of the "amino (-NRR')" as defined herein, in which R is a hydrogen atom and R' is the "alkyl". The monoalkylamino is, for example, mono-C₁-C₂₀ alkylamino, preferably mono-C₁-C₁₅ alkylamino, more preferably mono-C₁-C₁₀ alkylamino, most preferably mono-C₁-C₆ alkylamino. Specific examples of the monoalkylamino include methylamino, ethylamino, n-propylamino, i-propylamino, n-butylamino, s-butylamino, and t-butylamino.

The "dialkylamino" as used herein means a group of the "amino (-NRR')" as defined herein, in which R and R' are each independently the "alkyl". The dialkylamino is, for example, di-C₁-C₂₀ alkylamino, preferably di-C₁-C₁₅ alkylamino, more preferably di-C₁-C₁₀ alkylamino, most preferably di-C₁-C₆ alkylamino. Specific examples of the dialkylamino include dimethylamino, diethylamino, and methyl ethylamino.

The "cyclic amino" as used herein is a group of the "amino (-NRR')" as defined herein, in which R and R' form a ring together with the nitrogen atom to which they are attached. The cyclic amino is, for example, 3- to 14-membered cyclic amino, preferably 3- to 12-membered cyclic amino, more preferably 3- to 10-membered cyclic amino, still more preferably 4- to 9-membered cyclic amino, and most preferably 4- to 7-membered cyclic amino. Specific examples of the cyclic amino include 1-azetidyl, 1-pyrrolidyl, 1-piperidyl, 1-piperazyl, 4-morpholinyl, 3-oxazolidyl, 1,1-dioxidothiomorpholinyl-4-yl, and 3-oxa-8-azabicyclo[3.2.1]octan-8-yl.

The "cyclic aminooxy" as used herein is a group in which "cyclic amino" as defined herein is attached to an oxygen atom (-OR (R is cyclic amino)). The cyclic amino is, for example, 3- to 14-membered cyclic aminooxy, preferably 3- to 12-membered cyclic aminooxy, more preferably 3- to 10-membered cyclic aminooxy, still more preferably 4- to 9-membered cyclic aminooxy, and most preferably 4- to 7-membered cyclic aminooxy. Specific examples of the cyclic aminooxy include 1-azetidyloxy, 1-pyrrolidyloxy, 1-piperidyloxy, 1-piperazyloxy, 4-morpholinyloxy, 3-oxazolidyloxy, 1,1-dioxidothiomorpholinyl-4-yloxy, and 3-oxa-8-azabicyclo[3.2.1]octan-8-yloxy.

The "aminocarbonyl" as used herein is a group in which an "amino" as defined herein is attached to a carbon atom of a carbonyl. It is also sometimes referred to as amide. The aminocarbonyl is, for example, -CONH₂, mono-C₁-C₆ alkylaminocarbonyl, di-C₁-C₆ alkylaminocarbonyl, N-C₁-C₆ alkyl-N-C₂-C₆ alkenylaminocarbonyl, N-C₁-C₆ alkyl-N-C₁-C₆ alkoxy-C₁-C₆ alkylaminocarbonyl, and 4- to 9-membered cyclic aminocarbonyl. Specific examples of the aminocarbonyl include -CONH₂, methylaminocarbonyl, ethylaminocarbonyl, dimethylaminocarbonyl, diethylaminocarbonyl, 1-azetidinylcarbonyl, 1-pyrrolidinylcarbonyl, 1-piperidinylcarbonyl, 1-piperazinylcarbonyl, 4-morpholinylcarbonyl, and 3-oxazolidinylcarbonyl.

The "aminoalkyl" as used herein is a group in which one or more hydrogen atoms of the "alkyl" as defined herein are substituted with amino. The aminoalkyl is preferably a group in which one hydrogen atom of the alkyl is substituted with amino. The aminoalkyl is, for example, amino C₁-C₂₀ alkyl, preferably amino C₁-C₁₀ alkyl, more preferably amino C₁-C₈ alkyl, most preferably amino C₁-C₆ alkyl. Specific examples of the aminoalkyl include aminomethyl, aminoethyl, 4-aminobutyl, methylaminomethyl, dimethylaminomethyl, methylaminoethyl, and dimethylaminoethyl.

The "cycloalkylalkyl" as used herein is a group in which one or more hydrogen atoms of the "alkyl" as defined herein are substituted with "cycloalkyl". The cycloalkylalkyl is preferably a group in which one hydrogen atom of the alkyl is substituted with cycloalkyl. The cycloalkylalkyl is, for example, C₃-C₁₀ cycloalkyl-C₁-C₂₀ alkyl, preferably C₃-C₁₀ cycloalkyl-C₁-C₆ alkyl, more preferably C₃-C₈ cycloalkyl-C₁-C₆ alkyl, most preferably C₃-C₆ cycloalkyl-C₁-C₂ alkyl. Specific examples of the cycloalkylalkyl include cyclopropylmethyl, cyclobutylmethyl, cyclopentylmethyl, cyclohexylmethyl, cyclohexylethyl, and cyclohexylpropyl.

The "cycloalkoxyalkyl" as used herein is a group in which one or more hydrogen atoms of the "alkyl" as defined herein are substituted with "cycloalkoxy". The cycloalkoxyalkyl is preferably a group in which one hydrogen atom of the alkyl is substituted with cycloalkoxy. The cycloalkoxyalkyl is, for example, C₃-C₁₀ cycloalkoxy-C₁-C₆ alkyl, preferably C₃-C₈ cycloalkoxy-C₁-C₆ alkyl, more preferably C₃-C₇ cycloalkoxy-C₁-C₆ alkyl, most preferably C₃-C₆ cycloalkoxy-C₁-C₆ alkyl. Specific examples of the cycloalkoxyalkyl include cyclopropoxymethyl, cyclobutoxymethyl, and cyclopentyloxymethyl.

The "heterocyclylalkyl" as used herein is a group in which one or more hydrogen atoms of the "alkyl" as defined herein are substituted with "heterocyclyl". The heterocyclylalkyl is preferably a group in which one hydrogen atom of the alkyl is substituted with heterocyclyl. The heterocyclylalkyl is, for example, 3- to 14-membered heterocyclyl-C₁-C₆ alkyl, preferably 3-to 12-membered heterocyclyl-C₁-C₆ alkyl, more preferably 3- to 10-membered heterocyclyl-C₁-C₄ alkyl, most preferably 4- to 7-membered heterocyclyl-C₁-C₃ alkyl. Specific examples of the heterocyclylalkyl include azetidin-1-ylmethyl, oxetan-3-ylmethyl, 2-(tetrahydrofuran-3-yl)ethyl, (1-methylpyrrolidin-3-yl)methyl, 2-morpholinoethyl, 3-(1-piperidinyl)propyl, and 3-(4-methylpiperazin-1-yl)propyl.

The "alkylsulfanylalkyl" as used herein is a group in which one or more hydrogen atoms of the "alkyl" as defined herein are substituted with "alkylsulfanyl". The alkylsulfanylalkyl is preferably a group in which one hydrogen atom of the alkyl is substituted with alkylsulfanyl. The alkylsulfonylalkyl is, for example, C₁-C₂₀ alkylsulfanyl-C₁-C₄ alkyl, preferably C₁-C₁₀ alkylsulfanyl-C₁-C₄ alkyl, more preferably C₁-C₈ alkylsulfanyl-C₁-C₄ alkyl, most preferably C₁-C₆ alkylsulfanyl-C₁-C₆ alkyl. Specific examples of the alkylsulfanylalkyl include methylsulfanylmethyl, ethylsulfanylmethyl, 1-propylsulfanylmethyl, 2-propylsulfanylmethyl, n-butylsulfanylmethyl, i-butylsulfanylmethyl, s-butylsulfanylmethyl, and t-butylsulfanylmethyl.

The "alkylsulfinylalkyl" as used herein is a group in which one or more hydrogen atoms of the "alkyl" as defined herein are substituted with "alkylsulfinyl". The alkylsulfenylalkyl is preferably a group in which one hydrogen atom of the alkyl is substituted with alkylsulfinyl. The alkylsulfinylalkyl is, for example, C₁-C₂₀ alkylsulfinyl-C₁-C₄ alkyl, preferably C₁-C₁₀ alkylsulfinyl-C₁-C₄ alkyl, more preferably C₁-C₈ alkylsulfinyl-C₁-C₄ alkyl, most preferably C₁-C₆ alkylsulfinyl-C₁-C₆ alkyl. Specific examples of the alkylsulfinylalkyl include methylsulfinylmethyl, ethylsulfinylmethyl, 1-propylsulfinylmethyl, 2-propylsulfinylmethyl, n-butylsulfinylmethyl, i-butylsulfinylmethyl, s-butylsulfinylmethyl, and t-butylsulfinylmethyl.

The "heteroarylalkyl" as used herein is a group in which one or more hydrogen atoms of the "alkyl" as defined herein are substituted with "heteroaryl". The heteroarylalkyl is preferably a group in which one hydrogen atom of the alkyl is substituted with heteroaryl. The heteroarylalkyl is, for example, 5- to 10-membered heteroaryl-C₁-C₆ alkyl, preferably 5- to 10-membered heteroaryl-C₁-C₄ alkyl, more preferably 5- to 10-membered heteroaryl-C₁-C₃ alkyl, most preferably 5- to 10-membered heteroaryl-C₁-C₂ alkyl. Specific examples of the heteroarylalkyl include 2-pyridylmethyl, 3-pyridylmethyl, 4-pyridylmethyl, 2-furanylmethyl, 2-thienylmethyl, 3-thienylmethyl, and 4-thiazolylmethyl.

The "heteroarylalkoxy" as used herein is a group in which an alkyl moiety in the "heteroarylalkyl" as defined herein is attached to an oxygen atom. The heteroarylalkoxy is, for example, 5- to 10-membered heteroaryl-C₁-C₆ alkoxy, preferably 5- to 10-membered heteroaryl-C₁-C₄ alkoxy, more preferably 5- to 10-membered heteroaryl-C₁-C₃ alkoxy, most preferably 5-to 10-membered heteroaryl-C₁-C₂ alkoxy. Specific examples of the heteroarylalkoxy include 2-pyridylmethoxy, 3-pyridylmethoxy, 4-pyridylmethoxy, 2-furanylmethoxy, 2-thienylmethoxy, 3-thienylmethoxy, and 4-thiazolylmethoxy.

The "alkoxyalkyl" as used herein is a group in which one or more hydrogen atoms of the "alkyl" as defined herein are substituted with "alkoxy". The alkoxyalkyl is preferably a group in which one hydrogen atom of the alkyl is substituted with alkoxy. The alkoxyalkyl is, for example, C₁-C₆ alkoxy-C₁-C₂₀ alkyl, preferably C₁-C₆ alkoxy-C₁-C₁₅ alkyl, more preferably C₁-C₆ alkoxy-C₁-C₁₀ alkyl, most preferably C₁-C₆ alkoxy-C₁-C₆ alkyl. Specific examples of the alkoxyalkyl include methoxymethyl, ethoxymethyl, 1-propoxymethyl, 2-propoxymethyl, n-butoxymethyl, i-butoxymethyl, s-butoxymethyl, t-butoxymethyl, pentyloxymethyl, 3-methylbutoxymethyl, 1-methoxyethyl, 2-methoxyethyl, and 2-ethoxyethyl.

The "heteroarylalkoxyalkyl" as used herein means a group in which one or more hydrogen atoms of the "alkyl" as defined herein are substituted with the "heteroarylalkoxy" as defined herein. The heteroarylalkoxyalkyl is preferably a group in which one hydrogen atom of the alkyl is substituted with heteroarylalkoxy. The heteroarylalkoxyalkyl is, for example, 5- to 10-membered heteroaryl-C₁-C₆ alkoxy-C₁-C₆ alkyl, preferably 5- to 10-membered heteroaryl-C₁-C₄ alkoxy-C₁-C₆ alkyl, more preferably 5- to 10-membered heteroaryl-C₁-C₃ alkoxy-C₁-C₆ alkyl, most preferably 5- to 10-membered heteroaryl-C₁-C₂ alkoxy-C₁-C₆ alkyl. Specific examples of the heteroarylalkoxyalkyl include 2-pyridylmethoxymethyl, 3-pyridylmethoxymethyl, 4-pyridylmethoxymethyl, 2-furanylmethoxymethyl, 2-thienylmethoxymethyl, 3-thienylmethoxymethyl, and 4-thiazolylmethoxymethyl.

The "alkylene" as used herein means a divalent group derived by removing any one hydrogen atom from the "alkyl" as defined herein. The alkylene is preferably C₁-C₈ alkylene, more preferably C₁-C₃ alkylene. Examples of the alkylene specifically include -CH₂-, -(CH₂)₂-, -(CH₂)₃-, -CH(CH₃)CH₂-, -C(CH₃)₂-, -(CH₂)₄-, -CH(CH₃)CH₂CH₂-, -C(CH₃)₂CH₂-, - CH₂CH(CH₃)CH₂-, -CH₂C(CH₃)₂-, -CH₂CH₂CH(CH₃)-, -CH₂CH(CH₂CH₃)-, -(CH₂)₅-, - CH(CH₃)CH(CH₂CH₃)-, -(CH₂)₆-, -(CH₂)₇-, and -(CH₂)₈-.

The "peptide compound" as used herein means a compound wherein two or more amino acid residues are linked by an amide bond. As long as two or more amino acids are linked by an amide bond, the peptide chain may contain another bond such as an ester bond or a thioester bond. Examples of the number of residues of the amino acid contained in the peptide include 5 to 30 residues. The peptide may be linear, branched, or cyclic.

The "peptide chain" as used herein means a chain-like portion of a peptide in which two or more amino acid residues are linked by an amide bond. As long as two or more amino acids are linked by an amide bond, the peptide chain may contain another bond such as an ester bond or a thioester bond. Examples of the number of residues of the amino acid contained in the peptide chain include 5 to 30 residues.

The "optionally substituted" as used herein means that a certain group and/or certain atom may be substituted with an optional substituent and/or optional atom. That is, it encompasses both the state where the specific group and specific atom are not substituted by any optional substituent or optional atom, and the state where the specific group and/or specific atom are substituted by an optional substituent and/or optional atom. Each of the certain group and/or certain atom may be further substituted with an optional substituent and/or optional atom. The certain group and the optional substituent are not limited and may be, for example, freely selected from groups containing atoms selected from the group consisting of a hydrogen atom, a halogen atom, a carbon atom, an oxygen atom, a sulfur atom, a nitrogen atom, a boron atom, a silicon atom, and a phosphorus atom. The certain atom and the optional atom are not limited and may be, for example, freely selected from a halogen atom, a carbon atom, an oxygen atom, a sulfur atom, a nitrogen atom, a boron atom, a silicon atom, and a phosphorus atom. Examples of the optional substituent include alkyl, alkoxy, fluoroalkyl, fluoroalkoxy, oxo, aminocarbonyl, alkylsulfonyl, alkylsulfonylamino, cycloalkyl, aryl, heteroaryl, heterocyclyl, arylalkyl, heteroarylalkyl, halogen, nitro, amino, monoalkylamino, dialkylamino, cyano, carboxyl, alkoxycarbonyl, and formyl.

The term "optionally protected" as used herein means that a group may be protected by any protective group.

Examples of the "protective group for a carboxy group" as used herein include an alkyl ester-type protective group, a benzyl ester-type protective group, a substituted alkyl ester-type protective group, and an alkenyl ester-type protective group. Specific examples of the protective group for a carboxyl group include a methyl group, an ethyl group, a t-Bu group, a benzyl group, a trityl group, a cumyl group, a methoxytrityl group, a 2-(trimethylsilyl)ethyl group, a 2,2,2-trichloroethyl group, and an allyl group.

Examples of the "protective group for an amide group" as used herein include a phenylamide-type protective group, an alkylamide-type protective group, an alkenylamide-type protective group, a benzylamide-type protective group, and an alkoxyalkylamide-type protective group. Specific examples of the protective group for an amide group include a phenyl group, a t-Bu group, an allyl group, a benzyl group, a 4-methoxybenzyl group, a trityl group, a cumyl group, a methoxymethyl group, and a benzyloxymethyl group.

Examples of the "protective group for an amino group" as used herein include a carbamate protective group, an amide protective group, an aryl sulfonamide protective group, an alkylamine protective group, and an imide protective group. Specific examples of the carbamate protective group include an Fmoc group, a Troc group, a TSoc group, a BIBSoc group, an IPCSoc group, a BBSoc group, a CHBSoc group, a CDBSoc group, a Boc group, an Alloc group, a Cbz group, and a Teoc group. Examples of the amide protective group include a pentafluoropropionyl group, a trifluoroacetyl group, an acetyl group, and a benzoyl group. Examples of the arylsulfoneamide protective group include a benzenesulfonyl group, a 2-nitrobenzenesulfonyl group, a 4-nitrobenzenesulfonyl group, and a tosyl group, a nosyl group, a dinitronosyl group. Examples of the alkylamine group include a t-Bu group, and a trityl group. Examples of the imide protective group include phthaloyl group.

The "one or more" as used herein means the number of 1 or 2 or more. When the "one or more" is used in the context associated with a substituent of a group, the term means a number from one to the maximum number of substituents allowed by the group. Specifically, the "one or more" includes, for example, 1, 2, 3, 4, 5, 6, 7, 8, 9, 10, and/or a greater number.

The compound described herein or a salt thereof can be a solvate thereof. Examples of the salt of the compound include hydrochloric acid salts; hydrobromic acid salts; hydroiodic acid salts; phosphoric acid salts; phosphonic acid salts; sulfuric acid salts, sulfonic acid salts such as methanesulfonic acid salts, para-toluenesulfonic acid salts and trifluoromethanesulfonic acid salts; carboxylic acid salts such as acetic acid salts, citric acid salts, maleic acid salts, tartaric acid salts, succinic acid salts, and salicylic acid salts; alkali metal salts such as sodium salts and potassium salts; alkaline earth metal salts such as magnesium salts and calcium salts; and ammonium salts such as ammonium salts, alkylammonium salts, dialkylammonium salts, trialkylammonium salts and tetraalkylammonium salts. These salts can be produced by, for example, bringing the compound into contact with an acid or a base. The solvate as used herein means a molecular aggregate formed by a compound and a solvent, and is not particularly limited as long as it is a solvate formed by a solvent acceptable for ingestion along with administration of a medicament. Specific examples of the solvate include not only solvates formed with a single solvent such as water, alcohol (ethanol, methanol, 1-propanol, 2-propanol, or the like), or dimethyl sulfoxide, but also solvates formed with a plurality of solvents per one molecule of compound, or solvates formed with a plurality of types of solvents per one molecule of compound. For example, a solvate formed by a compound with water is called a hydrate. As the solvate of the compound of the present invention, the hydrate is preferred. Specifically, the hydrate is preferably 1 to 20 hydrates, more preferably 1 to 10 hydrates, further preferably 1 to 5 hydrates, most preferably 1 to 3 hydrates.

The "amino acid" as used herein includes a natural amino acid and a non-natural amino acid (sometimes also referred to as an amino acid derivative). The "amino acid" as used herein may mean an amino acid residue. The "natural amino acid" as used herein is any L-type amino acid selected from Gly (glycine), L-Ala (alanine), L-Ser (serine), L-Thr (threonine), L-Val (valine), L-Leu (leucine), L-Ile (isoleucine), L-Phe (phenylalanine), L-Tyr (tyrosine), L-Trp (tryptophan), L-His (histidine), L-Glu (glutamic acid), L-Asp (aspartate), L-Gln (glutamine), L-Asn (asparagine), L-Cys (cysteine), L-Met (methionine), L-Lys (lysine), L-Arg (arginine), or L-Pro (proline). The "non-natural amino acid" as used herein is an amino acid other than the natural amino acid. Examples of the non-natural amino acid include a β-amino acid, a γ-amino acid, a D-type amino acid, an N-substituted amino acid (excluding Pro), an α,α-disubstituted amino acid, and an amino acid having a side chain different from that of natural amino acids. As the amino acid herein, amino acids having any conformation are acceptable. The side chains of the amino acids are not particularly limited, and each side chain is freely selected from groups such as alkyl, alkenyl, alkynyl, aryl, heteroaryl, aralkyl, heteroaralkyl, cycloalkyl, or spiro-bonded cycloalkyl, and/or atoms such as a hydrogen atom. Each group and/or atom is further optionally substituted. In one non-limiting aspect, the amino acid as used herein may be a compound having a carboxy group and an amino group in the same molecule. Even in this case, compounds in which the nitrogen atom of the amino group and any atom of a side chain of the amino acid together form a ring, such as proline, hydroxyproline, and azetidine-2-carboxylic acid, are also included in the amino acid.

The backbone amino group of the amino acid may be unsubstituted (i.e., an NH₂ group) or may be substituted (i.e., a -NHR group wherein R represents alkyl, alkenyl, alkynyl, aryl, heteroaryl, aralkyl, or cycloalkyl optionally having a substituent, and one or two non-adjacent methylene groups in any of these groups may be substituted by an oxygen atom, a carbonyl group (-CO-), or a sulfonyl group (-SO₂-); and a carbon chain bonded to an N atom and a carbon atom at an α-position may form a ring, as in proline). The substituent of the R is selected in the same manner as in the substituent for the amino acid side chain mentioned above. In the case of a substituted backbone amino group, the R is included in the "side chain of the amino acid" as used herein. Such an amino acid having the substituted backbone amino group is referred to herein as the "N-substituted amino acid". In the present specification, examples of the "N-substituted amino acid" preferably include, but are not limited to, N-alkyl amino acids, N-C₁-C₆ alkyl amino acids, N-C₁-C₄ alkyl amino acids, and N-methyl amino acids.

The "amino acid" as used herein includes all isotopes corresponding to each. The isotope of the "amino acid" is a form in which at least one atom is replaced with an atom having the same atomic number (proton number) and a different mass number (total number of protons and neutrons) at an abundance ratio different from the natural abundance ratio. Examples of the isotope contained in the "amino acid" herein include a hydrogen atom, a carbon atom, a nitrogen atom, an oxygen atom, a phosphorus atom, a sulfur atom, a fluorine atom, and a chlorine atom, and they include ²H, ³H, ¹³C, ¹⁴C, ¹⁵N, ¹⁷O, ¹⁸O, ³²P, ³⁵S, ¹⁸F, ³⁶Cl, and the like, respectively. For the compounds as used herein, all the compounds containing any proportions of radioactive or non-radioactive isotopic elements are encompassed within the scope of the present invention.

The "liquid-phase synthesis method" as used herein is a method in which a compound is chemically reacted in a liquid phase (solution) without using a solid phase carrier to synthesize an intended compound. In the liquid-phase synthesis method for a peptide, an amino acid and a peptide which are not bound to a solid phase carrier can be used. The liquid-phase synthesis method for a peptide also includes, as a liquid-phase synthesis method, a method in which an intended peptide chain is synthesized with a functional group (tag) bound so as to solubilize a peptide in a solvent during a synthesis reaction of the peptide, and a poor solvent is added during isolation of an intended peptide to isolate the peptide as a solid with the intended peptide chain bound to the tag (which may also be called a liquid phase tag method, or a tag method). Examples of the liquid phase tag method include the following method using a hydrophobic tag (Reference Example: Y. Okada, H. Suzuki, T. Nakae, S. Fujita, H. Abe, K. Nagano, T. Yamada, N. Ebata, S. Kim and K. Chiba, Tag-Assisted Liquid-Phase Peptide Synthesis Using Hydrophobic Benzyl Alcohols as Supports, J. Org. Chem., 2013, 78, 320-327., or S. Yano et al., Molecules 2021, 26 (12), 3497).

Examples of the "halogen-derived substituent" as used herein include fluoro (-F), chloro (-Cl), bromo (-Br), and iodo (-I).

Examples of the "oxygen atom-containing substituent" as used herein include hydroxy (-OH), oxy (-OR), oxo (=O), carbonyl (-C(=O)-R), carboxy (-CO₂H), oxycarbonyl (-C(=O)-OR), carbonyloxy (-O-C(=O)-R), sulfanylcarbonyl (thiocarbonyl) (-C(=O)-SR), a carbonylsulfanyl (carbonylthio) group (-S-C(=O)-R), aminocarbonyl (-C(=O)-NHR), carbonylamino (-NH-C(=O)-R), oxycarbonylamino (-NH-C(=O)-OR), sulfonylamino (-NH-S(O)₂-R), aminosulfonyl (-S(O)₂-NHR), sulfamoylamino (-NH-S(=O)₂-NHR), sulfanylcarboxy (thiocarboxy) (-C(=O)-SH), and carboxycarbonyl (-C(=O)-CO₂H).

Examples of the oxy (-OR) include alkoxy, cycloalkoxy, alkenyloxy, alkynyloxy, aryloxy, heteroaryloxy, and aralkyloxy. The alkoxy is preferably C₁-C₄ alkoxy or C₁-C₂ alkoxy, particularly preferably methoxy or ethoxy.

Examples of the carbonyl (-C(=O)-R) include formyl (-C(=O)-H), alkylcarbonyl, cycloalkylcarbonyl, alkenylcarbonyl, alkynylcarbonyl, arylcarbonyl, heteroarylcarbonyl, and aralkylcarbonyl.

Examples of the oxycarbonyl (-C(=O)-OR) include alkyloxycarbonyl, cycloalkyloxycarbonyl, alkenyloxycarbonyl, alkynyloxycarbonyl, aryloxycarbonyl, heteroaryloxycarbonyl, and aralkyloxycarbonyl.

Examples of the carbonyloxy (-O-C(=O)-R) include alkylcarbonyloxy, cycloalkylcarbonyloxy, alkenylcarbonyloxy, alkynylcarbonyloxy, arylcarbonyloxy, heteroarylcarbonyloxy, and aralkylcarbonyloxy.

Examples of the sulfanylcarbonyl (also called thiocarbonyl) (-C(=O)-SR) include alkylsulfanylcarbonyl, cycloalkylsulfanylcarbonyl, alkenylsulfanylcarbonyl, alkynylsulfanylcarbonyl, arylsulfanylcarbonyl, heteroarylsulfanylcarbonyl, and aralkylsulfanylcarbonyl.

Examples of the carbonylsulfanyl (also called carbonylthio) (-S-C(=O)-R) include alkylcarbonylsulfanyl, cycloalkylcarbonylsulfanyl, alkenylcarbonylsulfanyl, alkynylcarbonylsulfanyl, arylcarbonylsulfanyl, heteroarylcarbonylsulfanyl, and aralkylcarbonylsulfanyl.

Examples of the aminocarbonyl (-C(=O)-NHR) include alkylaminocarbonyl (e.g., C₁-C₆ or C₁-C₄ alkylaminocarbonyl, specifically, ethylaminocarbonyl or methylaminocarbonyl), cycloalkylaminocarbonyl, alkenylaminocarbonyl, alkynylaminocarbonyl, arylaminocarbonyl, heteroarylaminocarbonyl, and aralkylaminocarbonyl. Additional examples thereof include groups in which the H atom bonded to the N atom in -C(=O)-NHR is further substituted with alkyl, cycloalkyl, alkenyl, alkynyl, aryl, heteroaryl, or aralkyl.

Examples of the carbonylamino (-NH-C(=O)-R) include alkylcarbonylamino, cycloalkylcarbonylamino, alkenylcarbonylamino, alkynylcarbonylamino, arylcarbonylamino, heteroarylcarbonylamino, and aralkylcarbonylamino. Additional examples thereof include groups in which the H atom bonded to the N atom in -NH-C(=O)-R is further substituted with alkyl, cycloalkyl, alkenyl, alkynyl, aryl, heteroaryl, or aralkyl.

Examples of the oxycarbonylamino (-NH-C(=O)-OR) include alkoxycarbonylamino, cycloalkoxycarbonylamino, alkenyloxycarbonylamino, alkynyloxycarbonylamino, aryloxycarbonylamino, heteroaryloxycarbonylamino, and aralkyloxycarbonylamino. Additional examples thereof include groups in which the H atom bonded to the N atom in -NH-C(=O)-OR is further substituted with alkyl, cycloalkyl, alkenyl, alkynyl, aryl, heteroaryl, or aralkyl.

Examples of the sulfonylamino (-NH-SO₂-R) include alkylsulfonylamino, cycloalkylsulfonylamino, alkenylsulfonylamino, alkynylsulfonylamino, arylsulfonylamino, heteroarylsulfonylamino, and aralkylsulfonylamino. Additional examples thereof include groups in which the H atom bonded to the N atom in -NH-SO₂-R is further substituted with alkyl, cycloalkyl, alkenyl, alkynyl, aryl, heteroaryl, or aralkyl.

Examples of the aminosulfonyl (-SO₂-NHR) include alkylaminosulfonyl, cycloalkylaminosulfonyl, alkenylaminosulfonyl, alkynylaminosulfonyl, arylaminosulfonyl, heteroarylaminosulfonyl, and aralkylaminosulfonyl. Additional examples thereof include groups in which the H atom bonded to the N atom in -SO₂-NHR is further substituted with alkyl, cycloalkyl, alkenyl, alkynyl, aryl, heteroaryl, or aralkyl.

Examples of the sulfamoylamino (-NH-SO₂-NHR) include alkylsulfamoylamino, cycloalkylsulfamoylamino, alkenylsulfamoylamino, alkynylsulfamoylamino, arylsulfamoylamino, heteroarylsulfamoylamino, and aralkylsulfamoylamino. Further, the two H atoms bonded to the N atom in -NH-SO₂-NHR is optionally substituted with substituents independently selected from the group consisting of alkyl, cycloalkyl, alkenyl, alkynyl, aryl, heteroaryl, and aralkyl, and the two substituents may form a ring which includes a nitrogen atom to which they are attached.

Examples of the "sulfur atom-containing substituent" as used herein include thiol (-SH), sulfanyl (thio (-S-R)), sulfinyl (-S(=O)-R), sulfonyl (-S(=O)₂-R), sulfo (-SO₃H), pentafluorosulfanyl (-SF₅), and disulfanyl (-S-S-R).

Examples of the sulfanyl (thio (-S-R)) include alkylsulfanyl, cycloalkylsulfanyl, alkenylsulfanyl, alkynylsulfanyl, arylsulfanyl, heteroarylsulfanyl, and aralkylsulfanyl.

Examples of the sulfanyl (-S(=O)-R) include alkylsulfinyl, cycloalkylsulfinyl, alkenylsulfinyl, alkynylsulfinyl, arylsulfinyl, heteroarylsulfinyl, and aralkylsulfinyl.

Examples of the sulfonyl (-S(=O)₂-R) include alkylsulfonyl, cycloalkylsulfonyl, alkenylsulfonyl, alkynylsulfonyl, arylsulfonyl, heteroarylsulfonyl, and aralkylsulfonyl.

Examples of the "nitrogen atom-containing substituent" as used herein include azide (-N₃, also referred to as "azide group"), cyano (-CN), primary amino (-NH₂), secondary amino (-NH-R), tertiary amino (-NR(R')), amidino (-C(=NH)-NH₂), substituted amidino (-C(=NR)-NR'R"), guanidino (-NH-C(=NH)-NH₂), substituted guanidino (-NR-C(=NR‴)-NR'R"), and aminocarbonylamino (-NR-CO-NR'R").

Examples of the secondary amino (-NH-R) include alkylamino, cycloalkylamino, alkenylamino, alkynylamino, arylamino, heteroarylamino, and aralkylamino.

Examples of the tertiary amino (-NR(R')) include an amino group having any two substituents independently selected from alkyl, cycloalkyl, alkenyl, alkynyl, aryl, heteroaryl, aralkyl, and the like, such as alkyl(aralkyl)amino, and the two substituents may form a ring which includes a nitrogen atom to which they are attached.

Examples of the substituted amidino (-C(=NR)-NR'R") include groups in which three substituents R, R', and R" on the N atoms are each independently selected from alkyl, cycloalkyl, alkenyl, alkynyl, aryl, heteroaryl, and aralkyl, for example, alkyl(aralkyl)(aryl)amidino.

Examples of the substituted guanidino (-NR-C(=NR‴)-NR'R") include groups in which R,R', R", and R‴ are each independently selected from alkyl, cycloalkyl, alkenyl, alkynyl, aryl, heteroaryl, and aralkyl, and groups in which these substituents form a ring which includes a nitrogen atom to which they are attached.

Examples of the aminocarbonylamino (-NR-CO-NR'R") include groups in which R, R', and R" are each independently selected from a hydrogen atom, alkyl, cycloalkyl, alkenyl, alkynyl, aryl, heteroaryl, and aralkyl, and groups in which these substituents form a ring which includes a nitrogen atom to which they are attached.

As used herein, the "amino acid residue" constituting a peptide compound is sometimes simply referred to as "amino acid".

As used herein, the "to" that indicates a numerical range includes values of both ends thereof. For example, "A to B" means the numerical range of A or more and B or less.

As used herein, the term "approximately", when used in combination with a numeric value, means the value range of +10% and -10% of the numeric value.

The term "and/or" as used herein is meant to include every combination of the terms "and" and "or" appropriately combined. Specifically, for example, the term "A, B, and/or C" includes the following seven variations: (i) A, (ii) B, (iii) C, (iv) A and B, (v) A and C, (vi) B and C, and (vii) A, B, and C.

### Method for producing compound represented by formula (1) or salt thereof

In an aspect, the present invention relates to a method for producing a compound represented by formula (1) or a salt thereof. The method comprises the step (metathesis step) of bringing a compound represented by formula (2) or a salt thereof into contact with a catalyst: 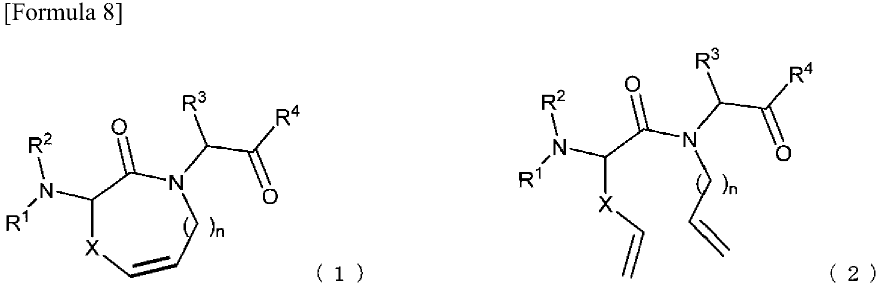 wherein R¹, R², R³, R⁴, n and X have the same meaning as R¹, R², R³, R⁴, n and X in the above [1], respectively.

In an aspect, the catalyst used in the metathesis step is a metal-alkylidene-type complex. Examples of the metal-alkylidene-type complex include a ruthenium-alkylidene-type complex, a molybdenum-alkylidene-type complex, and a tungsten-alkylidene-type complex, with a ruthenium-alkylidene-type complex being preferred. When the catalyst is a ruthenium-alkylidene-type complex, the catalyst preferably contains phosphine and/or N-heterocyclic carbene as a ligand.

Specific examples of the catalyst used in the metathesis step include first-generation Grubbs catalyst (dichloro(benzylidene)bis(tricyclohexylphosphine)ruthenium (II)), second-generation Grubbs catalyst(dichloro[1,3-bis(2,4,6-trimethylphenyl)-2-imidazolidinylidene](benzylidene)(tricyclohexylphosphine)ruthenium (II)), third-generation Grubbs catalyst(dichloro[1,3-bis(2,4,6-trimethylphenyl)-2-imidazolidinylidene](benzylidene)bis(3-bromopyridine)ruthenium (II)), Stewart-Grubbs catalyst(dichloro[1,3-bis(2-methylphenyl)-2-imidazolidinylidene](2-isopropoxyphenylmethylene)ruthenium (II)), nitro-Grela catalyst(dichloro[1,3-bis(2,4,6-trimethylphenyl)-2-imidazolidinylidene][(2-isopropoxy-5-nitrobenzylidene)]ruthenium (II)), Zhan catalyst-1B(dichloro[1,3-bis(2,4,6-trimethylphenyl)-2-imidazolidinylidene][[5-[(dimethylamino)sulfonyl]-2-(1-methylethoxy-O)phenyl]methylene-C]ruthenium(II)), Zhan catalyst-1C({[2-(isopropoxy)-5-(N,N-dimethylaminosulfonyl)phenyl]methylene}(tricyclohexylphosphine)dichlororuthenium(II)), first-generation Hoveyda-Grubbs catalyst(dichloro(2-isopropoxyphenylmethylene)(tricyclohexylphosphine)ruthenium), second-generation Hoveyda-Grubbs catalyst((1,3-bis-(2,4,6-trimethylphenyl)-2-imidazolidinylidene)dichloro(o-isopropoxyphenylmethylene)ruthenium), [1,3-bis(2,6-isopropylphenyl)-2-imidazolidinylidene]dichloro(2-isopropoxybenzylidene)ruthenium (II), bis[1-(2,6-diethylphenyl)-3,5,5-trimethyl-3-phenylpyrrolidine-2-ylidene](3-phenyl-1H-inden-1-ylidene)ruthenium chloride, [1-(2,6-diethylphenyl)-3,5,5-trimethyl-3-phenylpyrrolidin-2-yl](2-isopropoxy-5-nitrobenzylidene)ruthenium (II) chloride, or [1-[2,4,6-trimethylphenyl]-3,5,5-trimethyl-3-phenyl-2-pyrrolinylidene]dichloro(2-isopropoxybenzylidene)ruthenium (II). Among them, preferred are the catalyst is first-generation Hoveyda-Grubbs catalyst(dichloro(2-isopropoxyphenylmethylene)(tricyclohexylphosphine)ruthenium), second-generation Hoveyda-Grubbs catalyst((1,3-bis-(2,4,6-trimethylphenyl)-2-imidazolidinylidene)dichloro(o-isopropoxyphenylmethylene)ruthenium), or [1-[2,4,6-trimethylphenyl]-3,5,5-trimethyl-3-phenyl-2-pyrrolinylidene]dichloro(2-isopropoxybenzylidene)ruthenium (II).

The amount of the catalyst used in the metathesis step is not particularly limited, and is, for example, 0.001 to 0.5 molar equivalent, preferably 0.003 to 0.3 molar equivalent, and more preferably 0.05 to 0.1 molar equivalent, with respect to the compound represented by formula (2).

In an aspect, the compound represented by formula (2) or a salt thereof is a salt of a compound represented by formula (2). The salt of a compound represented by formula (2) is preferably a salt with a Bronsted acid having a pKa of 3 or less. Examples of the salt include hydrochloric acid salts, hydrobromic acid salts, sulfuric acid salts, phosphoric acid salts, methanesulfonic acid salts, para-toluenesulfonic acid salts, trifluoromethanesulfonic acid salts, or trifluoroacetic acid salts. Among them, hydrochloric acid salts are preferred.

In an aspect, the compound represented by formula (2) or a salt thereof is a compound represented by formula (2), and an acid is used in the metathesis step. The acid used is preferably a Bronsted acid having a pKa of 3 or less, or a Lewis acid, and more preferably a Lewis acid. Examples of the Bronsted acid having a pKa of 3 or less include hydrogen chloride, hydrobromic acid, sulfuric acid, phosphoric acid, methanesulfonic acid, para-toluenesulfonic acid, trifluoromethanesulfonic acid, or trifluoroacetic acid. Among them, hydrogen chloride is preferred. Examples of the Lewis acid include boron trihalide, a solvation complex thereof (e.g., a boron trifluoride-tetrahydrofuran complex and a boron trifluoride-diethyl ether complex), titanium tetrahalide (e.g., titanium tetrachloride and titanium tetrabromide), and tetraalkoxytitanium (e.g., tetra-i-propoxytitanium and tetra-n-butoxytitanium). Among them, a boron trifluoride-tetrahydrofuran complex or a boron trifluoride-diethyl ether complex is preferred.

When a Bronsted acid having a pKa of 3 or less is used in the metathesis step, the amount thereof is not particularly limited, and is, for example, 0.1 to 10.0 molar equivalent, preferably 1.0 to 3.0 molar equivalent, more preferably 1.0 to 2.0 molar equivalent, and still more preferably 1.0 to 1.5 molar equivalent, with respect to the compound represented by formula (2).

When a Lewis acid is used in the metathesis step, the amount thereof is not particularly limited, and is, for example, 0.1 to 10.0 molar equivalent, preferably 1.0 to 3.0 molar equivalent, more preferably 1.0 to 2.0 molar equivalent, and still more preferably 1.0 to 1.5 molar equivalent, with respect to the compound represented by formula (2).

In an aspect, alcohols and/or Bronsted acids are further used as an additive in the metathesis step, and pKa of the additive is 4 to 17. The "pKa" as used herein can be measured using water as a solvent. As the pKa used herein, measured values that have already been reported as pKa when using water as a solvent may be employed. In this case, a value from measurement at 25°C is applied as the measured value of pKa. When the measured value of pKa is not available, pKa may be calculated with ADMETPredictor (Simulations Plus Inc., ver8.0) and employed as pKa herein. Examples of the alcohol, among additives having a pKa of 4 to 17, include 1,1,1,3,3,3-hexafluoro-2-propanol (HFIP), 2-propanol, methanol, and 2,2,2-trifluoroethanol (TFE). Examples of the Bronsted acid, among additives having a pKa of 4 to 17, include acetic acid. Among additives, 1,1,1,3,3,3-hexafluoro-2-propanol (HFIP), or acetic acid is preferred.

When alcohols and/or Bronsted acids are used as an additive in the metathesis step, the amount thereof is not particularly limited, and is, for example, 0.1 mL to 100 mL, and preferably 2 mL to 10 mL, per g of the compound represented by formula (2).

In an aspect, the metathesis step is performed by a liquid-phase synthesis method. The solvent used in the liquid-phase synthesis method comprises at least one selected from the group consisting of a ketone-based solvent, a nitrile-based solvent, a halogen-based solvent, an ether-based solvent, an amide-based solvent, an ester-based solvent, an aromatic hydrocarbon-based solvent, an aliphatic hydrocarbon-based solvent, and a carbonate-ester-based solvent. Specific examples of the ketone-based solvent include acetone, methyl ethyl ketone, methyl isobutyl ketone, cyclohexanone, cyclopentanone, and diethyl ketone. Specific examples of the nitrile-based solvent include acetonitrile and propionitrile. Specific examples of the halogen-based solvent include dichloromethane, chloroform, and 1,2-dichloroethane. Specific examples of the ether-based solvent include diethyl ether, tetrahydrofuran, 2-methyltetrahydrofuran, cyclopentyl methyl ether, 4-methyltetrahydropyran, 1,3-dioxolane, 1,4-dioxane, 1,2-dimethoxyethane, diisopropyl ether, methyl t-butyl ether, diglyme, triglyme, anisole, and tetraglyme. Specific examples of the amide-based solvent include N,N-dimethylformamide (DMF), N-methyl-2-pyrrolidone (NMP), N,N-dimethylacetamide (DMA), N-ethyl-2-pyrrolidone (NEP), N-butyl-2-pyrrolidone (NBP), 1,3-dimethyl-2-imidazolidinone (DMI), and formamide. Specific examples of the ester-based solvent include methyl acetate, ethyl acetate, methyl propionate, butyl acetate, propyl acetate, isopropyl acetate, isobutyl acetate, pentyl acetate, and γ-valerolactone. Specific examples of the aromatic hydrocarbon-based solvent include toluene, o-xylene, m-xylene, p-xylene, mesitylene, chlorobenzene, fluorobenzene, and anisole. Specific examples of the aliphatic hydrocarbon-based solvent include pentane, hexane, heptane, octane, and cyclohexane. Specific examples of the carbonate-ester-based solvent include dimethyl carbonate, diethyl carbonate, and dibutyl carbonate. The solvent used in the cyclization step is preferably one or more selected from the group consisting of acetone, methyl t-butyl ether, dimethyl carbonate, 2-methyltetrahydrofuran, 4-methyltetrahydropyran, tetrahydrofuran, ethyl acetate, isopropyl acetate, dichloromethane, 1,2-dichloroethane, toluene, chlorobenzene, heptane, and cyclohexane, more preferably one or more selected from the group consisting of acetone, methyl t-butyl ether, dimethyl carbonate, ethyl acetate, toluene, and dichloromethane, still more preferably acetone or toluene, and most preferably acetone.

When the metathesis step is performed by a liquid-phase synthesis method, the concentration of the compound represented by formula (2) or a salt thereof, with respect to the solvent used in the liquid-phase synthesis method is 0.01 to 0.3 mol/L, preferably 0.02 to 0.3 mol/L, and more preferably 0.033 to 0.3 mol/L.

In an aspect, the metathesis step can be performed by stirring a reaction mixture at a reaction temperature of 20 to 100°C, and preferably of 30 to 70°C. The reaction temperature may be a temperature of the reaction mixture in a reaction vessel (internal temperature), or a set temperature of a temperature adjusting apparatus for the reaction vessel (external temperature). The metathesis step can be performed by stirring the reaction mixture for 10 minutes to 10 hours, preferably for 1 to 6 hours, and more preferably for 1 to 4 hours.

When the metathesis step is performed by a liquid-phase synthesis method, the preparation of the reaction mixture may be any of the following (a) to (d):
(a) preparing a mixture of the compound represented by formula (2) or a salt thereof, a catalyst, an acid, an additive and a solvent by adding a solvent mixture containing the compound represented by formula (2) or a salt thereof to a solvent mixture containing a catalyst;
(b) preparing a mixture of the compound represented by formula (2) or a salt thereof, a catalyst, an acid, an additive and a solvent by adding a solvent mixture containing the compound represented by formula (2) or a salt thereof to a solvent mixture containing a heated catalyst;
(c) preparing a mixture of the compound represented by formula (2) or a salt thereof, a catalyst, an acid, an additive and a solvent by adding a solvent mixture containing a catalyst to a solvent mixture containing the compound represented by formula (2) or a salt thereof; and
(d) preparing a mixture by adding a solvent to the compound represented by formula (2) or a salt thereof, and then a catalyst, an acid and an additive.

In an aspect, the compound represented by formula (2) or a salt thereof is obtained by condensing a compound represented by formula (6) and a compound represented by formula (7), and then deprotecting the carbamate protective group. 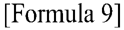 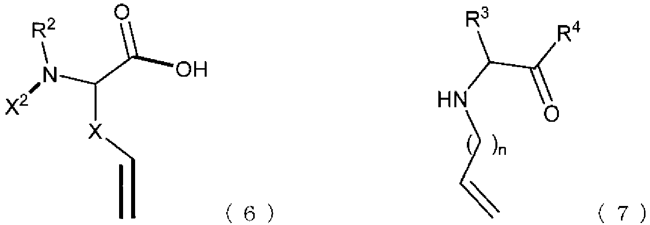 wherein R², R³, R⁴, n and X have the same meaning as R², R³, R⁴, n and X in the above [1], respectively, and X² is a carbamate protective group.

The condensation reaction of the compound represented by formula (6) and the compound represented by formula (7) can be carried out, for example, during solid phase synthesis and/or liquid phase synthesis using a condensation reagent and/or a base, and is preferably carried out during the liquid phase synthesis.

The condensation reagent and base used in the condensation reaction, as well as their amounts to be used, are not particularly limited, but a condensation reagent and a base commonly used in peptide synthesis, as well as amount to be used, are preferred (see, for example, Peptide Coupling Reagents, More than a Letter Soup (Chem. Rev. 2011, 111, 6557-6602.)). When a condensation reagent is not used in the condensation reaction, a compound in which a carboxyl group has been converted to an active ester or the like in advance may be used.

Specific examples of the condensation reagent include N,N'-dicyclohexylcarbodiimide (DCC), N,N'-diisopropylcarbodiimide (DIC), 1-ethyl-3-(3-dimethylaminopropyl)carbodiimide hydrochloride (EDCI HCl), 1-hydroxy-1H-benzotriazole (HOBt), 1-hydroxy-7-azabenzotriazole (HOAt), 2-cyano-2-(hydroxyimino)ethyl acetate (oxyma), 3,4-dihydro-3-hydroxy-4-oxo-1,2,3-benzotriazine (HOOBt or HODhbt), N-hydroxy-5-norbornene-2,3-dicarboximide (HONB), 2,3,4,5,6-pentafluorophenol (HOPfp), N-hydroxysuccinimide (HOSu), 6-chloro-1-hydroxy-1H-benzotriazole (Cl-HOBt), O-(1H-benzotriazol-1-yl)-N,N,N',N'-tetramethyluronium hexafluorophosphate (HBTU), O-(7-aza-1H-benzotriazol-1-yl)-N,N,N',N'-tetramethyluronium hexafluorophosphate (HATU), N-[1-(cyano-2-ethoxy-2-oxoethylideneaminooxy)dimethylamino(morpholino)]uronium hexafluorophosphate (COMU), O-[(ethoxycarbonyl)cyanomethyleneamino]-N,N,N',N'-tetramethyluronium hexafluorophosphate (HOTU), O-(1H-benzotriazol-1-yl)-N,N,N',N'-tetramethyluronium tetrafluoroborate (TBTU), O-(7-azabenzotriazol-1-yl)-N,N,N',N'-tetramethyluronium tetrafluoroborate (TATU), [ethyleyano(hydroxyimino)acetato-O²]tri-1-pyrrolidinylphosphonium hexafluorophosphate (PyOxim), 2-bromo-1-ethyl pyridinium tetrafluoroborate (BEP), 1H-benzotriazole-1-yloxy-tri(pyrrolidino)phosphonium hexafluorophosphate (PyBOP), 1H-benzotriazole-1-yloxy-tris (dimethylamino)phosphonium hexafluorophosphate (BOP), bromotri(pyrrolidino)phosphonium hexafluorophosphate (PyBroP), chlorotri(pyrrolidino)phosphonium hexafluorophosphate (PyCloP), (7-azabenzotriazol-1-yloxy)tripyrrolidino phosphonium hexafluorophosphate (PyAOP), bromotris(dimethylamino)phosphonium hexafluorophosphate (Brop), 3-(diethoxyphosphoryloxy)-1,2,3-benzotriazin-4(3H)-one (DEPBT), N,N,N',N'-tetramethyl-O-(N-succinimidyl)uronium tetrafluoroborate (TSTU), N,N,N',N'-tetramethyl-O-(N-succinimidyl)uronium hexafluorophosphate (HSTU), O-(3,4-dihydro-4-oxo-1,2,3-benzotriazin-3-yl)-N,N,N',N'-tetramethyluronium tetrafluoroborate (TDBTU), tetramethylthiuronium S-(1-oxide-2-pyridyl)-N,N,N',N'-tetrafluoroborate (TOTT), O-(2-oxo-1(2H)pyridyl)-N,N,N',N'-tetramethyluronium tetrafluoroborate (TPTU), N,N' -carbonyl diimidazole (CDI), 1,1'-carbonyl-di-(1,2,4-triazole) (CDT), 4-(4,6-dimethoxy-1,3,5-triazin-2-yl)-4-methylmorpholinium chloride (DMT-MM), propylphosphonic anhydride (T3P), and chloro-N,N,N',N'-tetramethylformamidinium hexafluorophosphate. Among them, one or more selected from the group consisting of HATU, T3P, and TCFH are preferred, and TCFH is more preferred.

As the base, organic bases are suitably used, and particularly, organic bases containing tertiary amines are preferred. Specific examples of such a base include 2,2,6,6-tetramethylpiperidine, N-methylmorpholine, N,N-diisopropylethylamine (DIPEA), 2,4,6-collidine, 2,6-lutidine, pyridine, 1,8-diazabicyclo[5.4.0]-7-undecene (DBU), 2,3,6,7-tetrahydro-1H,5H-9-azabenzo[ij]quinolidine, 1,4-diazabicyclo[2.2.2]octane (DABCO), 1,5-diazabicyclo[4.3.0]-5-nonene (DBN), 7-methyl-1,5,7-triazabicyclo[4.4.0]dec-5-ene, 1,1,3,3-tetramethylguanidine (TMG), 1,8-bis(tetramethylguanidino)naphthalene (TMGN), 2-tert-butyl-1,1,3,3-tetramethylguanidine (BTMG), triethylamine (TEA), trimethylamine, 1-methylpiperidine, N,N'-dimethylpiperazine, N-ethylmorpholine, and p-dimethylaminopyridine (DMAP). Among them, one or more selected from the group consisting of N-methylmorpholine, 2,6-lutidine, and N,N-diisopropylethylamine (DIPEA) are preferred, and N,N-diisopropylethylamine (DIPEA) is more preferred.

In an aspect, the condensation reaction is carried out by a liquid-phase synthesis method. The solvent used in the liquid-phase synthesis method may include one or more selected from the group consisting of a nitrile-based solvent, a halogen-based solvent, an ether-based solvent, an amide-based solvent, an ester-based solvent, and a carbonate-ester-based solvent. Specific examples of the nitrile-based solvent include acetonitrile and propionitrile. Specific examples of the halogen-based solvent include dichloromethane, chloroform, and 1,2-dichloroethane. Specific examples of the ether-based solvent include diethyl ether, tetrahydrofuran, 2-methyltetrahydrofuran, cyclopentyl methyl ether, 4-methyltetrahydropyran, 1,3-dioxolane, 1,4-dioxane, 1,2-dimethoxyethane, diisopropyl ether, methyl t-butyl ether, diglyme, triglyme, anisole, and tetraglyme. Specific examples of the amide-based solvent include DMF, NMP, DMA, NEP, NBP, DMI, and formamide. Specific examples of the ester-based solvent include methyl acetate, ethyl acetate, methyl propionate, butyl acetate, propyl acetate, isopropyl acetate, isobutyl acetate, pentyl acetate, and γ-valerolactone. Specific examples of the carbonate-ester-based solvent include dimethyl carbonate, diethyl carbonate, and dibutyl carbonate. The solvent for the condensation reaction is preferably one or more selected from the group consisting of acetonitrile, dimethyl carbonate, 2-methyltetrahydrofuran, 4-methyltetrahydropyran, tetrahydrofuran, ethyl acetate, isopropyl acetate, dichloromethane, DMF, and anisole, more preferably one or more selected from the group consisting of acetonitrile, 2-methyltetrahydrofuran, ethyl acetate, and dichloromethane, and still more preferably one or more selected from the group consisting of acetonitrile, and 2-methyltetrahydrofuran.

The condensation reaction of the compound represented by formula (6) and the compound represented by formula (7) can be carried out by, for example, stirring the reaction mixture at a reaction temperature of 10 to 50°C, preferably of 20 to 40°C. The reaction temperature may be a temperature of the reaction mixture in a reaction vessel (internal temperature), or a set temperature of a temperature adjusting apparatus for the reaction vessel (external temperature). The condensation reaction can be carried out by stirring the reaction mixture for 0.5 to 8 hours, and preferably for 1 to 5 hours.

The carbamate protective group in the compound obtained in the condensation reaction of the compound represented by formula (6) and the compound represented by formula (7) can be eliminated by a method known to those skilled in the art, and examples thereof include a method in which the reaction mixture is stirred for 1 to 6 hours at a reaction temperature of 10 to 40°C with or without a solvent such as 2-methyltetrahydrofuran, acetonitrile or toluene using an acid such as methanesulfonic acid or trifluoroacetic acid; or a base such as DBU or piperidine.

### Method for producing compound represented by formula (3) or salt thereof

In an aspect, the present invention relates to a method for producing a compound represented by formula (3) or a salt thereof. The method comprises the step of hydrogenating the compound represented by formula (1) or a salt thereof, which is obtained in the above-described production method. 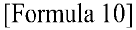 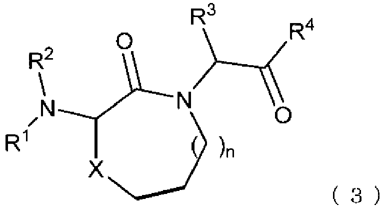 wherein R¹, R², R³, R⁴, n and X have the same meaning as R¹, R², R³, R⁴, n and X in the above [1], respectively.

The hydrogenation step can be performed by those skilled in the art, and examples thereof include a method in which the reaction mixture is stirred for 0.5 to 12 hours, and preferably for 1 to 6 hours, at a reaction temperature of 10 to 40°C in a hydrogen atmosphere with or without a solvent such as 2-methyltetrahydrofuran, methanol or ethyl acetate using a catalyst such as Pd/C, Pd(OH)₂/C or PtO₂.

### Method for producing compound represented by formula (4) or salt thereof

In an aspect, the present invention relates to a method for producing a compound represented by formula (4) or a salt thereof. The method comprises the step of protecting the amino group of the compound represented by formula (1) or a salt thereof, which is obtained in the above-described production method, with a carbamate protective group. 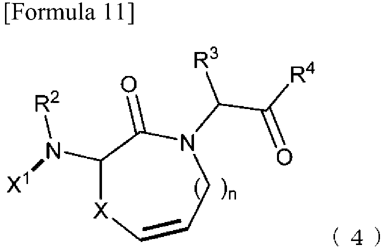 wherein R², R³, R⁴, n and X have the same meaning as R², R³, R⁴, n and X in the above [1], respectively, and X¹ is a carbamate protective group.

The step of protecting with a carbamate protective group can be performed by a method known to those skilled in the art, and examples thereof include a method in which the reaction mixture is stirred for 1 to 4 hours at a reaction temperature of 10 to 40°C with or without a solvent such as 2-methyltetrahydrofuran, acetonitrile or toluene using a carbamation reagent such as di-tert-butyl dicarbonate, N-carbobenzoxyoxysuccinimide or 9-fluorenylmethyl chloroformate.

In an aspect, the present invention relates to a method for producing (1S,4S,10S,138S,178S,208,268S,28R,32S,38S,42Z)-20-cyclopentyl-28-ethoxy-32-[2-[3-methoxy-4-(trifluoromethyl)phenyl]ethyl]-N,N,2,14,18,21,24,36-octamethyl-10-[(1S)-1-methylpropyl]-3,9,12,15,19,22,25,31,34,37,45-undecaoxo-13-propyl-38-[[4-(trifluoromethyl)phenyl]methyl]spiro[2,8,11,14,18,21,24,30,33,36,39-undecazatetracyclo[37.5.1.0^{4,8}.0^{26,30}]pentatetracont-42-ene-23,1'-cyclobutane]-17-carboxamide or a salt thereof. The method includes a method in which the amino group of the compound represented by formula (4) or a salt thereof, which is obtained in the above-described production method, is synthesized according to International Publication No. WO 2023/214576.

### Method for producing compound represented by formula (5) or salt thereof

In an aspect, the present invention relates to a method for producing a compound represented by formula (5) or a salt thereof. The method comprises the step of protecting the amino group of the compound represented by formula (2) or a salt thereof, which is obtained in the above-described production method, with a carbamate protective group. 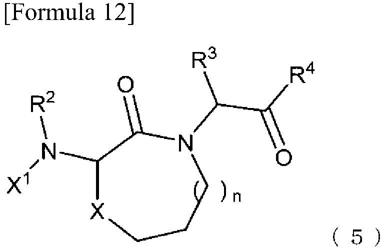 wherein R², R³, R⁴, n and X have the same meaning as R², R³, R⁴, n and X in the above [1], respectively, and X¹ is a carbamate protective group.

The step of protecting with a carbamate protective group can be performed by a method known to those skilled in the art, and examples thereof include a method in which the reaction mixture is stirred for 1 to 4 hours at a reaction temperature of 10 to 40°C with or without a solvent such as 2-methyltetrahydrofuran, acetonitrile or toluene using a carbamation reagent such as di-tert-butyl dicarbonate, N-carbobenzoxyoxysuccinimide or 9-fluorenylmethyl chloroformate.

The following is description of each symbol used in the structural formula of the compounds represented by formulae (1) to (7).

R¹ is hydrogen.

R² is hydrogen or C₁-C₆ alkyl. R² is preferably hydrogen or methyl.

R³ is hydrogen, optionally substituted C₁-C₆ alkyl, optionally substituted halo-C₁-C₆ alkyl, optionally substituted C₂-C₆ alkenyl, optionally substituted C₂-C₆ alkynyl, optionally substituted C₃-C₈ cycloalkyl, optionally substituted C₆-C₁₄ aryl, optionally substituted 5- to 14-membered heteroaryl, optionally substituted C₇-C₁₄ aralkyl, optionally substituted 3- to 14-membered heterocyclyl, optionally substituted 5- to 10-membered heteroaryl-C₁-C₆ alkyl, optionally substituted C₁-C₆ alkoxy-C₁-C₆ alkyl, optionally substituted C₁-C₆ alkylsulfanyl-C₁-C₆ alkyl, optionally substituted C₁-C₆ alkylsulfinyl-C₁-C₆ alkyl, optionally substituted carboxy-C₁-C₆ alkyl, optionally substituted C₇-C₁₄ aralkoxy-C₁-C₆ alkyl, optionally substituted C₃-C₈ cycloalkyl-C₁-C₆ alkyl, optionally substituted C₃-C₈ cycloalkoxy-C₁-C₆ alkyl, optionally substituted 4- to 7-membered heterocyclyl-C₁-C₃ alkyl, optionally substituted 5- to 10-membered heteroaryl-C₁-C₆ alkoxy-C₁-C₆ alkyl, or optionally substituted aminocarbonyl (the relevant amino is selected from the group consisting of -NH₂, mono-C₁-C₆ alkylamino, di-C₁-C₆ alkylamino, N-C₁-C₆ alkyl-N-C₂-C₆ alkenylamino, N-C₁-C₆ alkyl-N-C₁-C₆ alkoxy-C₁-C₆ alkylamino, and 4- to 9-membered cyclic amino). R³ is preferably hydrogen, optionally substituted C₁-C₆ alkyl, optionally substituted C₂-C₆ alkenyl, optionally substituted C₂-C₆ alkynyl, optionally substituted C₃-C₈ cycloalkyl, optionally substituted C₇-C₁₄ aralkyl, optionally substituted 5- to 10-membered heteroaryl-C₁-C₆ alkyl, or optionally substituted 3- to 14-membered heterocyclyl, more preferably hydrogen, C₁-C₆ alkyl, or C₇-C₁₄ aralkyl optionally substituted with halo-C₁-C₃ alkyl or C₁-C₆ alkyl, and still more preferably hydrogen, 2-methylpropyl, p-(trifluoromethyl)benzyl, or p-methylbenzyl.

R⁴ is OR⁵, NHR⁵', an amino acid residue, or a peptide chain containing 1 to 20 amino acid residues, the amino acid residue and the peptide chain may have a protective group, and R⁵ is a protective group for the carboxy group. R⁴ is preferably OR⁵, NHR⁵', an amino acid residue, or a peptide chain containing 2 to 13 amino acid residues. R⁵ is preferably an alkyl ester-type protective group, a benzyl ester-type protective group, a substituted alkyl ester-type protective group, or an alkenyl ester-type protective group, more preferably a methyl group, an ethyl group, a t-Bu group, a benzyl group, a trityl group, a cumyl group, a methoxytrityl group, a 2-(trimethylsilyl)ethyl group, a 2,2,2-trichloroethyl group, or an allyl group, and still more preferably tert-butyl, or benzyl. R⁵' is a phenylamide-type protective group, an alkylamide-type protective group, an alkenylamide-type protective group, a benzylamide-type protective group, or an alkoxyalkylamide-type protective group, more preferably a phenyl group, a t-Bu group, an allyl group, a benzyl group, a 4-methoxybenzyl group, a trityl group, a cumyl group, a methoxymethyl group, or a benzyloxymethyl group, and still more preferably phenyl. The amino acid residue is preferably glycine, alanine, isoleucine, leucine, valine, methionine, phenylalanine, tyrosine, proline, N-methylglycine, N-methylalanine, N-methylisoleucine, N-methylleucine, N-methylvaline, N-methylmethionine, N-methylphenylalanine, N-methyltyrosine, or N-methylproline. The amino acid residue is more preferably glycine, alanine, isoleucine, leucine, methionine, phenylalanine, tyrosine, N-methylglycine, N-methylalanine, N-methylisoleucine, N-methylleucine, N-methylmethionine, N-methylphenylalanine, or N-methyltyrosine.

n is an integer of 1 to 4. n is an integer of 1 or 2. n is most preferably 2.

X is optionally substituted C₁-C₃ alkylene, -CH₂OCH₂-, or -CH₂SCH₂-. X is preferably optionally substituted C₁-C₃ alkylene, or -CH₂OCH₂-, more preferably optionally substituted C₁-C₃ alkylene, still more preferably C₁-C₃ alkylene, and most preferably methylene.

X¹ is one selected from the group consisting of an Fmoc group, a Cbz group, a Troc group, an Alloc group, a Teoc group, a TSoc group, a BIBSoc group, an IPCSoc group, a BBSoc group, a CHBSoc group, a CDBSoc group, and a Boc group. X¹ is preferably one selected from the group consisting of an Fmoc group, a Cbz group, and a Boc group. X¹ is most preferably a Fmoc group.

X² is one selected from the group consisting of an Fmoc group, a Cbz group, a Troc group, an Alloc group, a Teoc group, a TSoc group, a BIBSoc group, an IPCSoc group, a BBSoc group, a CHBSoc group, a CDBSoc group, and a Boc group. X² is preferably one selected from the group consisting of an Fmoc group, a Cbz group, and a Boc group. X² is most preferably a Fmoc group.

As a combination of X¹ and X², it is preferred that each of both X¹ and X² be one selected from the group consisting of an Fmoc group, a Cbz group, a Troc group, an Alloc group, a Teoc group, a TSoc group, a BIBSoc group, an IPCSoc group, a BBSoc group, a CHBSoc group, a CDBSoc group, and a Boc group. More preferably, each of both X¹ and X² is one selected from the group consisting of an Fmoc group, a Cbz group, and a Boc group. Most preferably, each of both X¹ and X² is an Fmoc group.

### Method for producing cyclic peptide compound or salt thereof

In an aspect, the present invention relates to a method for producing a cyclic peptide compound or a salt thereof. The method includes a method in which the compound represented by each of formulae (1) to (5) or a salt thereof, which is obtained in the above-described production method, itself or a peptide compound obtained by chemically converting the compound or a salt thereof is cyclized.

In an aspect, the cyclic peptide compound is produced by amide cyclization of an N-terminal amino group and a C-terminal carboxy group of a peptide compound. The amide cyclization can be performed using, for example, a condensation reagent and/or a base. The condensation reagent and the base used in amide cyclization are not particularly limited. It is possible to use a condensation reagent and a base which are commonly used in peptide synthesis. The amide cyclization can also be performed by a liquid-phase synthesis method. The solvent used in the liquid-phase synthesis method is not particularly limited. It is possible to use a solvent which is commonly used in peptide synthesis.

In an aspect, the cyclic peptide compound is produced by thioether cyclization of N-terminal chloroacetyl and a C-terminal cysteine side chain of a peptide compound.

In an aspect, a cyclic peptide compound or a salt thereof produced by the method of the present invention contains 8 to 20, preferably 11 to 14, more preferably 11 to 13, most preferably 11 amino acid residues.

In an aspect, a cyclic peptide compound or a salt thereof produced by the method of the present invention can contain at least one, at least two, at least three, at least four, or at least five non-natural amino acid residues. In certain embodiments, the non-natural amino acid residues contained in the cyclic peptide compound or a salt thereof produced by the method of the present invention are N-methylamino acid residues.

In an aspect, a cyclic peptide compound produced by the method of the present invention may comprise a cyclic structure (cyclic portion) as its moiety structure. Specific examples of the cyclic structure include those in which the side chain of one amino acid residue is linked to a side chain of another amino acid residue, those in which the N-substituent of one amino acid residue is linked to the side chain of another amino acid residue, and those in which the N-substituent of one amino acid residue is linked to the N-substituent of another amino acid residue. The two amino acid residues involved in the linkage for the cyclic structure may be adjacent, or any number of amino acid residues, for example, 1, 2, 3, 4, 5, 6, 7, 8, 9, 10, 11, 12, 13, 14, 15, 16, 17, 18, or 19 amino acid residues may be present therebetween. Examples of the size of the ring formed by the cyclic structure include, but are not intended to be particularly limited to, a 4-membered ring, a 5-membered ring, a 6-membered ring, a 7-membered ring, an 8-membered ring, a 9-membered ring, a 10-membered ring, an 11-membered ring, a 12-membered ring, a 13-membered ring, a 14-membered ring, a 15-membered ring, a 16-membered ring, a 17-membered ring, an 18-membered ring, a 19-membered ring, a 20-membered ring, a 21-membered ring, a 22-membered ring, a 23-membered ring, a 24-membered ring, a 25-membered ring, a 26-membered ring, a 27-membered ring, a 28-membered ring, a 29-membered ring, a 30-membered ring, a 31-membered ring, a 32-membered ring, a 33-membered ring, a 34-membered ring, and a 35-membered ring. Preferably, the ring formed by the cyclic structure is a 5-membered ring, a 6-membered ring, a 7-membered ring, an 8-membered ring, a 9-membered ring, a 10-membered ring, an 11-membered ring, a 12-membered ring, a 13-membered ring, a 14-membered ring, a 15-membered ring, a 16-membered ring, a 17-membered ring, an 18-membered ring, a 19-membered ring, or a 20-membered ring, more preferably a 10-membered ring, an 11-membered ring, a 12-membered ring, a 13-membered ring, a 14-membered ring, a 15-membered ring, a 16-membered ring, a 17-membered ring, or an 18-membered ring, most preferably an 11-membered ring, a 12-membered ring, a 13-membered ring, or a 14-membered ring. When a cyclic structure is present in the peptide compound, the number of cyclic structures is not limited, but it is preferable that one, two, three, four, or five cyclic structures be present.

In an aspect, a cyclic peptide compound or a salt thereof produced by the method of the present invention can contain a cyclic portion composed of 4 or more, 6 or more, 8 or more, or 11 or more amino acid residues. In an embodiment, a cyclic peptide compound or a salt thereof produced by the method of the present invention consists of a cyclic portion composed of 4 to 14, preferably 6 to 14, further preferably 8 to 14, most preferably 11 to 14 amino acid residues. In an embodiment, a cyclic peptide compound or a salt thereof produced by the method of the present invention consists of a cyclic portion composed of 11 amino acid residues. In these aspects, the amide bond with which the amino group of the first amino acid or peptide and the carboxy group of the second amino acid or peptide are linked is contained in the cyclic portion at 1, 2, 3, 4, 5, 6, or 7 locations.

In an aspect, the cyclic peptide compound or a salt thereof produced by the method of the present invention is (1S,4S,10S,13S,17S,20S,26S,28R,32S,38S,42Z)-20-cyclopentyl-28-ethoxy-32-[2-[3-methoxy-4-(trifluoromethyl)phenyl]ethyl]-N,N,2,14,18,21,24,36-octamethyl-10-[(1S)-1-methylpropyl]-3,9,12,15,19,22,25,31,34,37,45-undecaoxo-13-propyl-38-[[4-(trifluoromethyl)phenyl] methyl] spiro [2, 8, 11,14,18,21,24,30,33,36,39-undecazatetracyclo[37.5.1.0^{4,8}.0^{26,30}]pentatetracont-42-ene-23,1'-cyclobutane]-17-carboxamide or a salt thereof.

In an aspect, the compound represented by formula (1) or a salt thereof, which is produced by the method of the present invention, may be a compound represented by formula (1a) or a salt thereof. 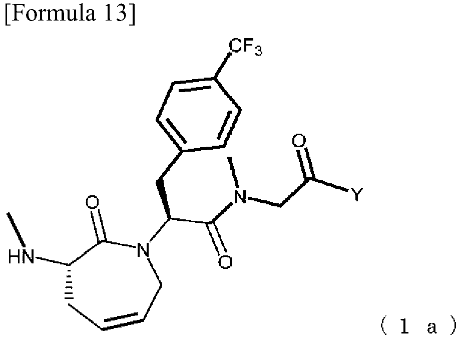 wherein Y is a hydroxyl group, optionally substituted C₁-C₁₀ alkoxy, optionally substituted C₆-C₁₆ aryloxy, optionally substituted C₇-C₁₄ alkoxy, or optionally substituted 3- to 12-membered cyclic aminooxy.

In an aspect, Y in formula (1a) is preferably C₁-C₈ alkoxy, C₆ aryloxy, C₁₀ aryloxy, C₇-C₁₆ aralkoxy, or 3- to 12-membered cyclic aminooxy, more preferably methoxy, ethoxy, tert-butoxy, phenoxy, 1-naphthyloxy, tolyloxy, benzyloxy, phenethyloxy, 1-pyrrolidyloxy, 1-piperidyloxy, or 1-piperazyloxy, and most preferably tert-butoxy.

In an aspect, the compound represented by formula (1a) or a salt thereof is tert-butyl N-methyl-N-((S)-2-((S)-3-(methylamino)-2-oxo-2,3,4,7-tetrahydro-1H-azepin-1-yl)-3-(4-(trifluoromethyl)phenyl)propanoyl)glycinate or a hydrochloride thereof.

In an aspect, the compound represented by formula (1) or a salt thereof, which is produced by the method of the present invention, may be a compound represented by formula (1b) or a salt thereof. 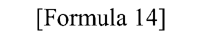 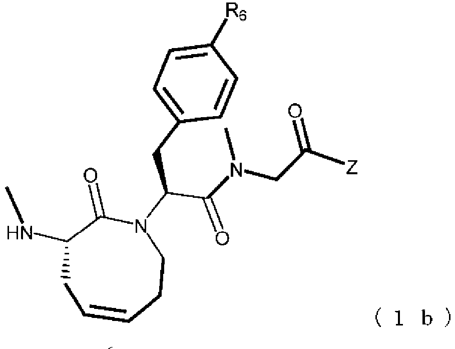 wherein R⁶ is methyl or trifluoromethyl, and Z is a hydroxyl group, optionally substituted C₁-C₁₀ alkoxy, optionally substituted C₆-C₁₆ aryloxy, optionally substituted C₇-C₁₄ alkoxy, or optionally substituted 3- to 12-membered cyclic aminooxy.

In an aspect, Z in formula (1b) is preferably C₁-C₈ alkoxy, C₆ aryloxy, C₁₀ aryloxy, C₇-C₁₆ aralkoxy, or 3- to 12-membered cyclic aminooxy, more preferably methoxy, ethoxy, tert-butoxy, phenoxy, 1-naphthyloxy, tolyloxy, benzyloxy, phenethyloxy, 1-pyrrolidyloxy, 1-piperidyloxy, or 1-piperazyloxy, and most preferably tert-butoxy.

In an aspect, R⁶ in formula (1b) is preferably methyl. In another aspect, R⁶ in formula (1b) is preferably trifluoromethyl.

In an aspect, the compound represented by formula (1b) or a salt thereof is tert-butyl N-methyl-N-((S)-2-((S,Z)-3-(methylamino)-2-oxo-3,4,7,8-tetrahydroazocin-1(2H)-yl)-3-(p-tolyl)propanoyl)glycinate or a hydrochloride thereof.

In an aspect, the compound represented by formula (1b) or a salt thereof is tert-butyl N-methyl-N-((S)-2-((S,Z)-3-(methylamino)-2-oxo-3,4,7,8-tetrahydroazocin-1(2H)-yl)-3-(4-(trifluoromethyl)phenyl)propanoyl)glycinate or a hydrochloride thereof.

In an aspect, the compound represented by formula (1b) or a salt thereof is tert-butyl N-methyl-N-((S)-2-((S,Z)-3-(methylamino)-2-oxo-2,3,4,5,8,9-hexahydro-1H-azonin-1-yl)-3-(p-tolyl)propanoyl)glycinate or a hydrochloride thereof.

In an aspect, the compound represented by formula (1b) or a salt thereof is tert-butyl (S)-N,N-dimethyl-2-((S,Z)-3-(methylamino)-2-oxo-3,4,7,8-tetrahydroazocin-1(2H)-yl)-3-(4-(trifluoromethyl)phenyl)propaneamide or a hydrochloride thereof.

In an aspect, the method for producing a compound represented by formula (1) or a salt thereof comprises the step (metathesis step) of bringing a compound represented by formula (2) or a salt thereof into contact with a catalyst. Formation of an impurity is suppressed as compared to a case where R¹ in formulae (1) and (2) is a carbamate protective group.

In an aspect, the method for producing a compound represented by formula (1) or a salt thereof comprises the step (metathesis step) of bringing a compound represented by formula (2) or a salt thereof into contact with a catalyst. Formation of a dimer is suppressed as compared to a case where R¹ in formulae (1) and (2) is a carbamate protective group.

In an aspect, the purity of the compound represented by formula (1) or a salt thereof is 90% or more, preferably 95% or more, more preferably 98% or more, and most preferably 99% or more as determined by a UVArea value at 210 nm from HPLC analysis.

In an aspect, the content ratio of an impurity contained in the compound represented by formula (1) or a salt thereof is less than 10%, preferably less than 5%, more preferably less than 1%, and most preferably less than 0.5% or an undetectable level as determined by a UVArea value at 210 nm from HPLC analysis.

Examples of the impurity as used herein may include a dimer in which compounds represented by formula (2) are intermolecularly linked. As such a dimer, a chain compound obtained by an intermolecular olefin metathesis reaction proceeding at one position (e.g., a compound represented by the following formula (8) in Examples 1 to 6) or a cyclic compound obtained by the reaction proceeding at two positions (e.g., a compound represented by the following formula (9) in Examples 1 to 6).

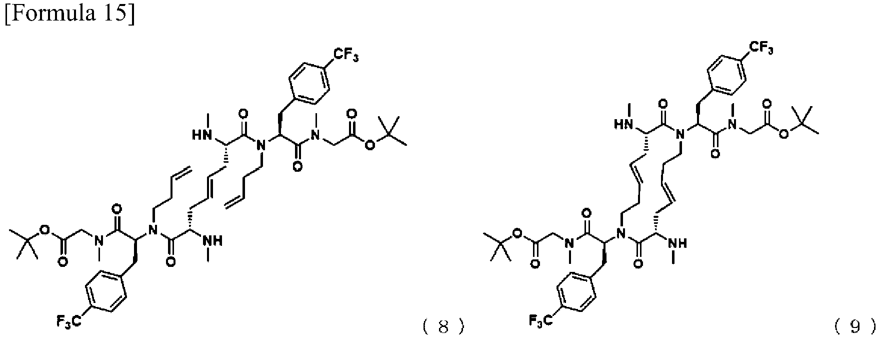

The impurity may include, in addition to compounds represented by formulae (8) and (9), compounds in which the olefin metathesis reaction occurs at a different position, and compounds having a different E/Z form of the double bond.

All references cited herein, including the following references, with inclusion of patent applications and publications, are incorporated herein by reference in their entirety: International Publication No. WO 2013/100132; International Publication No. WO 2018/225851; International Publication No. WO 2018/225864; International Publication No. WO 2019/117274; International Publication No. WO 2020/111238; International Publication No. WO 202012/2182; International Publication No. WO 2021/075478; International Publication No. WO 2021/090856; International Publication No. WO 2021132545; International Publication No. WO 2021246471; International Publication No. WO 2022/097540; International Publication No. WO 2022/138891; International Publication No. WO 20221/45444; International Publication No. WO 2022/234864; International Publication No. WO 2023/127869; International Publication No. WO 2022/234853; and International Publication No. WO 2023/214576.

### [Examples]

Hereinafter, the present disclosure will be described in more detail based on Examples, but the present disclosure is not limited to the following Examples.

In the following Examples, high performance liquid chromatography (HPLC) analysis was performed using any of the analytical conditions described below. Detection of each compound was performed using a photodiode array detector or mass spectrometer, although other techniques such as evaporative light scattering detection may also be used.

### HPLC analysis conditions: method 1

Apparatus: Waters ACQUITY UPLC H-Class + ACQUITY QDA
Column: Ascentis Express 90A C18 (Sigma-Aldrich Co. LLC), 2.1 mm ID × 50 mm, 2.7 µm
Mobile phase: 0.05% TFA/water (A), 0.05% TFA/MeCN (B)
Elution method: B) 5% (0 min) → 100% (5 min) → 5% (5.1 min) → 5% (7 min)
Flow rate: 0.5 mL/min
Column temperature: 35°C
Detection wavelength: 210 nm (PDA)

### HPLC analysis condition method 2

Apparatus: Waters ACQUITY UPLC H-Class
Column: Ascentis Express 90A C18 (Sigma-Aldrich Co. LLC), 2.1 mm ID × 50 mm, 2.7 µm
Mobile phase: 0.05% TFA/water (A), 0.05% TFA/MeCN (B)
Elution method: B): 5% (0 min) → 100% (5 min) → 5% (5.1 min) → 5% (7 min)
Flow rate: 0.5 mL/min
Column temperature: 35°C
Detection wavelength: 210 nm (PDA)

### HPLC analysis condition method 3

Apparatus: Waters ACQUITY UPLC H-Class + ACQUITY QDA
Column: Ascentis Express 90A C18 (Sigma-Aldrich Co. LLC), 2.1 mm ID × 50 mm, 2.7 µm
Mobile phase: 0.05% TFA/water (A), 0.05% TFA/MeCN (B)
Elution method: B) 50% (0 min)→100% (3 min) → 100% (5 min) → 50% (5.1 min) → 50% (7 min)
Flow rate: 0.5 mL/min
Column temperature: 35°C
Detection wavelength: 220 nm (PDA)

### HPLC analysis condition method 4

Apparatus: Waters ACQUITY UPLC H-Class
Column: Ascentis Express RP-amide(Sigma-Aldrich Co. LLC), 3.0 mm ID × 50 mm, 2.7 µm
Mobile phase: 0.05% TFA/water (A), 0.05% TFA/MeCN (B)
Elution method: B): 5% (0 min) → 100% (5 min) → 5% (5.1 min) → 5% (7 min)
Flow rate: 0.5 mL/min
Column temperature: 35°C
Detection wavelength: 210 nm (PDA)

### HPLC analysis condition method 5

Apparatus: Waters ACQUITY UPLC H-Class
Column: Ascentis Express 90A C18 (Sigma-Aldrich Co. LLC), 2.1 mm ID × 50 mm, 2.7 µm
Mobile phase: 0.05% TFA/water (A), 0.05% TFA/MeCN (B)
Elution method: B) 5% (0 min)→100% (5 min) → 100% (7 min) → 5% (7.1 min) → 5% (9 min)
Flow rate: 0.5 mL/min
Column temperature: 35°C
Detection wavelength: 210 nm (PDA)

The conversion ratio of the reaction mixture from HPLC analysis and the ratio of a dimer formed through the intermolecular reaction to the product were calculated as follows. 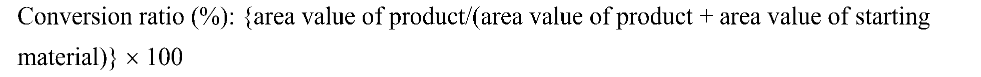 Conversion ratio (%): {area value of product/(area value of product + area value of starting material)} × 100 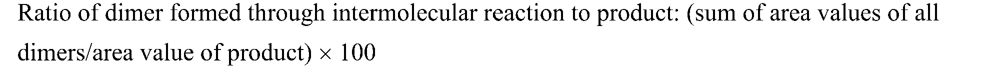 Ratio of dimer formed through intermolecular reaction to product: (sum of area values of all dimers/area value of product) × 100

¹H-NMR spectra were measured using a nuclear magnetic resonance device ECX500II (manufactured by JEOL Ltd.) and referenced to the deuterium lock signal from the sample solvent. As the sample solvent, commercially available deuterated solvents were used, depending on the purpose of measurement. The chemical shift of tetramethylsilane used as an internal standard was set to 0 ppm, and the chemical shift of the signal of the compound to be analyzed was expressed in ppm. Abbreviations of a signal were expressed as follows: s = singlet, brs = broad singlet, d = doublet, t = triplet, q = quartet, dd = double doublet, and m = multiplet. The splitting width of a signal was expressed as a J value (Hz). The integral value of signals was calculated on the basis of a ratio of the signal area intensity of each signal.

The measuring method by qNMR was performed by dissolving a residue containing a target compound and an internal standard substance in DMSO-d₆ and subjecting to the following analysis conditions.
Measuring apparatus: JNM-ECZ500R
Internal standard: 3,5-bis(trifluoromethyl)benzoic acid
Measurement conditions (¹H-NMR): DMSO-d₆, pulse angle 90°C, digital resolution 0.25 Hz, relaxation time 60 seconds, no spin, cumulative number 8 times

### Example 1-1

### Synthesis of (S)-2-(but-3-en-1-ylamino)-3-(4-(trifluoromethyl)phenyl)propionic acid (compound 1)

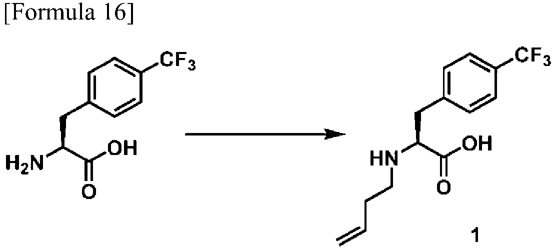

To a reaction vessel filled with nitrogen gas, acetonitrile (822 mL), 4-bromo-1-butene (235.07 g), and triethylamine (17.66 g) were added at an external temperature of 25°C, and the mixture was stirred for 1 hour. Subsequently, (S)-2-amino-3-(4-(trifluoromethyl)phenyl)propionic acid (135.46 g), water (676 mL), and triethylamine (158.19 g) were added, the external temperature was raised to 70°C, and the mixture was stirred for 3.5 hours. The reaction mixture was cooled to 25°C, and the precipitated solid was collected by filtration, and washed with a mixture of acetonitrile and water (v:v = 1:1, 676 mL). Subsequently, the obtained wet solid was further washed with acetonitrile (676 mL). The obtained wet solid was dried under reduced pressure at 40°C to afford compound 1 as a white solid (124.73 g).
LCMS (ESI) of compound 1, retention time: 2.34 minutes, m/z = 288 [M+H]⁺ (HPLC analysis conditions: method 1)

### Example 1-2

### Synthesis of tert-butyl (S)-N-(2-(but-3-en-1-ylamino)-3-(4-(trifluoromethyl)phenyl)propanoyl)-N-methylglycinate hydrochloride (compound 2-HCl)

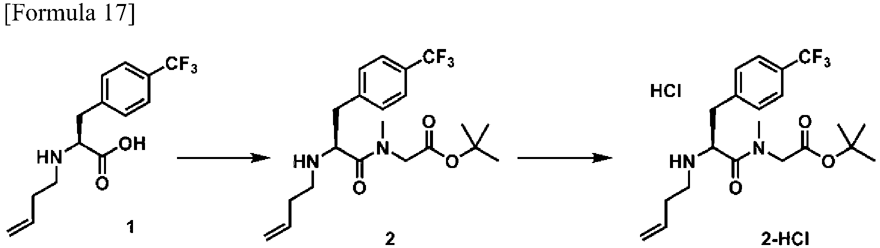

To a first reaction vessel filled with nitrogen gas, compound 1 (107.26 g) synthesized in Example 1-1, sarcosine tert-butyl ester hydrochloride (102.00 g), acetonitrile (751 mL) and DBU (233.03 g) were added at an external temperature of 25°C, and the mixture was stirred for 10 minutes. After the solution became homogeneous, the external temperature was set to 2°C and a 50% solution of T3P in 2-MeTHF (309.03 g) was added dropwise over 2 hours and 14 minutes. After the reaction was completed, toluene (751 mL) and a 1 mol/L aqueous sodium hydroxide solution (536 mL) were added to the reaction mixture. After the resulting mixture was stirred for 30 minutes, the aqueous layer was discharged. The organic layer was stored at approximately 25°C overnight. After storage, a 5% aqueous sodium carbonate solution (536 mL) was added to the organic layer, which was stirred for 10 minutes, and the aqueous layer was then discharged. Subsequently, a 5% aqueous sodium dihydrogen phosphate solution (751 mL) was added to the organic layer, which was stirred for 10 minutes, and the aqueous layer was then discharged. Further, a 5% aqueous sodium dihydrogen phosphate solution (751 mL) was added to the organic layer, which was stirred for 10 minutes, and the aqueous layer was then discharged. Subsequently, a 5% aqueous sodium chloride solution (751 mL) was added to the organic layer, which was stirred for 10 minutes, and the aqueous layer was then discharged. The organic layer was stored at an external temperature of 5°C overnight. After storage, the organic layer was concentrated under reduced pressure at 40°C until the liquid volume of the organic layer reached about 215 mL. Toluene (215 mL) was added to the concentrated solution, the mixture was concentrated under reduced pressure at 40°C to about 215 mL, and this operation was repeated two more times. The precipitated inorganic salt was filtered, and toluene (276.6 mL) was added to the obtained filtrate. In a second reaction vessel, pyridine hydrochloride (43.28 g) and acetonitrile (148 mL) were added, and the prepared solution was added dropwise over 45 minutes to the solution of compound 2 in toluene at an external temperature of 25°C. Precipitation of a crystal was confirmed during the dropwise addition. The second reaction vessel was rinsed using acetonitrile (74 mL), and the resulting solution was added to the mixture containing compound 2. The resulting mixture was stirred for 1 hour. Thereafter, toluene (1.7 L) was added, and the mixture was stirred for 1 hour, the external temperature was then lowered to 0°C, and the mixture was further stirred for 2 hours. The obtained crystal was filtered, and washed twice with toluene (296 mL) which was cooled to 0°C. The obtained wet solid was stored at an external temperature of 5°C for a weekend. After storage, the wet solid was dried under reduced pressure at the external temperature of 40°C to afford compound 2-HCl as a white solid (98.66 g).
LCMS (ESI) of compound 2, retention time: 3.03 minutes, m/z = 415 [M+H]⁺ (HPLC analysis conditions: method 1)

### Example 1-3

### Synthesis of (5S,8S)-5-allyl-7-(but-3-en-1-yl)-1-(9H-fluoren-9-yl)-4,10-dimethyl-3,6,9-trioxo-8-(4-(trifluoromethyl)benzyl)-2-oxa-4,7,10-triazadodecane-12-acid tert-butyl (compound Fmoc-3)

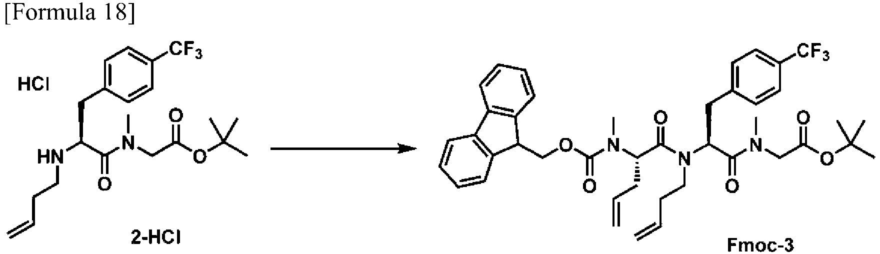

The compound 2-HCl (2.70 kg, 6.00 mol) synthesized in the above Example 1-2 was added to a reaction vessel. Then, (S)-2-(((9-H-fluoren-9-yl)methoxy)carbonyl)(methyl)amino)-4-pentenoic acid (2.74 kg), acetonitrile (6.34 kg) and MeTHF (5.74 kg) were added, the external temperature of the reaction vessel was set to 15°C, and DIPEA (3.86 kg) was added. Then, a solution obtained by dissolving TCFH (3.35 kg) in acetonitrile (7.58 kg) was added to the reaction vessel, the external temperature of the reaction vessel was set to 25°C, and the reaction mixture was then stirred for 5 hours. To the reaction vessel, MeTHF (5.74 kg) was added, the external temperature of the reaction vessel was then set to 15°C, a 5% aqueous potassium carbonate solution (13.45 kg) was then added, and the reaction mixture was then stirred for 30 minutes. The aqueous layer was discharged, and the obtained organic layer was then washed with 5% sulfuric acid (13.45 kg). To the reaction vessel, MeTHF (5.74 kg) was added, and the organic layer was then washed with a 5% aqueous potassium carbonate solution (13.45 kg). The obtained organic layer was concentrated under reduced pressure, acetonitrile (16.91 kg) was added, and the mixture was further concentrated to afford a residue containing compound Fmoc-3.
LC retention time of compound Fmoc-3: 5.442 minutes (HPLC analysis conditions: method 2) LCMS (ESI) of compound Fmoc-3, retention time: 5.491 minutes, m/z = 770 [M+Na]⁺ (HPLC analysis conditions: method 1)

### Example 1-4

### Synthesis of tert-butyl N-((S)-2-((S)-N-(but-3-en-1-yl)-2-(methylamino)pent-4-enamide)-3-(4-(trifluoromethyl)phenyl)propanoyl)-N-methylglycinate hydrochloride (compound 3-HCl)

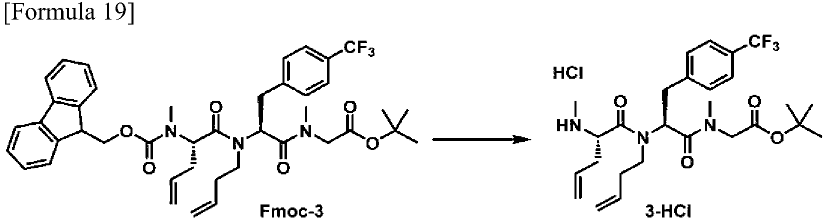

The residue containing compound Fmoc-3 obtained in the above Example 1-3 was added to a reaction vessel. DBU (2.59 kg) was then added at 15°C, and the resulting mixture was stirred for 1 hour. Triethylamine (2.29 kg) and sodium hydrogen sulfite (2.12 kg) were then added, and the reaction mixture was stirred at 25°C for 1 hour. To the reaction vessel, toluene (18.38 kg) and 10% aqueous ammonia (33.92 kg) were added, the mixture was stirred for 10 minutes, and the aqueous layer was then discharged. The obtained organic layer was washed three times with 10% aqueous ammonia (33.92 kg), and then with a 5% aqueous sodium chloride solution (33.92 kg). The obtained organic layer was concentrated under reduced pressure. To the obtained residue, toluene (7.7 kg) was added, and the mixture was concentrated under reduced pressure. The addition of toluene (7.7 kg) to the obtained residue and the operation of concentration under reduced pressure were repeated twice to afford a residue containing compound 3.

To the obtained residue, toluene (0.86 kg) was added, and a solution obtained by dissolving pyridine hydrochloride (0.65 kg) in acetonitrile (3.17 kg) was then added at 25°C. The resulting mixture was stirred for 1 hour, MTBE (11.92 kg) was then added, and the mixture was stirred. The resulting precipitate was collected by filtration under reduced pressure, and the obtained wet solid was washed twice with MTBE (3.97 kg). The obtained solid was dried under reduced pressure to afford compound 3-HCl (2.60 kg, 4.63 mol, yield in two steps from compound 2-HCl: 77.5%).
UV intensity ratio: 100%
(Detection wavelength 210 nm, retention time 3.145 minutes, HPLC analysis conditions: method 2)
LCMS (ESI) of compound 3, retention time: 3.213 minutes, m/z = 526 [M+H]⁺ (HPLC analysis conditions: method 1)

### Example 1-5

### Synthesis of compound Fmoc-4 by ring-closing metathesis of compound Fmoc-3

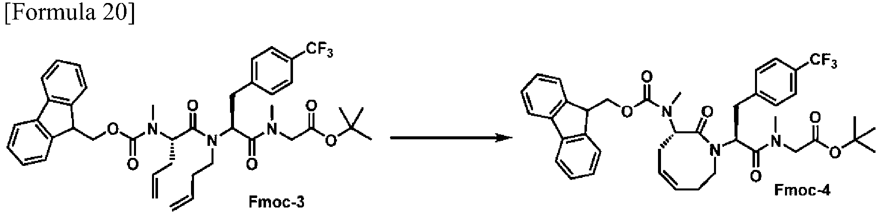

To the residue (55 ml) containing compound Fmoc-3 obtained in the above Example 1-3, acetonitrile (20 mL) was added, and azeotropic dehydration under reduced pressure was repeated twice. The resultant residue was purified by silica gel column chromatography (mobile phase: heptane/ethyl acetate, volume ratio 0 : 100 → 40 : 60). The collected solution was concentrated, and MTBE (20 mL) was added to the obtained concentrated mixture to precipitate compound Fmoc-3. The obtained solid was filtered to afford Fmoc-3 (4.90 g) as a white solid.

To a first reaction vessel, Fmoc-3 (1.00 g, 1.34 mmol) was added, and dissolved with acetone (7 mL) and HFIP (4.46 g, 2.8 mL) to prepare a solution of Fmoc-3. To a second reaction vessel, Hoveyda-Grubbs catalyst ([1-[2,4,6-trimethylphenyl]-3,5,5-trimethyl-3-phenyl-2-pyrrolinylidene]dichloro(2-isopropoxybenzylidene)ruthenium (II)) (8.5 mg, 0.013 mmol) and acetone (5 mL) were added. After the second reaction vessel was filled with nitrogen gas, the solution of compound Fmoc-3 prepared as described above was added dropwise over 2 hours while the solution in the second reaction vessel was heated and refluxed at 65°C. Then, the first reaction vessel was washed with acetone (1 mL), and the obtained wash solution was added dropwise over 10 minutes to the solution in the second reaction vessel. After completion of the dropwise addition, the reaction mixture was stirred for 4 hours, and the reaction mixture was sampled, and analyzed by HPLC. It was confirmed that the conversion ratio was 95.2%, and the ratio of the dimer formed through the intermolecular reaction to the product (Fmoc-4) was 361%.
LCMS (ESI) of compound Fmoc-4, retention time: 2.784 minutes, m/z = 742 [M+Na]⁺ (HPLC analysis conditions: method 3)

### Example 1-6

### Synthesis of compound 4-HCl by ring-closing metathesis of compound 3-HCl

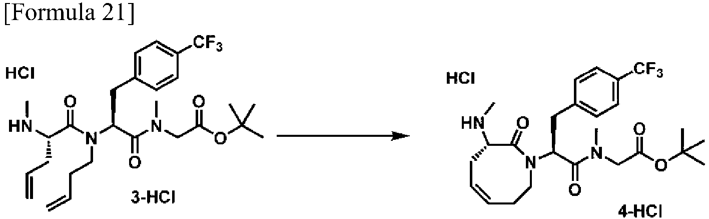

To a reaction vessel, compound 3-HCl (50.2 mg) obtained in the above Example 1-4 was added, and dissolved with acetone (0.35 mL) and HFIP (0.14 mL). Separately, a catalyst solution was prepared by dissolving Hoveyda-Grubbs catalyst ([1-[2,4,6-trimethylphenyl]-3,5,5-trimethyl-3-phenyl-2-pyrrolinylidene]dichloro(2-isopropoxybenzylidene)ruthenium (II)) (2.8 mg) in acetone (0.25 mL), and the resulting catalyst solution was added to the reaction vessel. The resulting reaction mixture was heated and refluxed at 65°C with stirring for 5 hours. After the reaction, the reaction mixture was sampled, and analyzed by HPLC. It was confirmed that the conversion ratio was 99.3%, and the ratio of the dimer formed through the intermolecular reaction to the product (4-HCl) was 0.42%.
LCMS (ESI) of compound 4, retention time: 2.975 minutes, m/z = 498 [M+H]⁺ (HPLC analysis conditions: method 1)

Table 1 shows comparison between the above Examples 1-5 and 1-6.

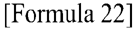

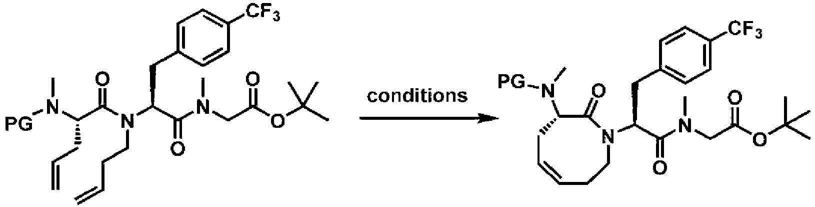

**[Table 1]**

| PG | Reaction conditions | Conversion ratio (%) | Dimer ratio (%) |
|---|---|---|---|
| H (HCl salt) | 5 mol % catalyst* | 99.3 | 0.42 |
| | Acetone (12 v/w) | | |
| | HFIP (2.8 v/w) reflux, 5 h | | |
| Fmoc | 1 mol % catalyst* | 95.2 | 361 |
| | Acetone (13 v/w) | | |
| | HFIP (2.8 v/w) reflux, 4h | | |

| | | | |
|---|---|---|---|
| * Hoveyda-Grubbs catalyst ([1-[2,4,6-trimethylphenyl]-3,5,5-trimethyl-3-phenyl-2-pyrrolinylidene]dichloro(2-isopropoxybenzylidene)ruthenium (II)) | | | |

It was confirmed that the use of the unprotected compound 3-HCl allowed the reaction to proceed in an intramolecularly selective manner as compared to the use of the Fmoc-protected compound Fmoc-3 as shown in Table 1.

### Example 1-7

### Synthesis of tert-Butyl N-methyl-N-((S)-2-((S,Z)-3-(methylamino)-2-oxo-3,4,7,8-tetrahydroazocin-1(2H)-yl)-3-(4-(trifluoromethyl)phenyl)propanoyl)glycinate hydrochloride (compound 4-HCl)

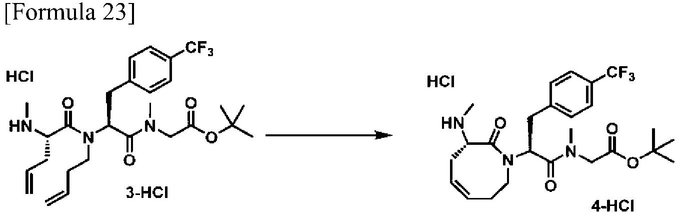

To a first reaction vessel, compound 3-HCl (0.64 kg) obtained in the above Example 1-4 was added, and the mixture was dissolved with acetone (3.52 kg) and HFIP (2.88 kg) to prepare a solution of the compound 3-HCl. In a second reaction vessel, a catalyst solution was prepared using Hoveyda-Grubbs catalyst ([1-[2,4,6-trimethylphenyl]-3,5,5-trimethyl-3-phenyl-2-pyrrolinylidene]dichloro(2-isopropoxybenzylidene)ruthenium (II)) (7.12 g) and acetone (2.51 kg), and then the second reaction vessel was filled with nitrogen gas. Then, the compound 3-HCl solution prepared as described above was added to the second reaction vessel dropwise over 1.5 hours while the catalyst solution in the second reaction vessel was heated and refluxed at 65°C. After completion of dropwise addition, the reaction mixture was stirred for 1 hour, allowed to cool to 25°C, and then stirred for another hour. The resulting precipitate was collected by filtration, and the obtained wet solid was rinsed twice with acetone (2.52 kg). The obtained solid was dried under reduced pressure to afford 0.55 kg of compound 4-HCl (yield 90.7%).
UV intensity ratio: 99.1%
(Detection wavelength 210 nm, retention time 2.820 minutes, HPLC analysis conditions: method 2)
LCMS (ESI) of compound 4, retention time: 2.859 minutes, m/z = 498 [M+H]⁺ (HPLC analysis conditions: method 1)
¹H-NMR (500 MHz, DMSO-d₆, detected as a mixture of rotamers) δ 9.74 (brs, 1H), 8.63 (brs, 1H), 7.60-7.39 (m, 4H), 5.78-5.39 (m,3H), 4.53-4.48 (m, 1H), 4.18-3.74 (m, 3H), 3.61-3.50 (m, 1H), 3.11-2.83 (m,6H), 2.45-2.41 (m, 1H), 2.30-2.12 (m, 2H), 1.96 (s, 1H), 1.87 (s, 2H), 1.40 (s,3H), 1.39 (s, 6H)

### Example 1-8

### Study on additives in ring-closing metathesis of compound 3-HCl

To a reaction vessel, the compound 3-HCl obtained in the above Example 1-4, acetone (10 volumes based on the weight of compound 3-HCl), additives (2 volumes based on the weight of compound 3-HCl) and second-generation Hoveyda-Grubbs catalyst (5 mol%) were added, and then the reaction vessel was filled with nitrogen gas. The resulting mixture was stirred for 3-3.5 hours with an external temperature of 55°C. The resulting reaction mixture was sampled and analyzed by HPLC to determine a conversion ratio. Table 2 below shows a list of the additives used and conversion ratios.

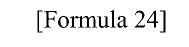

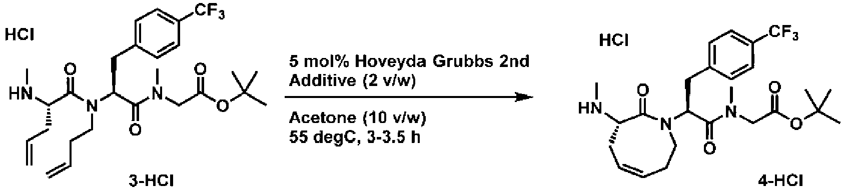

**[Table 2]**

| Additive | Conversion ratio (%) |
|---|---|
| HFIP | 76.0 |
| 2-propanol | 79.5 |
| MeOH | 60.9 |
| TFE | 79.5 |
| AcOH | 96.0 |

It was confirmed that the target 4-HCl was generated with a conversion ratio of 70% or more even when a plurality of weak acids and alcohols were used as additives as shown in Table 2.

### Example 1-9

### Synthesis of tert-butyl N-((S)-2-((S,Z)-3-((((9H-fluoren-9-yl)methoxy)carbonyl)(methyl)amino)-2-oxo-3,4,7,8-tetrahydroazocin-1(2H)-yl)-3-(4-(trifluoromethyl)phenyl)propanoyl)-N-methylglycinate (compound Fmoc-4)

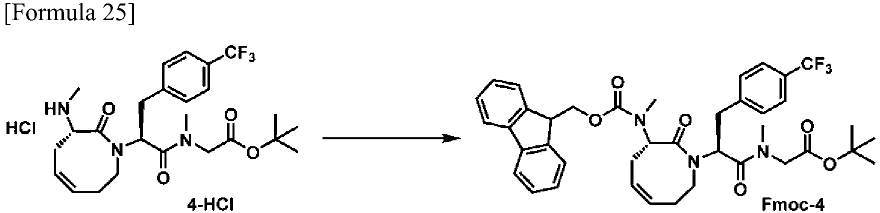

A reaction vessel was filled with nitrogen gas, and compound 4-HCl (3.14 kg, 5.88 mol) prepared in the same manner as in the above Example 1-7, acetonitrile (7.34 kg), MeTHF (2.54 kg) and DIPEA (1.90 kg) were added to the reaction vessel. Then, a solution obtained by dissolving 9-fluorenylmethyl chloroformate (1.60 kg) in MeTHF (5.36 kg) was added to the reaction vessel, and the mixture was stirred at 15°C for 1 hour. To the reaction vessel, N,N-dimethylethylenediamine (0.10 kg) was added, and the mixture was stirred at 25°C for 0.5 hours. Then, 4% sulfuric acid (31.35 kg) was added at 15°C, the mixture was stirred, and the aqueous layer was discharged. To the obtained organic layer, MeTHF (5.38 kg) and 4% sulfuric acid (31.47 kg) were added, the mixture was stirred, and the aqueous layer was discharged. To the obtained organic layer, MeTHF (5.38 kg) and a 5% aqueous sodium carbonate solution (31.39 kg) were added, the mixture was stirred, and the aqueous layer was discharged. To the obtained organic layer, MeTHF (5.38 kg) and a 5% aqueous sodium chloride solution (31.43 kg) were added, the mixture was stirred, and the aqueous layer was discharged. The obtained organic layer was concentrated, and azeotropic dehydration with the addition of MeTHF was performed to afford a concentrated solution containing compound Fmoc-4.
LC retention time of compound Fmoc-4: 4.980 minutes (HPLC analysis conditions: method 4)

In the ring-closing metathesis of Fmoc-3, compounds derived from intermolecular reactions are main products. On the other hand, Fmoc-4 was successfully obtained as a main product via a sequence of ring-closing metathesis of 3-HCl and subsequent Fmoc protection of the N-terminus.

### Example 1-10

### Synthesis of tert-Butyl N-methyl-N-((S)-2-((S)-3-(methylamino)-2-oxoazocan-1-yl)-3-(4-(trifluoromethyl)phenyl)propanoyl)glycinate hydrochloride (compound 5-HCl)

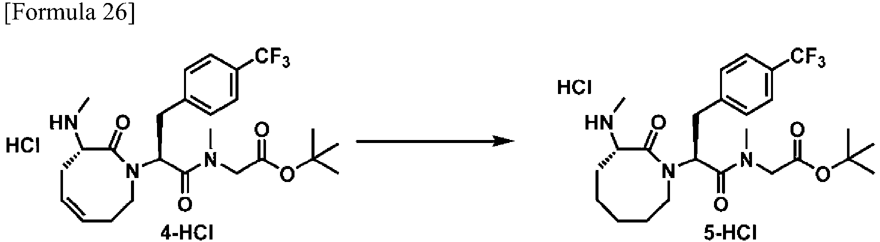

The compound 4-HCl (240 mg, 0.45 mmol) obtained in the above Example 1-7, methanol (4.8 mL) and 10% Pd/C (water content 54%, 51.8 mg) were added to a reaction vessel, and the reaction vessel was then filled with hydrogen gas. The mixture was stirred at approximately 25°C for 75 minutes, the reaction mixture was then filtered, and palladium carbon separated by filtration was washed with methanol (3 mL). The obtained filtrate was dried under reduced pressure to afford compound 5-HCl (221 mg, 0.41 mmol, yield 91.9%) as a white solid.
UV intensity ratio: 99.5%
(Detection wavelength 210 nm, retention time 2.808 minutes, HPLC analysis conditions: method 1, m/z = 500 [M+H]⁺ (ESI))

The compound obtained by ring-closing metathesis was successfully converted to the corresponding lactam 5-HCl by hydrogenation of the double bond into a single bond.

### Example 1-11

### Synthesis of (S)-2-(but-3-en-1-ylamino)-3-(p-tolyl)propanoic acid (compound 6)

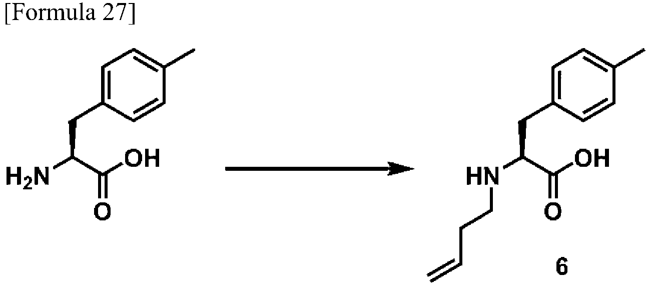

(S)-2-amino-3-(p-tolyl)propanoic acid (3.45 g, 19.3 mmol), ethanol (20.7 mL), water (17.3 ml), triethylamine (8.05 mL, 57.8 mmol), and 4-bromo-1-butene (5.82 mL, 57.8 mmol) were added to a reaction vessel, and the resulting mixture was stirred at 70°C for 4 hours. The reaction mixture was cooled to 25°C, the resulting precipitate was then collected by filtration, and the obtained solid was dried under reduced pressure to afford compound 6 (2.40 g, yield 53%) as a white solid.
UV intensity ratio: 99.0%
(Detection wavelength 210 nm, retention time 1.983 minutes, HPLC analysis condition method 2)

### Example 1-12

### Synthesis of tert-butyl (S)-N-(2-(but-3-en-1-ylamino)-3-(p-tolyl)propanoyl)-N-methylglycinate (compound 7)

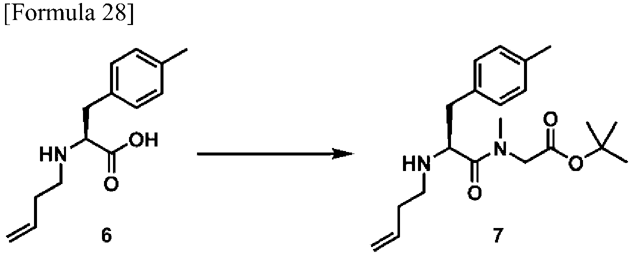

Compound 6 (2.05 g, 8.79 mmol) obtained in the above Example 1-11, sarcosine tert-butyl hydrochloride (3.20 g, 17.6 mmol), acetonitrile (20 mL) and DIPEA (6.91 mL, 39.5 mmol) were added at approximately 25°C to a reaction vessel. After the reaction vessel was filled with nitrogen gas, chlorotripyrrolidinophosphonium hexafluorophosphate (5.60 g, 13.3 mmol) was then added, and the mixture was stirred at 72°C for 5 hours. The reaction mixture was cooled to 25°C, a 10% aqueous potassium carbonate solution (10 mL) was then added, and the mixture was stirred for 6 hours. The aqueous layer was discharged, and the obtained organic layer was washed with a 10% aqueous sodium hydrogen sulfate solution (16 mL × 2 times). To the obtained organic layer, a 1 mol/L aqueous sodium hydroxide solution (10 mL) and MeTHF (10 mL) were added, the mixture was stirred, and the aqueous layer was discharged. To the obtained organic layer, toluene (10 mL) was added, and the mixture was washed with water (10 mL × 2) and a 5% aqueous sodium chloride solution (10 mL). The obtained organic layer was concentrated, and azeotropic dehydration with the addition of toluene (20 mL) was performed to afford a residue (5.08 g) containing compound 7.
LCMS (ESI) of compound 7, retention time: 2.756 minutes, m/z = 361 [M+H]⁺ (HPLC analysis conditions: method 1)

### Example 1-13

### Synthesis of tert-butyl N-((S)-2-((S)-N-(but-3-en-1-yl)-2-(methylamino)pent-4-enamide)-3-(p-tolyl)propanoyl)-N-methylglycinate (compound 8)

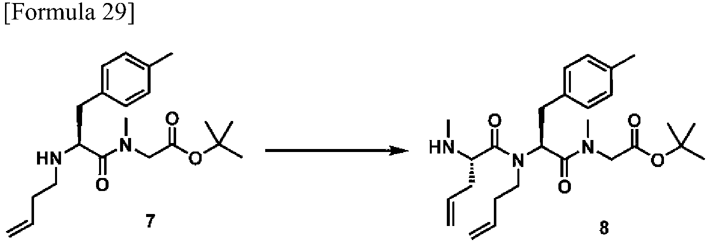

The residue (5.75 g) containing compound 7 (10.6 mmol) obtained by the method of the above Example 1-12, (S)-2-(((9-H-fluoren-9-yl)methoxy)carbonyl)(methyl)amino)-4-pentenoic acid (4.50 g, 12.8 mmol), acetonitrile (19 mL) and DIPEA (7.43 mL) were added at approximately 25°C to a reaction vessel. After the reaction vessel was filled with nitrogen gas, TCFH (5.97 g, 21.3 mmol) was then added, and the mixture was stirred at 25°C for 4 hours. Then, MTBE (19 mL), a 5% aqueous potassium carbonate solution (19 mL), and 1-methylimidazole (0.85 mL) were added, and the mixture was stirred at approximately 25°C for 10 minutes. The aqueous layer was discharged, and the obtained organic layer was washed with a 10% aqueous sodium hydrogen sulfate solution (19 mL × 2) and a 5% aqueous sodium chloride solution (19 mL). The obtained organic layer was concentrated, DBU (4.81 mL) and acetonitrile (7.6 mL) were then added. The resulting mixture was stirred at approximately 25°C for 45 minutes. Then, sodium hydrogen sulfite (2.76 g), triethylamine (5.93 mL) and water (1.92 mL) were added, and the mixture was stirred at approximately 25°C for 2 hours. Then, toluene (19 mL), isopropyl acetate (19 mL) and 20% aqueous ammonia (36 mL) were added, and the mixture was stirred. The aqueous layer was discharged, and the obtained organic layer was then washed with 20% aqueous ammonia (36 mL × 3) and an aqueous sodium chloride solution (19 mL). The obtained organic layer was concentrated under reduced pressure, and subsequently, azeotropic dehydration with the addition of toluene (19 mL × 2) was performed to afford a residue (9.01 g) containing compound 8.
LCMS (ESI) of compound 8, retention time: 3.038 minutes, m/z = 472 [M+H]⁺ (HPLC analysis conditions: method 1)

### Example 1-14

### Synthesis of tert-Butyl N-methyl-N-((S)-2-((S,Z)-3-(methylamino)-2-oxo-3,4,7,8-tetrahydroazocin-1(2H)-yl)-3-(p-tolyl)propanoyl)glycinate (compound 9)

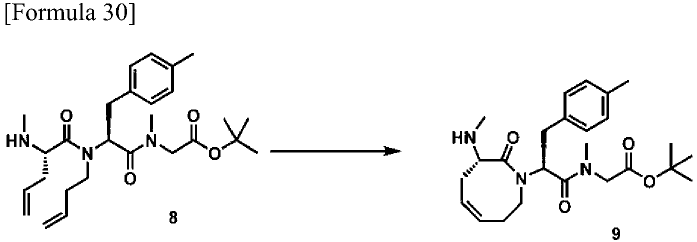

A purified product of compound 8 synthesized in the above Example 1-13 (purified with a silica gel column chromatography using dichloromethane/methanol as a mobile phase) (41.6 mg, 0.088 mmol) was added to a reaction vessel. Then, a toluene solution (0.45 mL) containing toluene (2.2 mL), boron trifluoride-tetrahydrofuran complex (9.7 µL, 0.088 mmol) and first-generation Hoveyda-Grubbs catalyst (2.6 mg, 4.4 µmol) was added to the reaction vessel. After the reaction vessel was filled with nitrogen gas, the resulting mixture was stirred at an external temperature of 40°C for 3 hours. The reaction mixture was then sampled, and analyzed by HPLC. It was confirmed that the conversion ratio was 74.8%, and the ratio of the dimers, formed through the intermolecular reactions, to the product (9) was 1.7%.
LCMS (ESI) of compound 9, retention time: 2.492 minutes, m/z = 444 [M+H]⁺ (HPLC analysis conditions: method 1)

### Example 1-15

### Synthesis of (5S,8S)-5-allyl-7-(but-3-en-1-yl)-4,10-dimethyl-8-(4-methylbenzyl)-3,6,9-trioxo-1-phenyl-2-oxa-4,7,10-triazadodecane-12-acid tert-butyl (compound Cbz-8)

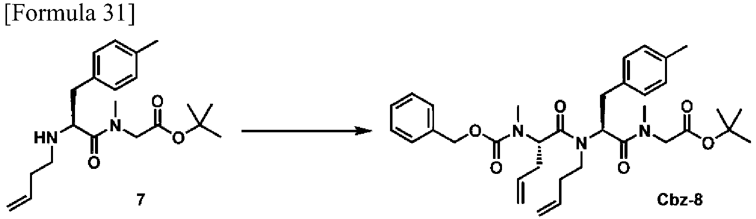

A compound 7 (0.50 g, 1.39 mmol) synthesized by a method similar to that of the above Example 1-12 was added to a reaction vessel, and (S)-2-((benzyloxy)carbonyl)(methyl)amino)-4-pentenoic acid (0.43 g, 1.66 mmol), acetonitrile (2.5 mL) and DIPEA (1.09 mL) were then added. TCFH (0.78 g) was added at approximately 25°C, and the resulting mixture was stirred at approximately 25°C for 1 hour. Then, MeTHF (2.5 mL) and a 5% aqueous sodium hydrogen carbonate solution (5 mL) were added, the mixture was stirred, and the aqueous layer was then discharged. The organic layer was washed with a 5% aqueous sodium hydrogen carbonate solution (5 mL), a 5% aqueous potassium hydrogen sulfate solution (5 mL) and a 2.5% aqueous sodium chloride solution (5 mL) in this order. The obtained organic layer was concentrated to afford a residue containing compound Cbz-8. The residue was purified by silica gel column chromatography (mobile phase: heptane/ethyl acetate, volume ratio 100 : 0 → 80 : 20) to afford compound Cbz-8 (0.66 g).
LCMS (ESI) of compound Cbz-8: retention time, 4.867 minutes, m/z = 628 [M+Na]⁺ (HPLC analysis conditions: method 1)

### Example 1-16

### Synthesis of tert-Butyl N-((S)-2-((S,Z)-3-(((benzyloxy)carbonyl)(methyl)amino)-2-oxo-3,4,7,8-tetrahydroazocin-1(2H)-yl)-3-(p-tolyl)propanoyl)-N-methylglycinate (compound Cbz-9)

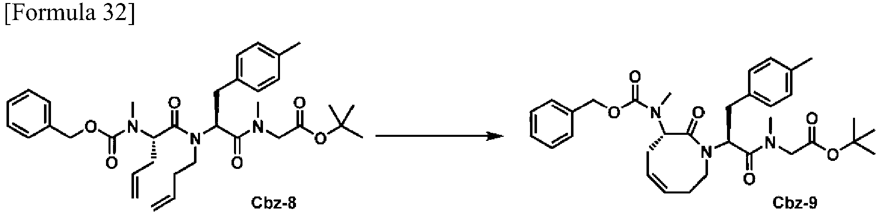

Compound Cbz-8 (48.6 mg, 0.080 mmol) obtained in the above Example 1-15, toluene (2.67 mL), first-generation Hoveyda-Grubbs catalyst (2.3 mg, 3.9 µmol) were added to a reaction vessel. After the reaction vessel was filled with nitrogen gas, the reaction mixture was stirred at an external temperature of 70°C for 2 hours. The reaction mixture was then sampled, and analyzed by HPLC. It was confirmed that the conversion ratio was 39.4%, and the ratio of the dimers, formed through the intermolecular reactions, to the product (Cbz-9) was 35.9%. LCMS (ESI) of compound Cbz-9: retention time, 1.877 minutes, m/z = 600 [M+Na]⁺ (HPLC analysis conditions: method 3)

Table 3 below shows comparison between the ring-closing metathesis of compound 8 in which the N-terminus is not protected (Example 1-14) and the ring-closing metathesis of the Cbz-protected compound Cbz-8 (Example 1-16).

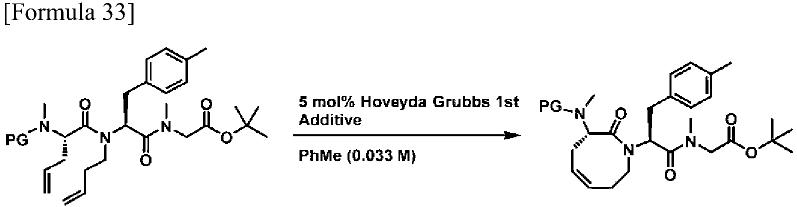

**[Table 3]**

| PG | Additive | Temperature | Time | Conversion ratio (%) | Dimer ratio (%) |
|---|---|---|---|---|---|
| H | - | 70°C then 100°C | 1.5 h then 1 h | 0 | - |
| H | BF₃THF 1 eq | 40°C | 3h | 74.8 | 1.7 |
| Cbz | - | 70°C | 2h | 39.4 | 35.8 |

It was confirmed that the use of the unprotected compound 8 allowed the ring-closing metathesis reaction to proceed with a higher conversion ratio and in an intramolecularly selective manner as compared to the use of the Cbz-protected compound Cbz-8.

### Example 1-17

### Synthesis of tert-butyl N-((S)-2-((S)-N-(but-3-en-1-yl)-2-(methylamino)pent-4-enamide)-3-(p-tolyl)propanoyl)-N-methylglycinate hydrochloride (compound 8-HCl)

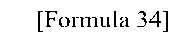

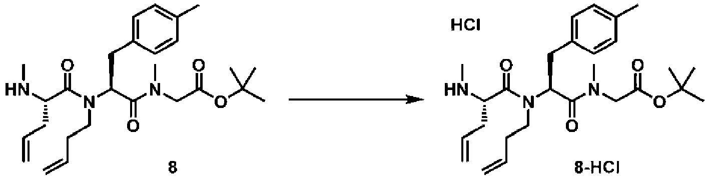

To the residue (9.01 g) containing the compound 8 obtained by the method of the above Example 1-13, a pyridine hydrochloride solution obtained by dissolving pyridine hydrochloride (0.86 g) in acetonitrile (1.9 mL) was added followed by an addition of MTBE (11.4 mL). The resulting mixture was stirred at approximately 25°C for 10 minutes, and MTBE (7.6 mL) was added. The reaction mixture was stirred for 1 hour, MTBE (19 mL) was then added, and the mixture was stirred for 1 hour, and then left to stand for 15 hours. The resulting precipitate was collected by filtration, and the obtained solid was dried under reduced pressure to afford 2.79 g of compound 8-HCl (content 97.5%, product of NMR content calculated by qNMR and UV intensity ratio) as a beige solid.
UV intensity ratio: 99.1%
(Detection wavelength 210 nm, retention time 3.042 minutes, HPLC analysis conditions: method 1, m/z = 472 [M+H]⁺ (ESI))

### Example 1-18

### Synthesis of tert-Butyl N-methyl-N-((S)-2-((S,Z)-3-(methylamino)-2-oxo-3,4,7,8-tetrahydroazocin-1(2H)-yl)-3-(p-tolyl)propanoyl)glycinate hydrochloride (compound 9-HCl)

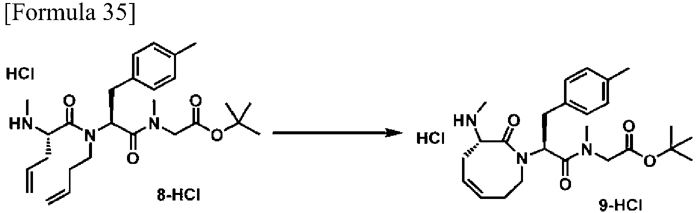

In a first reaction vessel, compound 8-HCl (1.02 g, content 97.5%) obtained in Example 1-17 was dissolved in acetone (5 mL) and HFIP (0.41 mL) to prepare a compound 8-HCl solution. To a second reaction vessel, second-generation Hoveyda-Grubbs catalyst (0.061 g), acetone (15 mL) and HFIP (1.8 mL) were added to prepare a catalyst solution. The second reaction vessel was filled with nitrogen gas, and the catalyst solution in the second vessel was heated and refluxed at an external temperature of 65°C. The compound 8-HCl solution prepared as described above was then added to the second reaction vessel dropwise over 1 hour. After completion of the dropwise addition, the reaction mixture was stirred for 1 hour, and the reaction mixture was sampled, and analyzed by HPLC. It was confirmed that the conversion ratio was 99.3%, and the ratio of the dimers, formed through the intermolecular reactions, to the product (9-HCl) was 1.7%. The reaction mixture was cooled to approximately 25°C, MTBE (10 mL) was added to the reaction mixture, and the resulting suspension was then filtered. Then, the obtained wet solid was washed twice with a mixed solution of acetone/MTBE (volume ratio 2 : 1, 5 mL). The obtained solid was dried under reduced pressure to afford 0.79 g of a compound 9-HCl (81.4%, content 97.0%, product of NMR content calculated by qNMR and UV intensity ratio) as a white solid.
UV intensity ratio: 98.6%
(Detection wavelength 210 nm, retention time 2.630 minutes, HPLC analysis conditions: method 1, m/z = 444 [M+H]⁺ (ESI))
¹H-NMR (500 MHz, DMSO-d₆, detected as a mixture of rotamers) δ 8.93-9.54 (br, 1H), 8.29-8.93 (br, 1H), 7.03-7.14 (m, 4H),5.70-5.36 (m, 3H), 4.49-4.45 (m, 1H), 4.23-3.80 (m, 2H), 3.73-3.67 (m, 1H),3.59-3.49 (m, 1H), 2.96-2.78 (m, 6H), 2.44-2.40 (m, 1H), 2.28-2.09 (m, 5H),1.94 (s, 1.2H), 1.92 (s, 1.8H), 1.43 (s, 3.5H), 1.40 (s, 5.5H)

By using compound 8-HCl in which the N-terminus is not protected, a ring-closing metathesis reaction successfully proceeded to afford cyclic compound 9-HCl in ≥80% yield with an LC purity of ≥98%.

### Example 1-19

### Synthesis of (S)-2-(arylamino)-3-(4-(trifluoromethyl)phenyl)propanoic acid (compound 10)

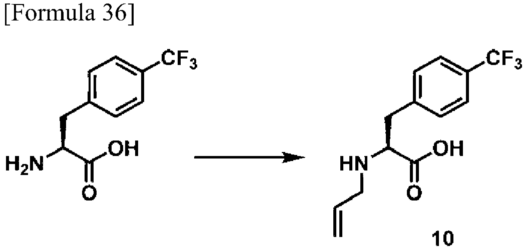

(S)-2-amino-3-(4-(trifluoromethyl)phenyl)propanoic acid (3.34 g, 14.3 mmol), acetonitrile (10 mL), water (8.3 ml), triethylamine (5.99 mL, 43.0 mmol), and allyl bromide (3.72 mL, 43.0 mmol) were added to a reaction vessel, and the reaction mixture was stirred at approximately 25°C for 1 hour. The resulting precipitate was collected by filtration, and the obtained solid was dried under reduced pressure to afford compound 10 (3.05 g, yield 77.9%) as a white solid.
UV intensity ratio: 96.4%
(Detection wavelength 210 nm, retention time 1.925 minutes, HPLC analysis conditions: method 1, m/z = 274 [M+H]⁺ (ESI))

### Example 1-20

### Synthesis of tert-butyl (S)-N-(2-(arylamino)-3-(4-(trifluoromethyl)phenyl)propanoyl)-N-methylglycinate (compound 11)

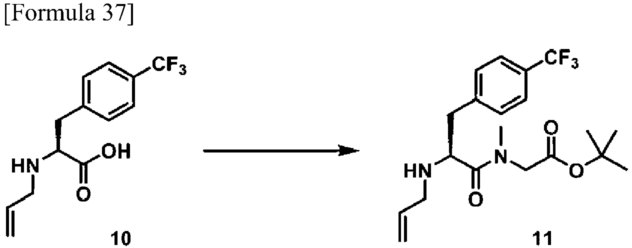

To a reaction vessel, compound 10 (2.51 g, 9.19 mmol) obtained in the above Example 1-19, sarcosine tert-butyl hydrochloride (2.52 g, 13.9 mmol), acetonitrile (18 mL) and DBU (5.68 mL, 37.7 mmol) were added at approximately 25°C. T3P (50 wt% MeTHF solution, 7.38 mL) was then added to the reaction vessel at the external temperature of 0°C, and the resulting mixture was stirred at approximately 25°C for 1 hour. Toluene (20 mL) and a 1 mol/L aqueous sodium hydroxide solution (13 mL) were added, the mixture was stirred for 15 minutes, and the aqueous layer was discharged. The obtained organic layer was washed with a 5% aqueous sodium carbonate solution (13 mL), a 5% aqueous sodium dihydrogen phosphate solution (20 mL × 3 times) and a 10% aqueous sodium chloride solution (5 mL) in this order. The obtained organic layer was concentrated under reduced pressure, and azeotropic dehydration with the addition of toluene (20 mL) was performed. Filtration and concentration of the resulting mixture afforded a residue (5.80 g) containing compound 11.
LCMS (ESI) of compound 11, retention time: 2.768 minutes, m/z = 401 [M+H]⁺ (HPLC analysis conditions: method 1)

### Example 1-21

### Synthesis of (5S,8S)-5,7-diallyl-1-(9H-fluoren-9-yl)-4,10-dimethyl-3,6,9-trioxo-8-(4-(trifluoromethyl)benzyl)-2-oxa-4,7,10-triazadodecane-12-acid tert-butyl (compound Fmoc-12)

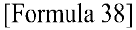

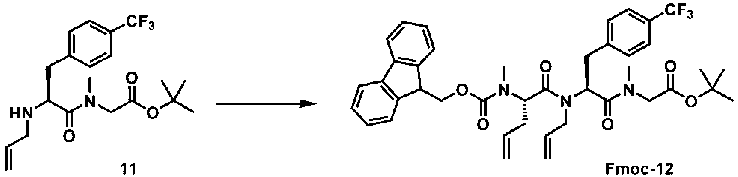

To a reaction vessel, a residue (5.41 g) containing compound 11 obtained by the same method as in the above Example 1-20, (S)-2-((9-H-fluoren-9-yl)methoxy)carbonyl)(methyl)amino)-4-pentenoic acid (3.92 g), acetonitrile (17 mL), MeTHF (9 mL) and DIPEA (7.46 mL) were added at room temperature. After the reaction vessel was filled with nitrogen gas, TCFH (4.81 g) was added, and the mixture was stirred at approximately 25°C for 3.5 hours. Then, MeTHF (20 mL) and a 5% aqueous potassium carbonate solution (17 mL) were added, and the mixture was stirred at approximately 25°C. The aqueous layer was discharged, and the obtained organic layer was washed with 5% sulfuric acid (17 mL) and a 5% aqueous potassium carbonate solution (17 mL). The obtained organic layer was concentrated under reduced pressure, acetonitrile (20 mL) was then added, and the mixture was concentrated. The resulting concentrated solution was cooled to 0°C, and the resulting precipitate was then collected by filtration. The obtained wet solid was washed with acetonitrile (10 mL), and dried under reduced pressure to afford compound Fmoc-12 (2.18 g) as a white solid.
LC retention time of compound Fmoc-12: 5.231 minutes (HPLC analysis conditions: method 2)

### Example 1-22

### Synthesis of tert-butyl N-((S)-2-((S)-N-allyl-2-(methylamino)pent-4-enamide)-3-(4-(trifluoromethyl)phenyl)propanoyl)-N-methylglycinate hydrochloride (compound 12-HCl)

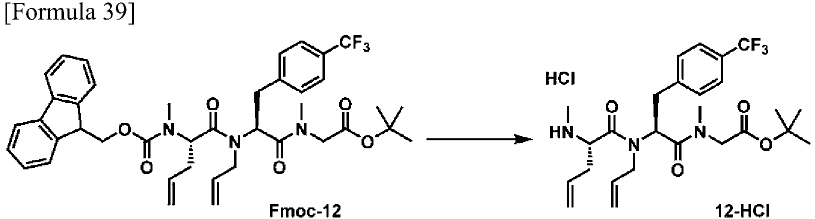

Compound Fmoc-12 (2.18 g, 2.97 mmol) obtained in the above Example 1-21, acetonitrile (7 mL) and DBU (0.45 mL) were added at approximately 25°C to a reaction vessel, and the reaction mixture was stirred for 1.5 hours. Then, to the reaction mixture, water (0.54 mL), triethylamine (1.66 mL) and sodium hydrogen sulfite (0.77 g) were added, and the mixture was stirred at approximately 25°C for 2 hours. To the reaction mixture, toluene (30 mL) and 10% aqueous ammonia (16 mL) were added, the mixture was stirred, and the aqueous layer was then discharged. The obtained organic layer was washed three times with 10% aqueous ammonia (16 mL), and then with a 5% aqueous sodium chloride solution (10 mL). The obtained organic layer was concentrated under reduced pressure, and azeotropic dehydration with the addition of toluene (20 mL) was performed 3 times.

To the obtained residue, an acetonitrile solution (1 mL) containing pyridine hydrochloride (0.36 g) was added. MTBE (6 mL) and heptane (6 mL) were then added, and the resulting mixture was stirred. The resulting precipitate was collected by filtration, and the obtained wet powder was washed with heptane (5 mL). The obtained solid was dried under reduced pressure to afford 1.50 g of compound 12-HCl (2.74 mmol, yield 92.1%).
UV intensity ratio: 99.5%
(Detection wavelength 210 nm, retention time 3.030 minutes, HPLC analysis conditions: method 1, m/z = 512 [M+H]⁺ (ESI))

### Example 1-23

### Synthesis of tert-Butyl N-methyl-N-((S)-2-((S)-3-(methylamino)-2-oxo-2,3,4,7-tetrahydro-1H-azepin-1-yl)-3-(4-(trifluoromethyl)phenyl)propanoyl)glycinate hydrochloride (compound 13-HCl)

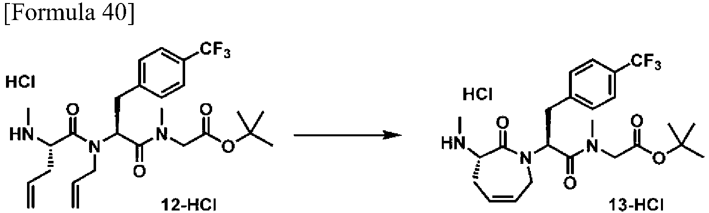

Compound 12-HCl (75.8 mg, 0.14 mmol) obtained in the above Example 1-22, second-generation Hoveyda-Grubbs catalyst (4.44 mg), acetone (1.02 mL) and HFIP (0.68 mL) were added to a reaction vessel. After the reaction vessel was filled with nitrogen gas, the reaction mixture was then heated at the external temperature of 65°C for 1 hour. The reaction mixture was allowed to cool to approximately 25°C, and the reaction mixture was then sampled, and analyzed by HPLC. The results showed that the conversion ratio was 98.1%, and dimers formed by the intermolecular reactions were not detected. The reaction mixture was concentrated, acetone (1 mL) was then added, and the resulting precipitate was filtered. The obtained wet solid was washed with acetone (1 mL), and the obtained solid was dried under reduced pressure to afford compound 13-HCl (57.7 mg, yield 80.2%) as a white solid.
UV intensity ratio: 98.9%
(Detection wavelength 210 nm, retention time 2.733 minutes, HPLC analysis conditions: method 1, m/z = 484 [M+H]⁺ (ESI))
¹H-NMR (500 MHz, DMSO-d₆) δ 9.44 (brs, 1H),8.69 (brs, 1H), 7.62-7.42 (m, 4H), 5.81-5.35 (m, 3H), 4.75-4.70 (m, 1H),4.38-4.29 (m, 1H), 4.11-3.87 (m, 2H), 3.83-3.60 (m, 1H), 3.14-2.81 (m, 5H),2.65-2.71 (m, 1H), 2.22-2.07 (m, 4H), 1.40 (s, 9H)

By using compound 12-HCl in which the N-terminus is not protected, a ring-closing metathesis reaction successfully proceeded to afford cyclic compound 13-HCl in ≥80% yield with an LC purity of ≥98%.

### Example 1-24

### Synthesis of tert-butyl (S)-N-(2-(but-3-en-1-ylamino)-3-(p-tolyl)propanoyl)-N-methylglycinate hydrochloride (compound 7-HCl)

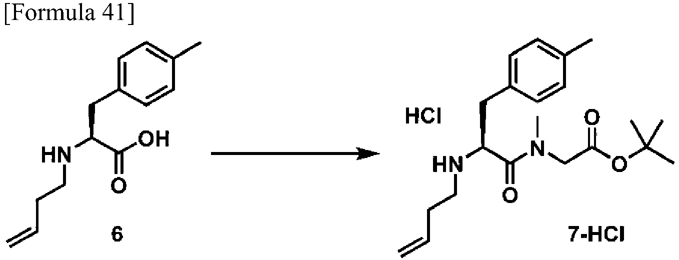

Compound 6 (1.10 g, 4.71 mmol) obtained in the above Example 1-11, 1,1'-carbonyldiimidazole (0.92 g, 5.66 mmol) and THF (16.5 mL) were added to a reaction vessel. After the reaction vessel was filled with nitrogen gas, trifluoromethanesulfonic acid (0.96 mL, 10.8 mmol) was added at 25°C, and the mixture was stirred at 50°C for 1.5 hours. The mixture was cooled to 25°C, a solution obtained by dissolving sarcosine tert-butyl hydrochloride (1.03 g, 5.66 mmol) in THF (2 mL), acetonitrile (3 mL) and DIPEA (3.8 mL, 21.7 mmol) was then added to the mixture. The resulting mixture was stirred for 2 hours. Then, a 5% aqueous potassium carbonate solution (11 mL) was added, the mixture was stirred for 5 minutes, and the aqueous layer was discharged. The obtained organic layer was washed with a 10% aqueous sodium hydrogen sulfate solution (11 mL) and a 5% aqueous sodium hydrogen sulfate solution (11 mL). Each aqueous layer was washed three times with MeTHF (11 mL). All organic layers were mixed, and concentrated under reduced pressure. To the obtained residue, acetonitrile (12 mL) and 2 M hydrochloric acid (5 mL) were added, and the mixture was concentrated under reduced pressure. The resulting precipitate was filtered, washed with water, and then dried under reduced pressure to afford 0.48 g of compound 7-HCl.
LCMS (ESI) of compound 7, retention time: 2.689 minutes, m/z = 361 [M+H]⁺ (HPLC analysis conditions: method 1)

### Example 1-25

### Synthesis of tert-butyl N-((S)-2-((S)-N-(but-3-en-1-yl)-2-(methylamino)hex-5-enamide)-3-(p-tolyl)propanoyl)-N-methylglycinate hydrochloride (compound 14-HCl)

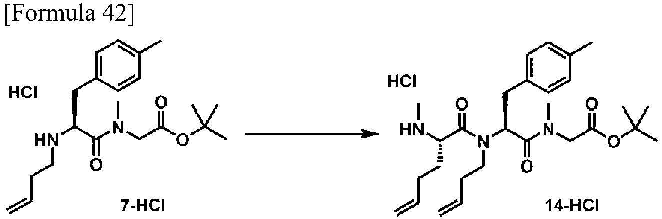

Compound 7-HCl (0.89 g, 2.25 mmol) obtained by the same method as in the above Example 1-24, (S)-2-((((9-H-fluoren-9-yl)methoxy)carbonyl)(methyl)amino)-5-hexenoic acid (1.07 g, 2.92 mmol), acetonitrile (4.5 mL), MeTHF (2.3 mL) and DIPEA (1.96 mL, 11.2 mmol) were added to a reaction vessel. To the reaction mixture, TCFH (1.29 g) was added, and the mixture was stirred at approximately 25°C for 2.5 hours. Then, MeTHF (18 mL) and a 5% aqueous potassium carbonate solution (9 mL) were added, the mixture was stirred, and the aqueous layer was discharged. The obtained organic layer was washed with 5% sulfuric acid (5.4 mL) and a 5% aqueous potassium carbonate solution (4.5 mL). The obtained organic layer was concentrated, acetonitrile (20 mL) was added, and azeotropic dehydration was performed. To the obtained residue, acetonitrile (2 mL) and DBU (0.51 mL) were added, and the mixture was stirred for 1 hour. DBU (0.34 mL) was added, and the mixture was stirred for 20 minutes. Then, to the reaction mixture, triethylamine (1.25 mL), water (0.41 mL) and sodium hydrogen sulfite (0.58 g) were added, and the mixture was stirred for 1 hour. To the reaction mixture, triethylamine (0.62 mL) was added, and the mixture was stirred for 45 minutes. Then, to the reaction mixture, toluene (15 mL) and 10% aqueous ammonia (20 mL) were added, the mixture was stirred, and the aqueous layer was discharged. The obtained organic layer was washed four times with 10% aqueous ammonia (10 mL), and then with a 5% aqueous sodium chloride solution (10 mL). The obtained organic layer was concentrated under reduced pressure, toluene (10 mL) was added, and azeotropic dehydration was performed. The obtained residue was filtered, the filtrate was then concentrated, and acetonitrile (1 mL) containing pyridine hydrochloride (0.27 g) was then added. To the mixture, MTBE (10 mL) was added, and the resulting mixture was stirred at approximately 25°C for 1 hour. The mixture was cooled to 0°C, and the resulting precipitate was then filtered. The obtained wet solid was washed with MTBE (5 mL), and dried under reduced pressure to afford compound 14-HCl (0.81 g).
UV intensity ratio: 99.3%
(Detection wavelength 210 nm, retention time 3.150 minutes, HPLC analysis conditions: method 1, m/z = 486 [M+H]⁺ (ESI))

### Example 1-26

### Synthesis of tert-Butyl N-methyl-N-((S)-2-((S,Z)-3-(methylamino)-2-oxo-2,3,4,5,8,9-hexahydro-1H-azonin-1-yl)-3-(p-tolyl)propanoyl)glycinate hydrochloride (compound 15-HCl)

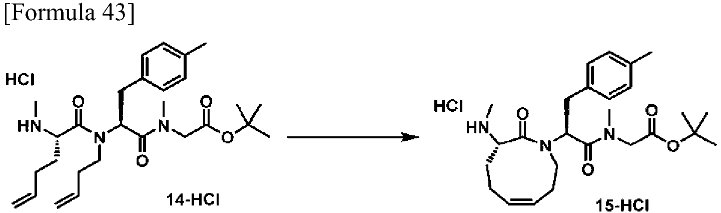

Compound 14-HCl (53.3 mg, 0.10 mmol) obtained in the above Example 1-25, second-generation Hoveyda-Grubbs catalyst (3.1 mg, 0.005 mmol), acetone (1 mL) and HFIP (0.12 mL) were added to a reaction vessel. After the reaction vessel was filled with nitrogen gas, the reaction mixture was heated at an external temperature of 60°C for 1 hour. The reaction mixture was sampled, and analyzed by HPLC. The results showed that the conversion ratio was 97.8%, and the ratio of the dimers, formed through the intermolecular reactions, to the product (15-HCl) was 6.1%. The reaction mixture was further stirred for 1 hour, and allowed to cool to approximately 25°C, and MTBE (1 mL) was then added. The resulting precipitate was filtered, and the obtained wet powder was washed with a mixed solution of acetone and MTBE (0.4 mL, volume ratio 1 : 1), and then with MTBE (1 mL). The obtained solid was dried under reduced pressure to afford compound 15-HCl (19.7 mg, yield 39.1%).
UV intensity ratio: 98.2%
(Detection wavelength 210 nm, retention time 2.768 minutes, HPLC analysis condition method 2)
LCMS (ESI) of compound 15, retention time: 2.696 minutes, m/z = 458 [M+H]⁺ (HPLC analysis conditions: method 1)
¹H-NMR (500 MHz, DMSO-d₆, detected as a mixture of rotamers) δ 9.29 (brs, 1H), 8.55 (brs, 1H), 7.15-6.99 (m, 4H), 5.97-5.55 (m,3H), 4.32-3.72 (m, 4H), 3.54-3.47 (m, 1H), 3.13-3.01 (m, 3H), 2.93-2.78 (m,2H), 2.27-1.71 (m, 9H), 1.67 (s, 1.8H), 1.52 (s, 1.2H), 1.46 (s, 3.2H), 1.37(s, 5.8H)

### Example 1-27

### Synthesis of (5S,8S)-5,7-di(but-3-en-1-yl)-1-(9H-fluoren-9-yl)-4,10-dimethyl-3,6,9-trioxo-8-(4-(trifluoromethyl)benzyl)-2-oxa-4,7,10-triazadodecane-12-acid tert-butyl (compound Fmoc-16)

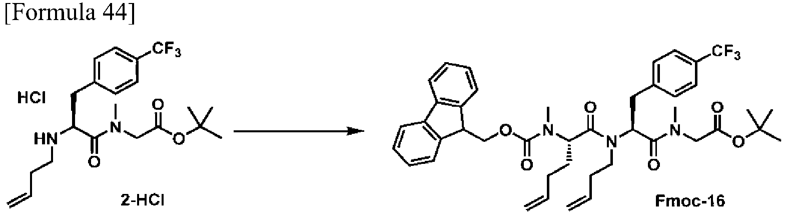

Compound 2-HCl (2.53 g, 5.61 mmol) obtained by the method of the above Example 1-2, (S)-2-((((9-H-fluoren-9-yl)methoxy)carbonyl)(methyl)amino)-5-hexenoic acid (1.07 g, 7.03 mmol), acetonitrile (12.5 mL), MeTHF (6.3 mL) and DIPEA (4.89 mL) were added to a reaction vessel. Then, TCFH (3.20 g) was added to the reaction vessel, and the resulting mixture was stirred at approximately 25°C for 2.5 hours, and then left to stand for 23 hours. Then, MeTHF (25 mL) and a 5% aqueous potassium carbonate solution (18 mL) were added, the mixture was stirred, and the aqueous layer was discharged. The obtained organic layer was washed with 5% sulfuric acid (18 mL) and a 5% aqueous potassium carbonate solution (18 mL), and concentrated under reduced pressure. To the obtained residue, acetonitrile (20 mL) was added, and azeotropic dehydration was performed. The resulting precipitate was then filtered, and the obtained wet solid was washed with acetonitrile (10 mL). The obtained solid was dried under reduced pressure to afford compound Fmoc-16 (2.32 g).
UV intensity ratio: 99.4%
(Detection wavelength 210 nm, retention time 5.590 minutes, HPLC analysis condition method 5)

### Example 1-28

### Study on synthesis of tert-butyl N-((S)-2-((S,Z)-3-((((9H-fluoren-9-yl)methoxy)carbonyl)(methyl)amino)-2-oxo-2,3,4,5,8,9-hexahydro-1H-azonin-1-yl)-3-(4-(trifluoromethyl)phenyl)propanoyl)-N-methylglycinate (compound Fmoc-17)

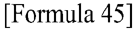

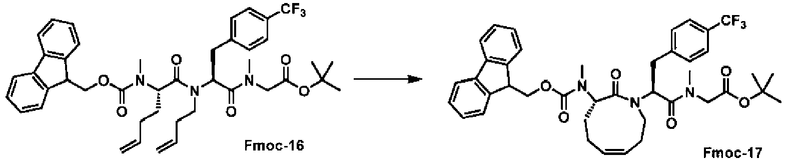

To a reaction vessel, compound Fmoc-16 (71.7 mg, 0.094 mmol) obtained in the above Example 1-27, second-generation Hoveyda-Grubbs catalyst (2.9 mg, 0.0045 mmol), acetone (0.94 mL) and HFIP (0.11 mL) were added. After the reaction vessel was filled with nitrogen gas, the reaction mixture was heated at 60°C for 1 hour. The reaction mixture was sampled, and analyzed by HPLC. The results showed that the conversion ratio was 77.6%, and the ratio of the dimers, formed through the intermolecular reactions, to the product (Fmoc-17) was 161%. LCMS (ESI) of compound Fmoc-17, retention time: 2.932 minutes, m/z = 756 [M+Na]⁺ (HPLC analysis conditions: method 3

Table 4 below shows comparison between the ring-closing metathesis of compound 14-HCl in which the N-terminus is not protected (Example 1-26) and the ring-closing metathesis of Fmoc-protected compound Fmoc-16 (Example 1-28).

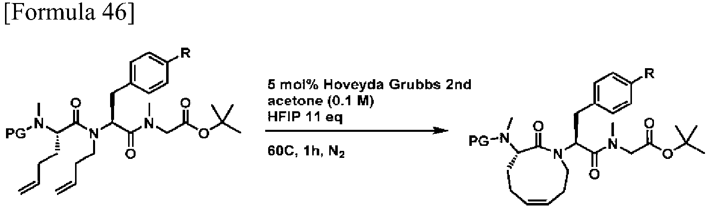

**[Table 4]**

| PG | R | Conversion ratio (%) | Dimer ratio (%) |
|---|---|---|---|
| H (HCl salt) | CH₃ | 97.8 | 6.1 |
| Fmoc | CF₃ | 77.6 | 161 |

It was confirmed that the use of compound 14-HCl in which the N-terminus is not protected allowed the ring-closing metathesis reaction to proceed with a higher conversion ratio and in an intramolecularly selective manner as compared to the use of Fmoc-protected compound Fmoc-16.

### Example 1-29

### Synthesis of (S)-2-(but-3-en-1-ylamino)-N,N-dimethyl-3-(4-(trifluoromethyl)phenyl)propaneamide (compound 18)

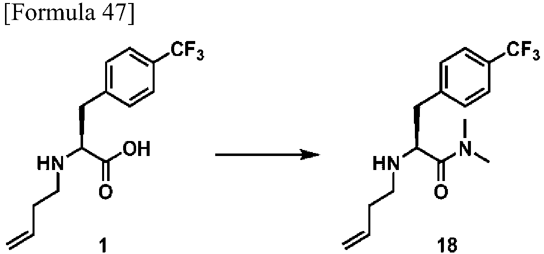

To a reaction vessel, compound 1 (2.21 g, 7.69 mmol) obtained in the above Example 1-1, dimethylamine (2 M THF solution, 7.69 mL, 15.4 mmol), acetonitrile (6.6 mL) and DBU (3.48 mL) were added at approximately 25°C. Then, T3P (50 wt% MeTHF solution, 6.18 mL) was added to the reaction mixture at the external temperature of 0°C, and the resulting mixture was stirred at approximately 25°C for 40 minutes. T3P (50 wt% MeTHF solution, 3 mL) and dimethylamine (2M THF solution, 3.0 mL, 6.0 mmol) were added to the reaction mixture, and the mixture was stirred for 45 minutes. Then, to the reaction mixture, MeTHF (22 mL) and a 1 mol/L aqueous sodium hydroxide solution (11 mL) were added. After discharging the aqueous layer, the obtained organic layer was washed twice with a 1 mol/L aqueous sodium hydroxide solution (11 mL), twice with a 5% aqueous sodium dihydrogen phosphate solution (11 mL), with a 1 mol/L aqueous sodium hydroxide solution (11 mL), and with a 5% aqueous potassium carbonate solution (11 mL). The obtained organic layer was concentrated, MeTHF (20 mL) was added, and azeotropic dehydration was performed. The obtained residue was filtered, and concentrated to afford a residue (2.98 g) containing compound 18.
LCMS (ESI) of compound 18, retention time: 2.207 minutes, m/z = 315 [M+H]⁺ (HPLC analysis conditions: method 1)

### Example 1-30

### Synthesis of (S)-N-(but-3-en-1-yl)-N-((S)-1-(dimethylamino)-1-oxo-3-(4-(trifluoromethyl)phenyl)propan-2-yl)-2-(methylamino)pent-4-eneamide hydrochloride (compound 19-HCl)

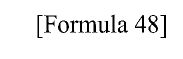

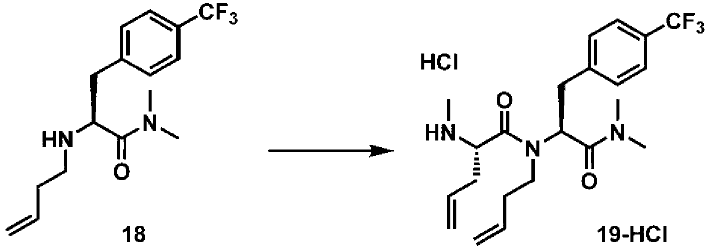

To a reaction vessel, a residue (2.98 g) containing the compound 18 obtained in the above Example 1-29, (S)-2-((9-H-fluoren-9-yl)methoxy)carbonyl)(methyl)amino)-4-pentenoic acid (2.70 g), acetonitrile (9.6 mL) and DIPEA (4.0 mL) were added. To the resulting mixture, TCFH (3.21 g) was added, and the mixture was then stirred at approximately 25°C for 2.5 hours. Then, to the reaction mixture, MeTHF (30 mL) and a 5% aqueous potassium carbonate solution (12 mL) were added, the mixture was then stirred, and the aqueous layer was discharged. The obtained organic layer was washed twice with 5% sulfuric acid (12 mL) and a 5% aqueous potassium carbonate solution (12 mL). The resulting organic layer was concentrated under reduced pressure. To the resulting mixture, MeTHF (30 mL) was added, and azeotropic dehydration was performed. To the obtained residue, acetonitrile (10 mL) and DBU (1.39 mL) were added. The reaction mixture was stirred at approximately 25°C for 75 minutes, triethylamine (4.29 mL), water (1.39 mL) and sodium hydrogen sulfite (1.99 g) were added, and the mixture was stirred for 2 hours. Then, to the reaction mixture, toluene (40 mL) and 10% aqueous ammonia (40 mL) were added, the mixture was stirred, and the aqueous layer was discharged. The obtained organic layer was washed three times with 10% aqueous ammonia (40 mL), and with a 5% aqueous sodium chloride solution (20 mL), and the organic layer was concentrated under reduced pressure. Azeotropic dehydration of the residue with toluene (20 mL) was repeated three times. The obtained slurry was filtered, and concentrated. To the residue, a solution prepared by dissolving pyridine hydrochloride (0.81 g) in acetonitrile (3 mL) was added. After an addition of MTBE (20 mL), the resulting mixture was stirred for 1 hour. The resulting precipitate was collected by filtration, and the obtained wet solid was washed with MTBE (5 mL). The obtained solid was dried under reduced pressure to afford compound 19-HCl (2.05 g).
UV intensity ratio: 98.9%
(Detection wavelength 210 nm, retention time 2.560 minutes, HPLC analysis conditions: method 1, m/z = 426 [M+H]⁺ (ESI))

### Example 1-31

### Synthesis of (S)-N,N-dimethyl-2-((S,Z)-3-(methylamino)-2-oxo-3,4,7,8-tetrahydroazocin-1(2H)-yl)-3-(4-(trifluoromethyl)phenyl)propaneamide hydrochloride (compound 20-HCl)

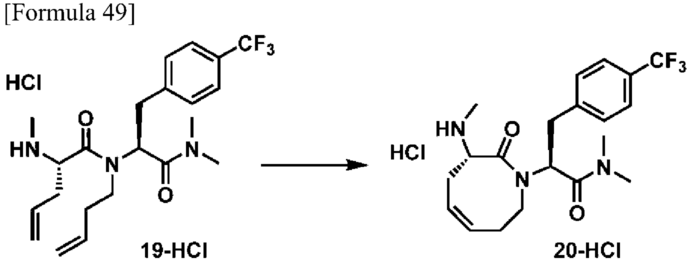

To a reaction vessel, compound 19-HCl (98.5 mg, 0.21 mmol) obtained in the above Example 1-30, second-generation Hoveyda-Grubbs catalyst (6.6 mg, 0.011 mmol), acetone (1.5 mL) and HFIP (0.5 mL) were added. After the reaction vessel was filled with nitrogen gas, the reaction mixture was heated at an external temperature of 65°C for 1 hour. The reaction mixture was then sampled and analyzed by HPLC. The results showed that the conversion ratio was 89.8%, and the ratio of the dimers, formed through the intermolecular reactions, to the product (20-HCl) was 4.6%. After the reaction mixture was allowed to cool to approximately 25°C, the resulting precipitate was filtered, and the obtained wet solid was washed with acetone (2 mL). The obtained solid was dried under reduced pressure to afford compound 20-HCl (46.9 mg, yield 50.7%).
UV intensity ratio: 98.2%
(Detection wavelength 210 nm, retention time 2.135 minutes, HPLC analysis conditions: method 1, m/z = 398 [M+H]⁺ (ESI))
¹H-NMR(DMSO-d₆,500 MHz) δ: 9.55 (brs, 1H), 8.57 (brs, 1H), 7.59 (d, J= 8.1 Hz, 2H), 7.50(d, J = 8.1 Hz, 2H), 5.72 (dd, J = 9.7, 5.5 Hz, 1H),5.62-5.58 (m, 1H), 5.42-5.37 (m, 1H), 4.50 (t, J = 8.0 Hz, 1H), 3.79-3.73 (m,1H), 3.59-3.53 (m, 1H), 3.11-3.01 (m, 2H), 2.88-2.81 (m, 7H), 2.46-2.41 (m,1H), 2.22-2.06 (m, 2H), 1.86 (s, 3H)

Cyclic dipeptide 20-HCl was successfully obtained with a purity of ≥98% through a ring-closing metathesis reaction.

### Example 1-32

### Synthesis of tert-butyl N-((S)-2-((S)-2-((((9H-fluoren-9-yl)methoxy)carbonyl)amino)-N-(but-3-en-1-yl)pent-4-eneamide)-3-(4-(trifluoromethyl)phenyl)propanoyl)-N-methylglycinate (compound Fmoc-21)

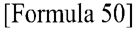

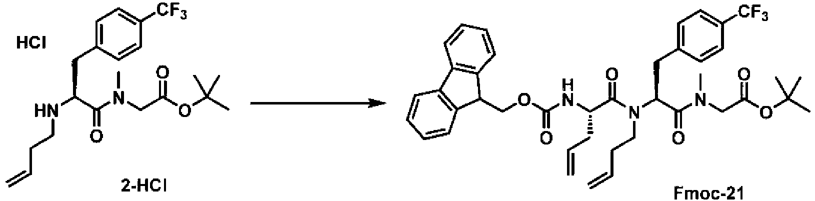

Compound 2-HCl (5.01 g, 11.1 mol) synthesized in Example 1-2 was added to a reaction vessel. Then, to the reaction vessel, (S)-2-(((9-H-fluoren-9-yl)methoxy)carbonyl)amino)-4-pentenoic acid (4.74 g), 1,3-dimethyl-2-imidazolidinone (25 mL) and 2,4,6-trimethylpyridine (7.35 mL) were added. Then, TCFH (4.93 g) was added to the mixture, and the resulting mixture was stirred at an external temperature of 25°C for 7 hours, and the reaction mixture was then left to stand for 16 hours. To the reaction vessel, a 5% aqueous potassium carbonate solution (25 mL) and MeTHF (30 mL) were added, the mixture was stirred, and the aqueous layer was discharged. The obtained organic layer was washed with 5% sulfuric acid (25 mL) and a 5% aqueous potassium carbonate solution (25 mL) in this order. The obtained organic layer was concentrated under reduced pressure, and azeotropic dehydration with the addition of MeTHF (20 mL) was repeated twice to afford a residue containing compound Fmoc-21. The obtained residue was purified by reversed phase column chromatography (mobile phase: water/acetonitrile volume ratio 40 : 60 → 0 : 100). The collected solution was concentrated, and azeotropic dehydration with the addition of acetonitrile was repeated to afford Fmoc-21 (5.00 g) as a white solid.
UV intensity ratio: 97.9%
(Detection wavelength 210 nm, retention time 5.182 minutes, HPLC analysis conditions: method 1, m/z = 756 [M+H]⁺ (ESI))

### Example 1-33

### Synthesis of tert-butyl N-((S)-2-((S)-2-amino-N-(but-3-en-1-yl)pent-4-enamide)-3-(4-(trifluoromethyl)phenyl)propanoyl)-N-methylglycinate (compound 21)

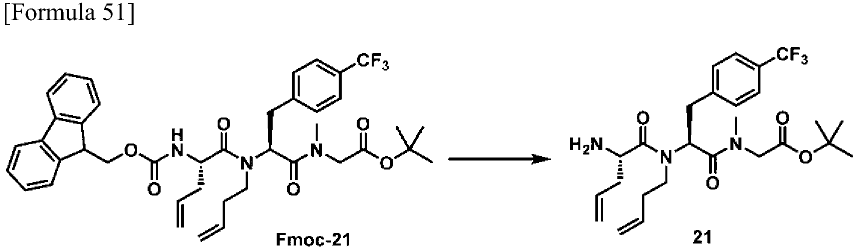

Compound Fmoc-21 (3.02 g) obtained in Example 1-32 was dissolved with acetonitrile (15 mL) in a reaction vessel. DBU (0.62 mL) was then added, and the resulting mixture was stirred at an external temperature of 25°C for 30 minutes. Then, to the reaction mixture, triethylamine (2.29 mL), sodium hydrogen sulfite (1.06 g) and water (0.74 mL) were added, and the reaction mixture was stirred at an external temperature of 25°C for 1.5 hours. To the reaction vessel, toluene (40 mL) and 10% aqueous ammonia (24 mL) were added, the obtained mixture was stirred for 10 minutes, and the aqueous layer was then discharged. The obtained organic layer was washed with 10% aqueous ammonia (24 mL), and then with a 10% aqueous sodium chloride solution (24 mL). The obtained organic layer was concentrated under reduced pressure. Azeotropic dehydration with the addition of toluene (20 mL) was performed to afford a residue containing compound 21. The obtained residue was purified by reversed phase column chromatography (mobile phase: water/acetonitrile volume ratio 80 : 20 → 40 : 60). The collected solution was concentrated, and azeotropic dehydration with the addition of acetonitrile was repeated to afford 1.36 g of compound 21 (content 91.6%, product of NMR content calculated by qNMR and UV intensity ratio) as a pale yellow oil.
UV intensity ratio: 93.7%
(Detection wavelength 210 nm, retention time 3.171 minutes, HPLC analysis conditions: method 2)

### Example 1-34

### Synthesis of tert-Butyl N-((S)-2-((S,Z)-3-(((benzyloxy)carbonyl)amino)-2-oxo-3,4,7,8-tetrahydroazocin-1(2H)-yl)-3-(4-(trifluoromethyl)phenyl)propanoyl)-N-methylglycinate (compound Cbz-22)

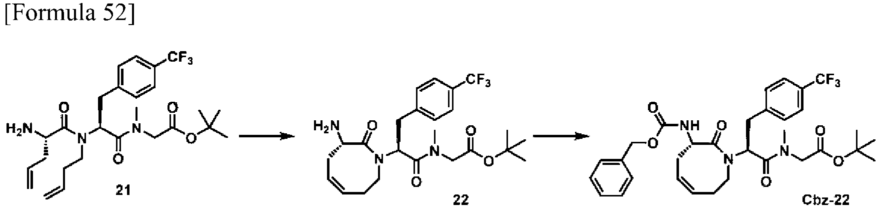

Compound 21 (130.8 mg, content 91.6%, 0.23 mmol) synthesized in Example 1-33, acetone (2.34 mL), HFIP (0.24 mL), boron trifluoride-diethyl ether complex (44.5 µL, 0.35 mmol), and second-generation Hoveyda-Grubbs catalyst (10.3 mg, 16.4 mmol) were added to a reaction vessel. After the reaction vessel was filled with nitrogen gas, the mixture in the reaction vessel was stirred at an external temperature of 65°C for 1 hour. The reaction mixture was sampled and analyzed by HPLC. It was confirmed that the conversion ratio was 95.1%, and the ratio of the dimers, formed through the intermolecular reactions, to the product (22) was 7.4%. The reaction mixture was allowed to cool to 25°C, triethylamine (52.3 µL) was then added, and the mixture was stirred. To the reaction mixture, MTBE (3 mL) and a 5% aqueous potassium carbonate solution (1 mL) were added, the mixture was stirred, and the aqueous layer was then discharged. The obtained organic layer was concentrated. Thereafter, to the obtained residue, acetonitrile (1 mL), N-carbobenzoxyoxysuccinimide (58.7 mg) and DIPEA (40.8 µL) were added, and the resulting mixture was stirred at an external temperature of 25°C for 30 minutes. The reaction mixture was concentrated under reduced pressure, and MTBE (3 mL) and a 1 M aqueous sodium hydroxide solution (1 mL) were then added. The resulting mixture was stirred, and the aqueous layer was then discharged. The obtained organic layer was washed with a 10% aqueous potassium hydrogen sulfate solution (1 mL), and then with a 5% aqueous potassium carbonate solution (1 mL). The obtained organic layer was concentrated under reduced pressure, and the obtained residue was purified twice by reversed phase column chromatography (mobile phase: water/acetonitrile, first: volume ratio 45 : 55 → 20 : 80, second: volume ratio 45 : 55 → 25 : 75). The collected solution was concentrated, and azeotropic dehydration with the addition of acetonitrile was repeated to afford 47.2 mg of compound Cbz-22 (yield: 32.6%) as a white solid.
UV intensity ratio: 98.3%
(Detection wavelength 210 nm, retention time 4.398 minutes, HPLC analysis conditions: method 1, m/z = 640 [M+H]⁺ (ESI))
¹H-NMR (500 MHz, DMSO-d₆, detected as a mixture of rotamers) δ 7.54 (t, J = 7.8 Hz, 2H), 7.46-7.18 (m, 8H), 5.66-5.59 (m, 1.6H), 5.50-5.45 (m, 0.6H), 5.36-5.29 (m, 0.8H), 5.05-5.01 (m, 0.9H), 4.95-4.91 (m, 1.1H), 4.74-4.68 (m, 1H), 4.24 (d, J = 18.5 Hz, 0.4H), 3.98 (d, J = 17.0 Hz, 0.6H), 3.83-3.42 (m, 3H), 3.18-3.08 (m, 1H), 2.86-2.74 (m, 4H), 2.64-2.58 (m, 1H), 2.51-2.36 (m, 1H), 2.31-2.08 (m, 2H), 1.46-1.21 (m, 9H)

### Example 1-35

### Synthesis of tert-butyl (S)-(1-oxo-1-(phenylamino)propan-2-yl)carbamate (compound Boc-23)

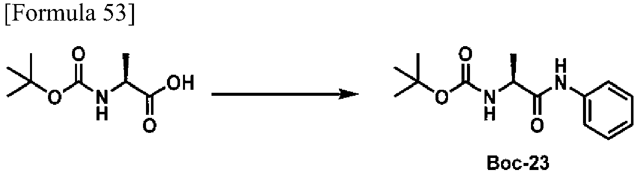

To a reaction vessel, N-(tert-butoxycarbonyl)-L-alanine (3.98 g), acetonitrile (4 mL), aniline (2.11 mL) and N-methylmorpholine (6.94 mL) were added. HATU (8.96 g) was then added to the reaction mixture at an external temperature of 0°C, and the resulting mixture was stirred at an external temperature of 25°C for 1.5 hours. Then, to the reaction mixture, a 5% aqueous potassium carbonate solution (16 mL) and 4-methyltetrahydropyran (40 mL) were added, and the mixture was stirred. The aqueous layer was discharged, and the obtained organic layer was then washed twice with 10% aqueous ammonia (20 mL), and then twice with a 5% aqueous sodium dihydrogen phosphate solution (20 mL). The obtained organic layer was concentrated under reduced pressure, and azeotropic dehydration with the addition of acetonitrile was repeated twice. To the obtained concentrated product, MTBE (10 mL) was added, the mixture was then stirred. The resulting solid was collected by filtration, and the obtained wet solid was washed with MTBE (5 mL). The obtained wet solid was dried under reduced pressure to afford compound Boc-23 (3.64 g) as a white solid.
UV intensity ratio: 99.6%
(Detection wavelength 210 nm, retention time 2.732 minutes, HPLC analysis conditions: method 1, m/z = 287 [M+H]⁺ (ESI))

### Example 1-36

### Synthesis of (S)-2-(amino)-N-phenylpropanamide (compound 23)

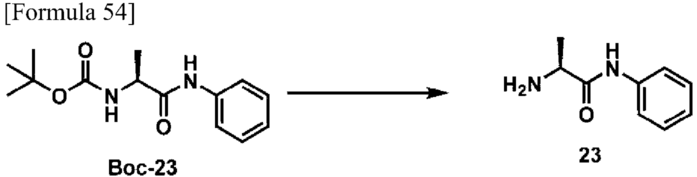

Compound Boc-23 (3.64 g) obtained in Example 1-35 and acetonitrile (15 mL) were added to a reaction vessel. Then, to the mixture, methanesulfonic acid (2.68 mL) was added. The resulting mixture was then stirred at an external temperature of 25°C for 3 hours. To the reaction mixture, 8 M aqueous sodium hydroxide solution (6.89 mL) and 4-methyltetrahydropyran (15 mL) were added at an external temperature of 0°C, and the mixture was then stirred. The organic layers were collected, and the aqueous layer was extracted with 4-methyltetrahydropyran (20 mL). The obtained organic layers were mixed and concentrated under reduced pressure. Azeotropic dehydration with the addition of 4-methyltetrahydropyran (20 mL) was performed twice. The obtained residue was filtered and washed with 4-methyltetrahydropyran (10 mL). The filtrate and the wash solution were mixed, and the mixture was then concentrated to afford a residue (3.13 g) containing compound 23.
LCMS (ESI) of compound 23, retention time: 1.118 minutes, m/z = 165 [M+H]⁺ (HPLC analysis conditions: method 1)

### Example 1-37

### Synthesis of ((S)-1-(allyl((S)-1-oxo-1-(phenylamino)propan-2-yl)amino)-1-oxopent-4-en-2-yl)(methyl)carbamic acid (9H-fluoren-9-yl)methyl (compound Fmoc-24)

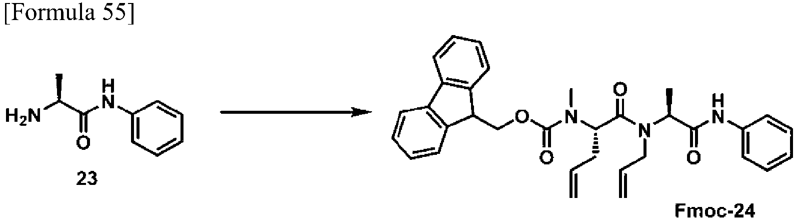

The residue (2.83 g) containing compound 23 obtained in Example 1-36 and DMF (6.1 mL) were added to a reaction vessel. Then, to the mixture, DIPEA (3.25 mL) and allyl bromide (1.29 ml) were added. The mixture was then stirred at external temperature of 25°C for 2 hours. Then, to the reaction mixture, acetonitrile (6.1 mL), (S)-2-(((9-H-fluoren-9-yl)methoxy)carbonyl)(methyl)amino)-4-pentenoic acid (3.49 g), DIPEA (5.2 mL) and TCFH (4.14 g) were added. The resulting mixture was then stirred at an external temperature of 25°C for 3 hours. To the reaction mixture, a 5% aqueous potassium carbonate solution (20 mL) and MTBE (30 mL) were added, the mixture was stirred, and the aqueous layer was then discharged. The obtained organic layer was washed with a 5% aqueous potassium carbonate solution (20 mL), 5% sulfuric acid (20 mL × 2 time) and a 5% aqueous potassium carbonate solution (20 mL) in this order. The obtained organic layer was concentrated under reduced pressure, and azeotropic dehydration with the addition of acetonitrile (20 mL) was performed twice. The obtained residue was purified by reversed phase column chromatography (mobile phase: water/acetonitrile volume ratio 50 : 50 → 15 : 85). The collected solution was concentrated, and azeotropic dehydration with the addition of acetonitrile was repeated to afford compound Fmoc-24 (2.63 g) as a white solid.
UV intensity ratio: 97.9%
(Detection wavelength 210 nm, retention time 4.475 minutes, HPLC analysis conditions: method 1, m/z = 560 [M+H]⁺ (ESI))

### Example 1-38

### Synthesis of (S)-N-allyl-2-(methylamino)-N-((S)-1-oxo-1-(phenylamino)propane-2-yl)pent-4-eneamide hydrochloride (compound 24-HCl)

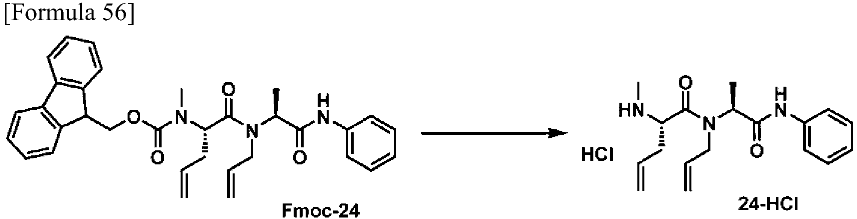

Compound Fmoc-24 (2.63 g) obtained in Example 1-37 and acetonitrile (8 mL) were added to a reaction vessel. Then, to the mixture, DBU (0.37 mL) was added, and the resulting mixture was stirred at an external temperature of 25°C for 75 minutes. To the reaction mixture, MTBE (26 mL) and a 5% aqueous potassium carbonate solution (13 mL) were added, the mixture was then stirred, and the aqueous layer was discharged. To the obtained organic layer, 0.5 M hydrochloric acid (15 mL) was added, the mixture was then stirred, and the organic layer was discharged. To the obtained aqueous layer, potassium carbonate (1.40 g) was added, and the resulting mixture was further stirred. To the mixture, 4-methyltetrahydropyran (25 mL) was added, and the mixture was further stirred. The resulting aqueous layer was discharged, and the obtained organic layer was then concentrated under reduced pressure. Azeotropic dehydration with the addition of 4-methyltetrahydropyran (20 mL) was performed. The obtained residue was then filtered, and the filtrate was concentrated. To the obtained residue, acetonitrile (20 mL) and 6 M hydrochloric acid (0.73 mL) were added, and the mixture was concentrated under reduced pressure. To the obtained residue, acetonitrile (5 mL) and MTBE (10 mL) were added, and the mixture was stirred until precipitation of a solid. MTBE (20 mL) was added, the mixture was further stirred, and the resulting solid was collected by filtration. The obtained wet solid was washed with MTBE (10 mL). The obtained wet solid was dried under reduced pressure to afford compound 24-HCl (1.21 g) as a white solid.
UV intensity ratio: 99.3%
(Detection wavelength 210 nm, retention time 1.991 minutes, HPLC analysis conditions: method 1, m/z = 316 [M+H]⁺ (ESI))

### Example 1-39

### Synthesis of (S)-2-((S)-3-(methylamino)-2-oxo-2,3,4,7-tetrahydro-1H-azepin-1-yl)-N-phenylpropanamide (compound 25)

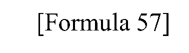

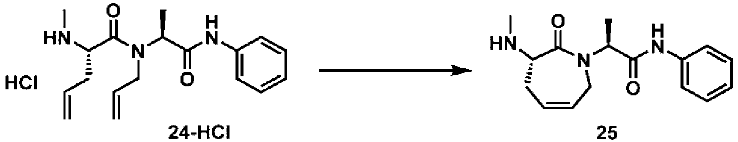

Compound 24-HCl (86.4 mg, 0.25 mmol) obtained in Example 1-38, second-generation Hoveyda-Grubbs catalyst (7.69 mg, 0.012 mmol), acetone (2.5 mL) and HFIP (0.28 mL) were added to a reaction vessel. After the reaction vessel was filled with nitrogen gas, the mixture in the reaction vessel was heated at an external temperature of 65°C for 2 hours. The reaction mixture was sampled and analyzed by HPLC. The results showed that the conversion ratio was 98.8%, and the dimers, formed through the intermolecular reactions, were undetected. The reaction mixture was allowed to cool to approximately 25°C, and then concentrated under reduced pressure. To the obtained residue, MTBE (3 mL) and 0.5 M hydrochloric acid (3 mL) were added, the mixture was then stirred, and the organic layer was discharged. To the obtained aqueous layer, potassium carbonate (0.23 g) was added. The mixture was stirred, and extraction with the addition of 4-methyltetrahydropyran (3 mL) was repeated three times. The obtained organic layers were mixed and concentrated under reduced pressure. Azeotropic dehydration with the addition of 4-methyltetrahydropyran (10 mL) was performed. The obtained residue was purified by reversed phase column chromatography (mobile phase: water/acetonitrile volume ratio 95 : 5 → 65 : 35). The collected solution was concentrated, and azeotropic dehydration with the addition of acetonitrile was repeated to afford compound 25 (39.8 mg, yield: 56.4%) as a brown oil.
UV intensity ratio: 98.8%
(Detection wavelength 210 nm, retention time 1.643 minutes, HPLC analysis conditions: method 1, m/z = 288 [M+H]⁺ (ESI))
¹H-NMR (500 MHz, DMSO-d₆) δ 9.75 (s, 1H), 7.54 (d, J = 7.6 Hz, 2H), 7.28 (t, J = 7.6 Hz, 2H), 7.03 (t, J = 7.6 Hz, 1H), 5.73-5.62 (m, 2H), 5.09 (q, J = 7.0 Hz, 1H), 4.27-4.23 (m, 1H), 3.82-3.73 (m, 2H), 2.47-2.23 (m, 5H), 2.13-1.91 (m, 1H), 1.29 (d, J = 7.0 Hz, 3H)

### [Industrial Applicability]

The present invention provides a method for efficiently producing a peptide compound having a peptide structure with a medium ring. The production method of the present invention enables reducing costs in production of the peptide compound and also reducing the environmental loads, and therefore, the production method of the present invention is particularly useful for synthesis of the peptide on a large scale.

## Claims

1. A method for producing a compound represented by formula (1) or a salt thereof, comprising the step (metathesis step) of bringing a compound represented by formula (2) or a salt thereof into contact with a catalyst: 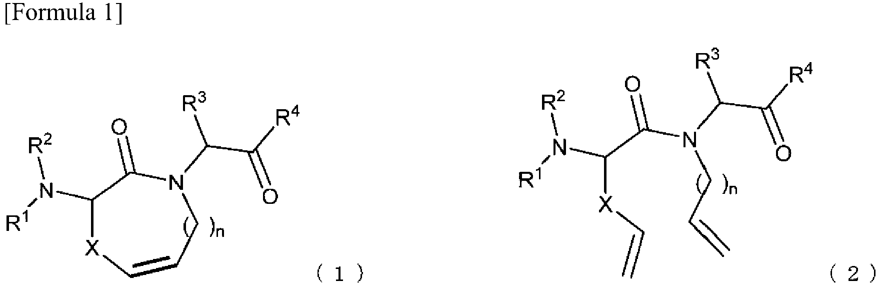 wherein
R¹ is hydrogen,
R² is hydrogen or C₁-C₆ alkyl;
R³ is hydrogen, optionally substituted C₁-C₆ alkyl, optionally substituted halo-C₁-C₆ alkyl,
optionally substituted C₂-C₆ alkenyl, optionally substituted C₂-C₆ alkynyl, optionally substituted C₃-C₈ cycloalkyl, optionally substituted C₆-C₁₄ aryl, optionally substituted 5- to 14-membered heteroaryl, optionally substituted C₇-C₁₄ aralkyl, optionally substituted 3- to 14-membered heterocyclyl, optionally substituted 5- to 10-membered heteroaryl-C₁-C₆ alkyl, optionally substituted C₁-C₆ alkoxy-C₁-C₆ alkyl, optionally substituted C₁-C₆ alkylsulfanyl-C₁-C₆ alkyl, optionally substituted C₁-C₆ alkylsulfinyl-C₁-C₆ alkyl, optionally substituted C₁-C₆ alkylsulfonyl-C₁-C₆ alkyl, optionally substituted carboxy-C₁-C₆ alkyl, optionally substituted C₇-C₁₄ aralkoxy-C₁-C₆ alkyl, optionally substituted C₃-C₈ cycloalkyl-C₁-C₆ alkyl, optionally substituted C₃-C₈ cycloalkoxy-C₁-C₆ alkyl, optionally substituted 4- to 7-membered heterocyclyl-C₁-C₃ alkyl, optionally substituted 5- to 10-membered heteroaryl-C₁-C₆ alkoxy-C₁-C₆ alkyl, or optionally substituted aminocarbonyl (the relevant amino is selected from the group consisting of -NH₂, mono-C₁-C₆ alkylamino, di-C₁-C₆ alkylamino, N-C₁-C₆ alkyl-N-C₂-C₆ alkenylamino, N-C₁-C₆ alkyl-N-C₁-C₆ alkoxy-C₁-C₆ alkylamino, and 4- to 9-membered cyclic amino),
R⁴ is OR⁵, NHR⁵', an amino acid residue, or a peptide chain containing 1 to 20 amino acid residues, where the amino acid residue and the peptide chain may have a protective group,
R⁵ is a protective group for a carboxy group,
R⁵' is a protective group for an amide group,
n is an integer of 1 to 4, and
X is optionally substituted C₁-C₃ alkylene, -CH₂OCH₂-, or -CH₂SCH₂-.

2. A method for producing a compound represented by formula (3) or a salt thereof, comprising the step of hydrogenating a compound represented by formula (1) or a salt thereof, which is obtained by the method of claim 1: 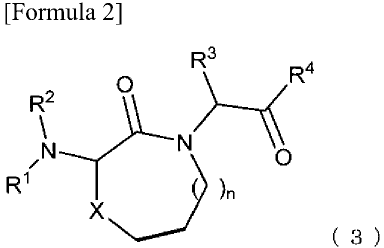
wherein R¹, R², R³, R⁴, n and X have the same meaning as R¹, R², R³, R⁴, n and X in [1],
respectively.

3. The method according to claim 1 or 2, wherein the catalyst is a metal-alkylidene complex.

4. The method according to any one of claims 1 to 3, wherein the compound represented by formula (2) or a salt thereof is a salt of a compound represented by formula (2).

5. The method according to any one of claims 1 to 3, wherein the compound represented by formula (2) or a salt thereof is a compound represented by formula (2), and an acid is used in the metathesis step.

6. The method according to any one of claims 1 to 5, wherein alcohols and/or Bronsted acids are further used as an additive in the metathesis step, and the pKa of the additive is 4 to 17.

7. The method according to any one of claims 1 to 6, wherein the metathesis step is performed by a liquid-phase synthesis method.

8. The method according to claim 7, wherein the solvent used in the liquid-phase synthesis method is one or more selected from the group consisting of acetone, methyl t-butyl ether, dimethyl carbonate, ethyl acetate, toluene, and dichloromethane.

9. The method according to claim 7 or 8, wherein a concentration of the compound represented by formula (2) or a salt thereof in the liquid-phase synthesis method is 0.01 to 0.5 mol/L.

10. The method according to any one of claims 1 to 9, wherein R² is hydrogen or linear C₁-C₃ alkyl.

11. The method according to any one of claims 1 to 10, wherein R³ is hydrogen, C₁-C₆ alkyl, or C₇-C₁₄ aralkyl optionally substituted with halo-C₁-C₃ alkyl or C₁-C₆ alkyl.

12. The method according to any one of claims 1 to 11, wherein R⁴ is OR⁵, NHR^{5'}, an amino acid residue, or a peptide chain containing 2 to 13 amino acid residues,
R⁵ is a protective group for a carboxy group,
R⁵' is a protective group for an amide group, and
the 2 to 13 amino acid residues are one or more selected from the group consisting of glycine, alanine, isoleucine, leucine, methionine, phenylalanine, tyrosine, N-methylglycine, N-methylalanine, N-methylisoleucine, N-methylleucine, N-methylmethionine, N-methylphenylalanine, and N-methyltyrosine.

13. The method according to any one of claims 1 to 12, wherein n is an integer of 1 or 2.

14. The method according to any one of claims 1 to 13, wherein X¹ is one selected from the group consisting of an Fmoc group, a Cbz group, a Troc group, an Alloc group, a Teoc group, a TSoc group, a BIBSoc group, an IPCSoc group, a BBSoc group, a CHBSoc group, a CDBSoc group, and a Boc group.

15. The method according to any one of claims 1 to 14, wherein X is optionally substituted C₁-C₃ alkylene.
